(19) Europäisches Patentamt
European Patent Office
Office européen des brevets

(11) EP 2 077 265 A1

(12) EUROPEAN PATENT APPLICATION
published in accordance with Art. 153(4) EPC

(43) Date of publication:
**08.07.2009 Bulletin 2009/28**

(21) Application number: **06821867.6**

(22) Date of filing: **13.10.2006**

(51) Int Cl.:
***C07D 405/14*** *(2006.01)*

(86) International application number:
**PCT/JP2006/320496**

(87) International publication number:
**WO 2007/122755 (01.11.2007 Gazette 2007/44)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **14.04.2006 PCT/JP2006/307934**

(71) Applicant: **Eisai R&D Management Co., Ltd.**
**Tokyo 112-8088 (JP)**

(72) Inventors:
- **KAWAHARA, Tetsuya**
  **Tsukuba-shi, Ibaraki 300-2635 (JP)**
- **HARADA, Hitoshi**
  **Tsukuba-shi, Ibaraki 300-2635 (JP)**
- **TERAUCHI, Hiroki**
  **Tsukuba-shi, Ibaraki 300-2635 (JP)**
- **WATANABE, Nobuhisa**
  **Tsukuba-shi, Ibaraki 300-2635 (JP)**
- **UEDA, Masato**
  **Tsukuba-shi, Ibaraki 300-2635 (JP)**
- **KAMADA, Atsushi**
  **Tsukuba-shi, Ibaraki 300-2635 (JP)**
- **KIKUCHI, Sachiko**
  **Tsukuba-shi, Ibaraki 300-2635 (JP)**
- **ISHIHARA, Hiroshi**
  **Tsukuba-shi, Ibaraki 300-2635 (JP)**
- **KOYAMA, Koichiro**
  **Tsukuba-shi, Ibaraki 300-2635 (JP)**

(74) Representative: **HOFFMANN EITLE**
**Patent- und Rechtsanwälte**
**Arabellastraße 4**
**81925 München (DE)**

# (54) SALT OF SULFINYLBENZIMIDAZOLE COMPOUND, AND CRYSTAL AND AMORPHOUS FORM THEREOF

(57) Salts of 2-[({4-[(2.2-dimethyl-1,3-dioxan-5-yl)methoxy]-3,5-dimethylpyridin-2-yl}methyl)sulfinyl]-1H-benzimidazole and their crystalline and amorphous forms.

EP 2 077 265 A1

## Description

## Technical Field

The present invention relates to sulfinylbenzimidazole compound salts and their crystalline and amorphous forms that are useful as gastric secretion inhibitors. The invention further relates to crystalline and amorphous forms of free sulfinylberzimidazole compounds.

## Background Art

Peptic ulcers such as gastric ulcer and duodenal ulcer are believed to occur as a consequence of autodigestion which is triggered by imbalance between offensive factors such as acid and pepsin, and defensive factors such as mucous and blood.

Peptic ulcers are generally treated by medical therapy, and a variety of medicament therapies have been attempted. Recently, medicaments that specifically inhibit the enzyme H+, K+-ATPase which exists in gastric parietal cell and governs the final process of gastric acid secretion, suppress acid secretion and as a result prevent autodigestion, such as omeprazole, esomeprazole, pantoprazole, lansoprazole, rabeprazole and the like, have been developed and are clinically used.

These medicaments have excellent therapeutic effects, but a demand exists for medicaments with longer lasting gastric secretion inhibitory action, higher safety and more suitable physicochemical stability.

As of the filing date of this patent application, it has not been disclosed that 2-[({4-[(2,2-dimethyl-1,3-dioxan-5-yl)methoxy]-3,5-dimethylpyridin-2-yl}methyl)sulfinyl]-1H-benzimidazole (hereinafter also referred to as "compound (1)") exhibits an excellent gastric secretion inhibitory action (especially a long-lasting gastric secretion inhibitory action and the ability to maintain a high gastric pH for long periods), and particularly that it is useful for treatment or prevention of gastroesophageal reflux, symptomatic gastroesophageal reflux disease, gastric ulcer or duodenal ulcer.
Consequently, 2-[({4-[(2,2-dimethyl-1,3-dioxan-5-yl)methoxy]-3,5-dimethylpyridin-2-yl}methyl)sulfinyl]-1H-benzimidazole salts and their crystalline forms have also not been disclosed.

Compounds related to the present invention are described in Patent documents 1 and 2, but the specific compounds mentioned in these patent documents differ in chemical structure from the specific compounds of the present application.

[Patent document 1] WO 91/19712
[Patent document 2] Japanese Unexamined Patent Publication No. SHO59-181277

## Disclosure of the Invention

### Problems to be Solved by the Invention

The physical properties of medically useful compounds and their salts, as well as their crystalline and amorphous forms, significantly affect medicament bioavailability, drug substance purity and formulation, and therefore pharmaceutical development requires research on the compounds in terms of whether their salts, crystalline forms or amorphous forms are best as pharmaceuticals. That is, because the physical properties depend on the attributes of the individual compounds it is generally difficult to make predictions regarding the salt/crystalline form/amorphous form of a drug substance having satisfactory physical properties, and actual experimentation should be conducted for each compound.

It is therefore an object of the present invention to provide various salts of compound (1), and their crystalline and amorphous forms. It is another object of the invention to provide crystalline and amorphous forms of compound (1).

### Means for Solving the Problems

The present inventors, upon synthesizing and isolating various salts and crystals, examining their physical properties and forms and conducting experimentation, have discovered salts and crystals of compound (1) as drug substances or as production intermediates for production, having satisfactory physical properties of drug substances, and have thereupon completed this invention.

Specifically, the invention relates to the following [1]-[63].

[1] A salt of 2-[({4-[(2,2-dimethyl-1,3-dioxan-5-yl)methoxy]-3,5-dimethylpyridin-2-yl}methyl)sulfinyl]-1H-benzimidazole, wherein the salt is a salt selected from the group consisting of a sodium salt, a potassium salt, a magnesium

salt, a lithium salt, a calcium salt and a zinc salt.

[1-2] A salt of 2-[({4-[(2,2-dimethyl-1,3-dioxan-5-yl)methoxy]-3,5-dimethylpyridin-2-yl}methyl)sulfinyl]-1H-benzimidazole, wherein the salt is a salt selected from the group consisting of a potassium salt, a magnesium salt, lithium salt, calcium salt and zinc salt.

[2] A salt according to [1], wherein the salt is a sodium salt.

[3] A crystalline form of a salt according to [2], having a peak at a chemical shift (±0.5 ppm) of 16.7 ppm in a $^{13}C$ solid state NMR spectrum (A-form crystals of sodium salt).

[4] A crystalline form according to [3], further having a peak at a chemical shift (±0.5 ppm) of 62.8 ppm in a $^{13}C$ solid state NMR spectrum.

[5] A crystalline form according to [4], further having peaks at chemical shifts (±0.5 ppm) of 32.5, 53.3, 120.6, 122.7, 149.8 and 157.2 ppm in a $^{13}C$ solid state NMR spectrum.

[6] A crystalline form of a salt according to [2], having absorption peaks at wavenumbers (±2 $cm^{-1}$) of 827, 1021 and 1295 $cm^{-1}$ in an infrared absorption spectrum (ATR method) (A-form crystals of sodium salt).

[7] A crystalline form according to [6], further having absorption peaks at wavenumbers (±2 $cm^{-1}$) of 739 and 1099 $cm^{-1}$ in an infrared absorption spectrum (ATR method).

[8] A crystalline form according to [7], further having an absorption peak at a wavenumber (±2 $cm^{-1}$) of 992 $cm^{-1}$ in an infrared absorption spectrum (ATR method).

[9] A crystalline form of a salt according to [2], having diffraction peaks at diffraction angles (2θ ±0.2°) of 5.0°, 8.0°, 9.9°, 10.8°, 11.7° and 12.9° in a powder X-ray diffraction (A-form crystals of sodium salt).

[10] A crystalline form of a salt according to [2], having a peak at a chemical shift (±0.5 ppm) of 25.3 ppm in a $^{13}C$ solid state NMR spectrum (B-form crystals of sodium salt).

[11] A crystalline form according to [10], further having a peak at a chemical shift (±0.5 ppm) of 21.7 ppm in a $^{13}C$ solid state NMR spectrum.

[12] A crystalline form according to [11], further having peaks at chemical shifts (±0.5 ppm) of 17.8 and 70.2 ppm in a $^{13}C$ solid state NMR spectrum.

[13] A crystalline form of a salt according to [2], having absorption peaks at wavenumbers (±2 $cm^{-1}$) of 829, 1022 and 1289 $cm^{-1}$ in an infrared absorption spectrum (ATR method) (B-form crystals of sodium salt).

[14] A crystalline form according to [13], further having absorption peaks at wavenumbers (±2 $cm^{-1}$) of 738 and 1387 $cm^{-1}$ in an infrared absorption spectrum (ATR method).

[15] A crystalline form according to [14], further having an absorption peak at a wavenumber (±2 $cm^{-1}$) of 798 $cm^{-1}$ in an infrared absorption spectrum (ATR method).

[16] A crystalline form of a salt according to [2], having diffraction peaks at diffraction angles (2θ ±0.2°) of 5.0°, 9.5°, 9.9°, 11.4°, 13.6°, 15.6° and 16.5° in a powder X-ray diffraction (B-form crystals of sodium salt).

[17] A crystalline form of a salt according to [2], having a peak at a chemical shift (±0.5 ppm) of 137.7 ppm in a $^{13}C$ solid state NMR spectrum (C-form crystals of sodium salt).

[18] A crystalline form according to [17], further having a chemical shift (±0.5 ppm) of 22.7 ppm in a $^{13}C$ solid state NMR spectrum.

[19] A crystalline form according to [18], further having a chemical shift (±0.5 ppm) of 144.0 ppm in a $^{13}C$ solid state NMR spectrum.

[20] A crystalline form of a salt according to [2], having absorption peaks at wavenumbers (±2 $cm^{-1}$) of 822 and 1022 $cm^{-1}$ in an infrared absorption spectrum (ATR method) (C-form crystals of sodium salt).

[21] A crystalline form according to [20], further having an absorption peak at a wavenumber (±2 $cm^{-1}$) of 1244 $cm^{-1}$ in an infrared absorption spectrum (ATR method).

[22] A crystalline form of a salt according to [2], having diffraction peaks at diffraction angles (2θ ±0.2°) of 4.9°, 9.8°, 11.4°, 11.7°, 14.6°, 15.7°, 16.2°, 17.3° and 18.9° in a powder X-ray diffraction (C-form crystals of sodium salt).

[23] A crystalline form of a salt according to [2], having a peak at a chemical shift (±0.5 ppm) of 159.8 ppm in a $^{13}C$ solid state NMR spectrum (D-form crystals of sodium salt).

[24] A crystalline form according to [23], further having peaks at chemical shifts (±0.5 ppm) of 49.6 and 165.2 ppm in a $^{13}C$ solid state NMR spectrum.

[25] A crystalline form according to [24], further having peaks at chemical shifts (±0.5 ppm) of 27.8 and 72.2 ppm in a $^{13}C$ solid state NMR spectrum.

[26] A crystalline form of a salt according to [2] having an absorption peak at a wavenumber (±2 $cm^{-1}$) of 1077 $cm^{-1}$ in an infrared absorption spectrum (ATR method) (D-form crystals of sodium salt).

[27] A crystalline form according to [26], further having absorption peaks at wavenumbers (±2 $cm^{-1}$) of 830 and 1093 $cm^{-1}$ in an infrared absorption spectrum (ATR method).

[28] A crystalline form according to [27], further having an absorption peak at a wavenumber (±2 $cm^{-1}$) of 1264 $cm^{-1}$ in an infrared absorption spectrum (ATR method).

[29] A crystalline form of a salt according to [2], having diffraction peaks at diffraction angles (2θ ±0.2°) of 4.1°,

10.4˚, 12.2˚, 12.7˚, 17.1˚, 18.5˚ and 19.4˚ in a powder X-ray diffraction (D-form crystals of sodium salt).
[30] A crystalline form of a salt according to [2], having diffraction peaks at diffraction angles (2θ ±0.2˚) of 4.4˚, 9.6˚, 10.3˚, 16.2˚, 17.5˚, 18.5˚ and 19,7˚ in a powder X-ray diffraction (E-form crystals of sodium salt).
[31] A crystalline form of a salt according to [2], having diffraction peaks at diffraction angles (2θ ±0.2˚) of 4.3˚, 8.6˚, 9.7˚, 10.3˚, 12.7˚, 13.2˚, 16.6˚, 17.5˚, 18.5˚, 19.2˚ and 19.8˚ in a powder X-ray diffraction (F-form crystals of sodium salt).
[32] A crystalline form of a salt according to [2], having diffraction peaks at diffraction angles (2θ ±0.2˚) of 4.1˚, 10.6˚, 16.9˚, 17.3˚, 17.9˚, 18.4˚, 19.4˚ and 19.7˚ in a powder X-ray diffraction (G-form crystals of sodium salt).
[33] A crystalline form of a salt according to [2], having diffraction peaks at diffraction angles (2θ ±0.2˚) of 4.4˚, 10.1˚, 10.3˚, 16.6˚, 17.7˚, 18.6˚, 19.8˚, 20.3˚ and 21.3˚ in a powder X-ray diffraction (H-form crystals of sodium salt).
[34] An amorphous form of a salt according to [2], having a broad peak centered at a chemical shift of

(1) 116.5-120.5 ppm (with a peak half-width of 4.0 ppm or greater) in a $^{13}C$ solid state NMR spectrum.

[35] An amorphous form according to [34], further having broad peaks centered at chemical shifts of

(2) 145.5-151.0 ppm (with a peak half-width of 5.5 ppm or greater),
(3) 125.0-128.0 ppm (with a peak half-width of 3,0 ppm or greater) and
(4) 10.5-14.0 ppm (with a peak half-width of 3.5 ppm or greater)

in a $^{13}C$ solid state NMR spectrum.
[36] An amorphous form according to [35], further having broad peaks centered at chemical shifts of

(5) 97.0-98.5 ppm (with a peak half-width of 1.5 ppm or greater) and
(6) 59.0-62.0 ppm (with a peak half-width of 3.0 ppm or greater)

in a $^{13}C$ solid state NMR spectrum.
[37] An amorphous form of a salt according to [2], having absorption peaks at wavenumbers (±2 $cm^{-1}$) of 803 and 1269 $cm^{-1}$ in an infrared absorption spectrum (ATR method).
[38] An amorphous form according to [37], further having absorption peaks at wavenumbers (±2 $cm^{-1}$) of 822 and 1248 $cm^{-1}$ in an infrared absorption spectrum (ATR method).
[39] An amorphous form according to [38], further having an absorption peak at a wavenumber (±2 $cm^{-1}$) of 1071 $cm^{-1}$ in an infrared absorption spectrum (ATR method).
[40] A salt according to [1], wherein the salt is a potassium salt.
[41] A crystalline form of a salt according to [40], having diffraction peaks at diffraction angles (2θ ±0.2˚) of 5.2˚, 10.4˚, 14.5˚, 16.2˚ and 19.5˚ in a powder X-ray diffraction.
[42] A salt according to [1], wherein the salt is a magnesium salt.
[43] A crystalline form of a salt according to [42], having diffraction peaks at diffraction angles (2θ ±0.2˚) of 5.4˚, 16.2˚ and 19.9˚ in a powder X-ray diffraction.
[44] A salt according to [1], wherein the salt is a lithium salt.
[45] A crystalline form of a salt according to [44], having diffraction peaks at diffraction angles (2θ ±0.2˚) of 4.9˚, 9.9˚, 15.9˚, and 22.9˚ in a powder X-ray diffraction.
[46] A salt according to [1], wherein the salt is a calcium salt.
[47] A salt according to [1], wherein the salt is a zinc salt.
[48] A crystalline form of 2-[({4-[(2,2-dimethyl-1,3-dioxan-5-yl)methoxy]-3,5-dimethylpyridin-2-yl}methyl)sulfinyl]-1H-benzimidazole, having diffraction peaks at diffraction angles (2θ ±0.2˚) of 8.1˚, 12.5˚ and 20.5˚ in a powder X-ray diffraction (A-form crystals of free acid).
[49] A crystalline form of 2-[({4-[(2,2-dimethyl-1,3-dioxan-5-yl)methoxyl-3,5-dimethylpyridin-2-yl}methyl)sulfinyl]-1H-benzimidazole having diffraction peaks at diffraction angles (2θ ±0.2˚) of 6.0˚, 9.0˚ and 17.3˚ in a powder X-ray diffraction (B-form crystals of free acid).
[50] An amorphous form of 2-[({4-[(2,2-dimethyl-1,3-dioxan-5-yl)methoxy]-3,5-dimethylpyridin-2-yl}methyl)sulfinyl]-1H-benzimidazole having a broad peak centered at a chemical shift of

(1) 119.0-126.5 ppm (with a peak half-width of 7.5 ppm or greater)

in a $^{13}C$ solid state NMR spectrum.
[51] An amorphous form according to [50], having broad peaks centered at chemical shifts of

(2) 110.5-114.0 ppm (with a peak half-width of 3.5 ppm or greater) and
(3) 133.5-136.0 ppm (with a peak half-width of 2.5 ppm or greater)

in a $^{13}C$ solid state NMR spectrum.
[52] An amorphous form according to [51] having broad peaks centered at chemical shifts of

(5) 142.5-145.0 ppm (with a peak half-width of 2.5 ppm or greater) and
(6) 147.5-152.0 ppm (with a peak half-width of 4.5 ppm or greater)

in a $^{13}C$ solid state NMR spectrum.
[53] An amorphous form of 2-[({4-[(2,2-dimethyl-1,3-dioxan-5-yl)methoxy]-3,5-dimethylpyridin-2-yl}methyl)sulfinyl]-1H-benzimidazole, having absorption peaks at wavenumbers ($\pm$2 $cm^{-1}$) of 822, 1269 and 1396 $cm^{-1}$ in an infrared absorption spectrum (ATR method).
[54] An amorphous form according to [53], further having an absorption peak at a wavenumber ($\pm$2 $cm^{-1}$) of 746 $cm^{-1}$ in an infrared absorption spectrum (ATR method).
[55] An amorphous form according to [54], further having absorption peaks at wavenumbers ($\pm$2 $cm^{-1}$) of 1010 and 1034 $cm^{-1}$ in an infrared absorption spectrum (ATR method).
[55-2] An amorphous form of 2-[({4-[(2,2-dimethyl-1,3-dioxan-5-yl)methoxy]-3,5-dimethylpyridin-2-yl}methyl)sulfinyl]-1H-benzimidazole sodium salt.
[55-3] An amorphous form of 2-[({4-[(2,2-Dimethyl-1,3-dioxan-5-yl)methoxy]-3-5-dimethylpyridin-2-yl}methyl)sulfinyl]-1H-benzimidazole.
[55-4] (S)-2-[({4-[(2,2-Dimethyl-1,3-dioxan-5-yl)methoxy]-3,5-dimethylpyridin-2-yl}methyl)sulfinyl]-1H-benzimidazole or sodium salt thereof.
(Including not only substances with an optical purity of 1.0% ee but also all mixtures containing (S)-2-[({4-[(2,2-dimethyl-1,3-dioxan-5-yl)methoxy]-3,5-dimethylpyridin-2-yl}methyl)sulfinyl]-1H-benzimidazole or sodium salt thereof.)
[55-5] (R)-2-[({4-[(2,2-Dimethyl-1,3-dioxan-5-yl)methoxy]-3,5-dimethylpyridin-2-yl}methyl)sulfinyl]-1H-benzimidazole or sodium salt thereof.
(Including not only substances with an optical purity of 100% ee but also all mixtures containing (R)-2-[({4-[(2,2-dimethyl-1,3-dioxan-5-yl)methoxy]-3,5-dimethylpyridin-2-yl}methyl)sulfinyl]-1H-benzimidazole or sodium salt thereof.)
[55-6] (S)-2-[({4-[(2,2-dimethyl-1,3-dioxan-5-yl)methoxy]-3,5-dimethylpyridin-2-yl}methyl)sulfinyl]-1H-benzimidazole sodium salt.
(Including not only substances with an optical purity of 100% ee but also all mixtures containing (S)-2-[({4-[(2,2-dimethyl-1,3-dioxan-5-yl)methoxy]-3,5-dimethylpyridin-2-yl}methyl)sulfinyl]-1H-benzimidazole sodium salt.)
[55-7] (R)-2-[({4-[(2,2-dimethyl-1,3-dioxan-5-yl)methoxy]-3,5-dimethylpyridin-2-yl}methyl)sulfinyl]-1H-benzimidazole sodium salt.
(Including not only substances with an optical purity of 100% ee but also all mixtures containing (R)-2-[({4-[(2,2-dimethyl-1,3-dioxan-5-yl)methoxy]-3,5-dimethylpyridin-2-yl}methyl)sulfinyl]-1H-benzimidazole sodium salt.)
[55-8] (R)-2-[({4-[(2,2-dimethyl-1,3-dioxan-5-yl)methoxy]-3,5-dimethylpyridin-2-yl}methyl)sulfinyl]-1H-benzimidazole sodium salt ((R) form) having an (S)-2-[({4-[(2,2-dimethyl-1,3-dioxan-5-yl)methoxy]-3,5-dimethylpyridin-2-yl}methyl)sulfinyl]-1H-benzimidazole sodium salt ((S) form) content of no greater than 1.5 wt% (percentage based on the total weight of the (S) form and (R) form).
(That is, (R)-2-[({4-[(2,2-dimethyl-1,3-dioxan-5-yl)methoxy]-3,5-dimethylpyridin-2-yl}methyl)sulfinyl]-1H-benzimidazole sodium salt with an optical purity of 97.0% ee or greater.)
[55-9] (R)-2-[({4-[(2,2-dimethyl-1,3-dioxan-5-yl)methoxy]-3,5-dimethyipyridin-2-yl}methyl)sulfinyl]-1H-benzimidazole sodium salt ((R) form) having an (S)-2-[({4-[(2,2-dimethyl-1,3-dioxan-5-yl)methoxy]-3,5-dimethylpyridin-2-yl}methyl)sulfinyl]-1H-benzimidazole sodium salt ((S) form) content of no greater than 1 wt% (percentage based on the total weight of the (S) form and (R) form).
(That is, (R)-2-[({4-[(2,2-dimethyl-1,3-dioxan-5-yl)methoxy]-3,5-dimethylpyridin-2-yl}methyl)sulfinyl]-1H-benzimidazole sodium salt with an optical purity of 98.0% ee or greater.)
[55-10] (R)-2-[({4-[(2,2-dimethyl-1,3-dioxan-5-yl)methoxy]-3,5-dimethylpyridin-2-yl}methyl)sulnnyl]-1H-benzimidazole sodium salt ((R) form) having an (S)-2-[({4-[(2,2-dimethyl-1,3-dioxan-5-yl)methoxy]-3,5-dimethylpyridin-2-yl}methyl)sulfinyl]-1H-benzimidazole sodium salt ((S) form) content of no greater than 0.5 wt% (percentage based on the total weight of the (S) form and (R) form).
(That is, (R)-2-[({4-[(2,2-dimethyl-1,3-dioxan-5-yl)methoxy]-3,5-dimethylpyridin-2-yl}methyl)sulfinyl]-1H-benzimidazole sodium salt with an optical purity of 99.0% ee or greater.)
[55-11] (R)-2-[({4-[(2,2-dimethyl-1,3-dioxan-5-yl)methoxy]-3,5-dimethylpyridin-2-yl}methyl)sulfinyl]-1H-benzimidazole sodium salt ((R) form) having an (S)-2-[({4-[(2,2-dimethyl-1,3-dioxan-5-yl)methoxy]-3,5-dimethylpyridin-2-yl}methyl)sulfinyl]-1H-benzimidazole sodium salt ((S) form) content of no greater than 0.25 wt% (percentage based on the total weight of the (S) form and (R) form).
(That is, (R)-2-[({4-[(2,2-dimethyl-1,3-dioxan-5-yl)methoxy]-3,5-dimethylpyridin-2-yl}methyl)sulfinyl]-1H-benzimidazole sodium salt with an optical purity of 99.5% ee or greater.)
[56] A medicament comprising a salt, crystalline form or amorphous form according to any one of [1] to [55-11].
[57] A gastric secretion inhibitor comprising a salt, crystalline form or amorphous form according to any one of [1] to [55-11].
[58] A therapeutic and/or prophylactic agent for a disease associated with gastric acid, comprising a salt, crystalline form or amorphous form according to any one of [1] to [55-11].
[59] A therapeutic and/or prophylactic agent according to [58], wherein the disease associated with gastric acid is gastric ulcer, duodenal ulcer, stomal ulcer, gastroesophageal reflux (including recrudescent/recurring), Zollinger-Ellison syndrome, symptomatic gastroesophageal reflux, endoscope-negative gastroesophageal reflux, nonerosive gastroesophageal reflux, gastroesophageal reflux, NUD (Non-Ulcer Dyspepsia), laryngopharyngeal disorder, Barrett's esophagus, NSAID ulcer, gastritis, gastric hemorrhage, hemorrhagic gastritis, gastrointestinal hemorrhage, peptic ulcer, hemorrhagic ulcer, stress ulcer, gastric hyperacidity, dyspepsia, gastropathy, geriatric ulcer, intractable ulcer, acute gastric mucosal lesion, pyrosis, sleep apnea with pyrosis, bruxism, gastralgia, stomach heaviness, retching, nausea, temporomandibular arthrosis or gastric erosion.
[60] A therapeutic and/or prophylactic agent according to [58], wherein the disease associated with gastric acid is gastric ulcer, duodenal ulcer, stomal ulcer, gastroesophageal reflux, Zollinger-Ellison syndrome or symptomatic gastroesophageal reflux.
[61] A therapeutic and/or prophylactic agent according to [58], wherein the disease associated with gastric acid is gastroesophageal reflux or symptomatic gastroesophageal reflux.
[62] A therapeutic and/or prophylactic agent according to [58], wherein the disease associated with gastric acid is gastric ulcer or duodenal ulcer.
[63] A bacteria eliminator or bacteria eliminating adjuvant for intragastric *Helicobacter pylori,* comprising a salt, crystalline form or amorphous form according to any one of [1] to [55-11].

The meanings of the terms and symbols used throughout the present specification will now be explained, followed by a more detailed description of the invention.

Throughout the present specification, the structural formulas for compounds will represent only one isomer for convenience, but the invention includes all optical isomers and isomer mixtures implied by the compound structure, being not limited to the formula shown for convenience, and may relate to any one of the isomers or a mixture thereof. Thus, the compounds of the invention may exist as optically active or racemic forms, all of which are included without limitations according to the invention. They may also exist in polymorphic crystalline forms or amorphous forms, and likewise are not limited either way and may be the crystalline forms or amorphous forms alone or mixtures thereof, while the compounds of the invention also include anhydrides, solvates and solvent-adhered forms (such as crystals containing water of adhesion), as well as solid forms containing solvents.

Unless otherwise specified, the terms "crystalline form" and "amorphous form" according to the invention are not particularly limited so long as they include the aforementioned "crystalline form" or "amorphous form", and they may be either 100% pure or containing other polymorphic crystalline forms or amorphous forms. Forms containing trace amounts of the "crystalline form" or "amorphous form" are also included within the terms crystalline form" and "amorphous form" according to the invention.

The term "salt" used throughout the present specification is not particularly limited so long as it is a salt of compound (1) and a base, and the salt is formed with a base at the NH group of position I or 3 of the benzimidazole skeleton of compound (1). Specifically, for example, the base may form a salt at an appropriate ratio of 0.1-5 molecules to one molecule of the compound, and preferably in the case of a salt with a monovalent base (sodium or the like), the base forms a salt at an appropriate ratio of about one molecule to one molecule of the compound, or in the case of a salt with a divalent base (magnesium or the like), the base forms a salt at an appropriate ratio of about 0.5 molecule to one molecule of the compound.

The term "solvate" according to the invention is not particularly limited so long as it is one formed by a solvent used during production of the salt or crystal, and specifically there may be mentioned hydrates, ethanol solvates, acetone solvates, toluene solvates, tert-butyl methyl ether solvates and the like, among which toluene solvates are preferred.

Since the diffraction angle (2θ) in a powder X-ray diffraction is usually obtained at an error within a range of $\pm 0.2^\circ$, the value for the aforementioned diffraction angle must be interpreted as including values within a range of about $\pm 0.2^\circ$. Thus, the invention encompasses not only crystalline forms having completely matching peaks (diffraction angles) in a powder X-ray diffraction, but also crystalline forms having peaks (diffraction angles) that match with an error of about $\pm 0.2^\circ$.

Throughout the present specification, for example, the phrase "having a diffraction peak at a diffraction angle ($2\theta \pm 0.2°$) of 4.9°" means "having a diffraction peak at a diffraction angle ($2\theta$) of 4.7°-5.1°".

Since the wavenumber ($cm^{-1}$) in an infrared absorption spectrum (ATR method: attenuated total reflection method) is usually obtained at an error within a range of $\pm 2$ $cm^{-1}$, the value for the aforementioned wavenumber must be interpreted as including values within a range of about $\pm 2$ $cm^{-1}$. Thus, the invention encompasses not only crystalline forms having completely matching absorption peaks (wavenumbers) in an infrared absorption spectrum (ATR method), but also crystalline forms having absorption peaks (wavenumbers) that match with an error of about $\pm 2$ $cm^{-1}$.

Throughout the present specification, for example, the phrase "having an absorption peak at a wavenumber ($\pm 2$ $cm^{-1}$) of 827 $cm^{-1}$" means "having an absorption peak at a wavenumber of 825-829 $cm^{-1}$".

Since the chemical shift (ppm) in a $^{13}C$ solid state NMR spectrum is usually obtained with some degree of error, the invention encompasses not only crystalline forms whose peaks (chemical shifts) in a $^{13}C$ solid state NMR spectrum completely match, but also crystalline forms having peaks with essentially equivalent chemical shifts when the $^{13}C$ solid state NMR spectrum is measured under ordinary measuring conditions or under essentially the same conditions as described in the present specification, and specifically it is interpreted as including values in a range of about $\pm 0.5$ ppm. Specifically, for example, it includes not only crystalline forms whose peaks (chemical shifts) in a $^{13}C$ solid state NMR spectrum completely match, but also crystalline forms whose peaks (chemical shifts) match within an error of about $\pm 0.5$ ppm.

Throughout the present specification, for example, the phrase "having a peak at a chemical shift ($\pm 0.5$ ppm) of 16.7 ppm" means "having a peak with a chemical shift substantially equivalent to 16.7 ppm upon measurement of the $^{13}C$ solid state NMR spectrum under ordinary measuring conditions or under essentially the same conditions as described in the present specification", and specifically it means "having a peak with a chemical shift of 16.2-17.2 ppm".

Throughout the present specification, the term "peak half-width" means the breadth (ppm) of the peak at a position at half the height of the peak when the peak shape is cut in the transverse direction. That is, the half-width is determined by (1) drawing a base line for each peak, (2) drawing a horizontal line at a position midway between the maximum value and the base line of the peak and (3) measuring the width between the two points where the horizontal line intersects the spectrum.

Since an amorphous form has broad peaks for the chemical shift (ppm) in a $^{13}C$ solid state NMR spectrum such that a large range of error is obtained, the invention encompasses not only amorphous forms whose peaks (chemical shifts) in a $^{13}C$ solid state NMR spectrum completely match, but also amorphous forms having peaks with essentially equivalent chemical shifts when the $^{13}C$ solid state NMR spectrum is measured under ordinary measuring conditions or under essentially the same conditions as described in the present specification.
Specifically, for example, "having a broad peak centered at a chemical shift of 116.5-120.5 ppm (with a peak half-width of 4.0 ppm or greater)" means "having a peak center at a chemical shift between 116.5-120.5 ppm when the $^{13}C$ solid state NMR spectrum is measured under ordinary measuring conditions or under essentially the same conditions as described in the present specification, and having a peak that is substantially equivalent to the peak with a peak half-width of 4.0 ppm or greater".

The measurements of a powder X-ray diffraction, infrared absorption spectroscopy (ATR method: attenuated total reflection method) and $^{13}C$ solid state NMR spectroscopy or $^{15}N$ solid state NMR spectroscopy may be appropriately combined to examine the state of crystalline forms or amorphous forms in a composition such as a formulation, or in a drug substance (including all polymorphic crystalline forms, amorphous forms, solvates and anhydrides of free acids or salts). The method of measurement and the conditions are not particularly limited so long as the devices and conditions are commonly employed, and specifically, for example, the devices and conditions described in the examples below may be used for measurement. Particularly, since no peak is detected for additives used in a formulation or the like in the case of $^{15}N$ solid state NMR spectroscopy, even in the case of small amounts, or when crystalline forms or amorphous forms are included, it is possible to achieve sensitive detection and determine the crystalline forms or amorphous forms. According to this method, it is possible to examine the crystalline forms or amorphous forms not only of compound (1) of the present application or its salts, but also of different compounds, in compositions such as formulations or in drug substances.

Specifically, it is possible to examine the crystalline forms or amorphous forms in compositions such as formulations or in drug substances by the following methods [X1] and [X2].

[X1] A method of examining a state of a crystalline form or amorphous form in a composition such as a formulation, or in a drug substance using measurements by powder X-ray diffraction, infrared absorption spectroscopy (ATR method: attenuated total reflection method) and $^{13}C$ solid state NMR spectroscopy and/or $^{15}N$ solid state NMR spectroscopy.

[X2] A method of examining a state of a crystalline form or amorphous form in a composition such as a formulation, or in a drug substance using measurements by $^{15}N$ solid state NMR spectroscopy.

The conditions for measurement by $^{15}N$ solid state NMR spectroscopy may be, for example, the following conditions.

Measuring apparatus: AVANCE 400 MHz (Bruker)
Probe: 7 mm-CP/MAS (Bruker)
NMR cell diameter: 7 mm
Cell spinning rate: 6000 rotations/sec
Measuring method: CPMASS method
Observed nucleus: $^{15}N$ (resonance frequency: 40.5568940 MHz)
Delay time: 3 sec
Contact time: 3000 microseconds
Number of scans: HOPE-DP: 22217 times, HOPE-DS: 18984 times
External standard: The chemical shift for glycine nitrogen was defined as 10 ppm.

The phrase "disease or symptom associated with gastric acid" is not particularly limited so long as it is a disease or symptom associated with gastric acid, and as preferred examples there may be mentioned gastric ulcer, duodenal ulcer, stomal ulcer, gastroesophageal reflux, Zollinger-Ellison syndrome, symptomatic gastroesophageal reflux, endoscope-negative gastroesophageal reflux, nonerosive gastroesophageal reflux and acute gastric mucosal lesion, while more preferred examples are gastroesophageal reflux, symptomatic gastroesophageal reflux, gastric ulcer and duodenal ulcer, and even more preferably preferred examples are (1) gastroesophageal reflux or symptomatic gastroesophageal reflux, or (2) gastric ulcer or duodenal ulcer.

Throughout the present specification, the term "prophylactic agent" includes agents administered before onset of a disease or symptom, as well as maintenance therapies and recurrence inhibitors administered after treatment.
Also throughout the present specification, the term "bacteria eliminating adjuvant" refers to a medicament that alters the environment so as to bring out the effect of a bacteria eliminating agent which has a poor effect under acidic conditions.

Advantageous effect of the invention.

Salts, crystalline forms and amorphous forms of compound (1) of the invention have excellent gastric secretion inhibitory action as well as excellent physical properties as medical medicaments, and are therefore useful as therapeutic or prophylactic agents for gastroesophageal reflux, symptomatic gastroesophageal reflux, gastric ulcer, duodenal ulcer and the like.

## Brief Explanation of the Drawings

Fig. 1 shows a $^{13}C$ solid state NMR spectrum for the A-form crystals of sodium salt obtained in Example 2X.
Fig. 2 shows a $^{13}C$ solid state NMR spectrum for the A-form crystals of sodium salt obtained in Example 2X.
Fig. 3 shows a $^{13}C$ solid state NMR spectrum for the B-form crystals of sodium salt obtained in Example 4X.
Fig. 4 shows a $^{13}C$ solid state NMR spectrum for the B-form crystals of sodium salt obtained in Example 4X.
Fig. 5 shows a $^{13}C$ solid state NMR spectrum for the C-form crystals of sodium salt obtained in Example 1X.
Fig. 6 shows a $^{13}C$ solid state NMR spectrum for the C-form crystals of sodium salt obtained in Example 1X.
Fig. 7 shows a $^{13}C$ solid state NMR spectrum for the B-form crystals of sodium salt obtained in Example 5X.
Fig. 8 shows a $^{13}C$ solid state NMR spectrum for the D-form crystals of sodium salt obtained in Example 5X.
Fig. 9 shows a $^{13}C$ solid state NMR spectrum for the amorphous form of sodium salt obtained in Example 9X.
Fig. 10 shows a $^{13}C$ solid state NMR spectrum for the amorphous form of sodium salt obtained in Example 9X.
Fig. 1 1 shows a $^{13}C$ solid state NMR spectrum for the amorphous form of free acid obtained in Example 3Y.
Fig. 12 shows an infrared absorption spectrum (ATR method) for the A-form crystals of sodium salt obtained in Example 2X.
Fig. 13 shows an infrared absorption spectrum (ATR method) for the B-form crystals of sodium salt obtained in Example 4X.
Fig. 14 shows an infrared absorption spectrum (ATR method) for the C-form crystals of sodium salt obtained in Example 1X.
Fig. 15 shows an infrared absorption spectrum (ATR method) for the D-form crystals of sodium salt obtained in Example 5X.
Fig. 16 shows an infrared absorption spectrum (ATR method) for the amorphous form of sodium salt obtained in Example 9X.

Fig. 17 shows an infrared absorption spectrum (ATR method) for the amorphous form of free acid obtained in Example 3Y.
Fig. 18 shows a powder X-ray diffraction pattern for the A-form crystals of sodium salt obtained in Example 2X.
Fig. 19 shows a powder X-ray diffraction pattern for the B-form crystals of sodium salt obtained in Example 4X.
Fig. 20 shows a powder X-ray diffraction pattern for the C-form crystals of sodium salt obtained in Example 1X.
Fig. 21 shows a powder X-ray diffraction pattern for the D-form crystals of sodium salt obtained in Example 5X.
Fig. 22 shows a powder X-ray diffraction pattern for the E-form crystals of sodium salt obtained in Example 8X.
Fig. 23 shows a powder X-ray diffraction pattern for the F-form crystals of sodium salt obtained in Example 10X
Fig. 24 shows a powder X-ray diffraction pattern for the G-form crystals of sodium salt obtained in Example 6X.
Fig. 25 shows a powder X-ray diffraction pattern for the H-form crystals of sodium salt obtained in Example 7X.
Fig. 26 shows a powder X-ray diffraction pattern for the amorphous form of sodium salt obtained in Example 3X.
Fig. 27 shows a powder X-ray diffraction pattern for the amorphous form of sodium salt obtained in Example 9X.
Fig. 28 shows a powder X-ray diffraction pattern for the A-form crystals of free acid obtained in Example 1Y.
Fig. 29 shows a powder X-ray diffraction pattern for the B-form crystals of free acid obtained in Example 2Y.
Fig. 30 shows a powder X-ray diffraction pattern for the amorphous form of free acid obtained in Example 3Y.
Fig. 31 shows a powder X-ray diffraction pattern for the crystals of magnesium salt obtained in Example 2Z.
Fig. 32 shows a powder X-ray diffraction pattern for the crystals of potassium salt obtained in Example 1Z.
Fig. 33 shows a powder X-ray diffraction pattern for the crystals of lithium salt obtained in Example 3Z.
Fig. 34 shows a powder X-ray diffraction pattern for the crystals of zinc salt obtained in Example 4Z.
Fig. 35 shows a powder X-ray diffraction pattern for the crystals of calcium salt obtained in Example 5Z.

**Best Mode for Carrying Out the Invention**

Compound (1) of the invention may be produced by the processes described below. However, the processes for production of the compound of the invention are not limited to these alone.

Compound (1) may be synthesized according to the examples described below.

Compound (1) used for production of a salt may be in any desired form. Specifically, it may be a hydrate or anhydride, in amorphous form or crystalline form (including a plurality of polymorphic crystalline forms), or a mixture thereof.

For production of a salt of compound (1), the compound (1) is combined with a base and a solvent, and there is no limitation on the order of adding the compound (1), base and solvent, while the additions may also be carried out in divided portions.

For addition of compound (1), a solid of compound (1) may be added or a mixture of the compound and a solvent (i.e. a solution, suspension, slurry or the like) may be added.

The base used for production of a salt is not particularly limited so long as it is a base that can produce a salt of compound (1) when added to the free form of compound (1), and as specific examples there may be mentioned metal alkoxides (for example, sodium alkoxides of C1-6 alcohols such as sodium methoxide and sodium ethoxide, magnesium alkoxides such as magnesium methoxide, potassium alkoxides such as potassium methoxide and lithium alkoxides such as lithium methoxide), sodium hydroxide, halogenated metal compounds (for example, halogenated zinc compounds such as zinc chloride), metal nitric acid salts (for example, calcium nitrate) and the like, either as solids or as solutions obtained by dissolution in solvents (water, C1-6 alcohols or their mixed solvents).

The base used for production of a salt may be added without dissolving a basic substance in solution, or a basic substance may be dissolved in water or an organic solvent for addition of a basic solution.

The procedure employed for production of a salt of compound (1) is not particularly limited so long as it is an ordinarily used procedure, and specifically, for example, the following procedure may be carried out to produce a salt of compound (1).

Compound (1) is mixed with the base and solution, and the mixture is then stirred and allowed to stand or the solvent is distilled off to precipitate a salt of compound (1). The precipitated salt may be separated by an ordinary filtration procedure, washed if necessary with an appropriate solvent (usually the same solvent used for precipitation) and then dried to produce a salt of the compound of the invention.

Also, as mentioned above, a salt may be formed first with compound (1) and the base, the solvent mixed therewith, and the mixture stirred and allowed to stand, or the solvent is distilled off, to precipitate a salt of compound (1). The precipitated salt may be separated by an ordinary filtration procedure, washed if necessary with an appropriate solvent (usually the same solvent used for precipitation) and then dried to produce a salt of the compound of the invention.

As typical solvents to be used for production of the salt there may be mentioned ketones such as methyl ethyl ketone and acetone, alcohols such as methanol, ethanol, isopropanol and n-propanol, esters such as ethyl acetate and methyl acetate, hydrocarbons such as hexane and heptane, ethers such as tetrahydrofuran, diethyl ether and dioxane, carboxylic acids such as acetic acid, and water, or mixtures of these solvents, but there is no limitation to these.

The amount of solvent used may be appropriately selected, with a lower limit as the amount which allows

compound (1) to mix with the base under heating (for example, a solution, suspension or slurry form), and an upper limit as the amount which does not notably reduce the salt yield. The temperature for mixing of compound (1) and the base may be selected as appropriate for the solvent. The final cooling temperature may be appropriately selected based on the salt yield and quality, but it is preferably between room temperature and 0˚C.

The salt of the invention may also be precipitated by addition of a poor solvent (hexane, heptane or the like) to the solution in which compound (1) and the base are mixed.

Drying of the salt separated by the filtration procedure may be accomplished by allowing it to stand in air, but for mass production this is not efficient, and therefore drying may be accomplished by heating.

The drying temperature may be appropriately selected depending on the production volume. The drying time may be appropriately selected as the time at which the residual solvent falls below a prescribed amount, and will depend on the production volume, the drying apparatus and the drying temperature. The drying may be carried out in a draft or under reduced pressure, but preferably it is carried out under reduced pressure. The degree of pressure reduction may be appropriately selected depending on the production volume, the drying apparatus and the drying temperature.

[General production process (1) for A-form crystals of sodium salt]

The following procedures (A)-(G) may be appropriately selected and modified to produce A-form crystals of the sodium salt of compound (1). Specifically, for example, A-form crystals of the sodium salt of compound (1) may be produced with reference to the examples described below.

(A) The sodium salt of compound (1) is mixed with the solvent.
(B) The mixture of the sodium salt of compound (1) and the solvent is subjected to heated stirring and/or sonication.
(C) Another solvent (an alkyl-based solvent such as hexane or heptane, a cycloalkyl-based solvent such as cyclohexane, an ether-based solvent such as diethyl ether, or the like) is further added to the mixture of the sodium salt of compound (1) and the solvent.
(D) The mixture of the sodium salt of compound (1) and the solvent is cooled or allowed to stand at the same temperature.
(E) The precipitate in the mixture of the sodium salt of compound (1) and the solvent is collected by filtration.
(F) The obtained precipitate is washed.
(G) The precipitate is dried.

The sodium salt of compound (1) used for crystallization may be in any form, as a solvate or an anhydride, in amorphous or crystalline form, or in a combination thereof.

The solvent used for crystallization may be one or a mixture of two solvents selected from the group consisting of alcohol-based solvents, ether-based solvents, alkylketone-based solvents and alkyl-based solvents, and is preferably a mixed solvent of ethanol and methyl t-butyl ether.

The mixing ratio (volume ratio) for a mixed solvent of ethanol and methyl t-butyl ether is preferably 2:13-1:7 and more preferably 1:9-1:10.

The amount of solvent used may be appropriately selected from a lower limit of an amount which allows the sodium salt of compound (1) to dissolve by heating, to an upper limit of an amount which does not notably reduce the crystal yield, and preferably the volume ratio with respect to the weight of the sodium salt of compound (1) is 15-40 fold (v/w) and more preferably 20-35 fold (v/w).

The temperature for dissolution of the sodium salt of compound (1) by heat may be appropriately selected as a temperature at which the sodium salt of compound (1) dissolves in the solvent, but preferably it is from the reflux temperature of the crystallization solvent to room temperature, and more preferably it is room temperature.

During crystallization, crystal formation occurs as the mixture stands while cooling or at the same temperature, but when crystallization is carried out while cooling, varying the cooling rate can produce crystals with different forms (polymorphic crystals), and therefore the cooling rate may be appropriately adjusted in consideration of its effects on crystal quality, particle size, etc.

The final crystallization temperature may be appropriately selected based on the salt yield and quality, but it is preferably 0-30˚C.

Seed crystals (a small amount of A-form crystals of the sodium salt of compound (1)) may be added for the crystallization, but their addition is not necessary.

The formed crystals are separated by an ordinary filtration procedure, and if necessary washed with solvent and dried to produce the desired crystals. The solvent used for crystal washing may be the same crystallization solvent, and preferably it is a mixture of ethanol and methyl t-butyl ether (1:9-1:10).

The crystals separated by the filtration procedure may be allowed to stand at room temperature under a nitrogen stream, or heated, for drying. The drying may also be carried out under reduced pressure. The degree of pressure

reduction may be appropriately selected depending on the production volume, the drying apparatus and the drying temperature.
The drying time may be appropriately selected as the time at which the residual solvent falls below a prescribed amount, and will depend on the production volume, the drying apparatus and the drying temperature.

[General production process (2) for A-form crystals of sodium salt]

The following procedures (A)-(I) may be appropriately selected and modified to produce A-form crystals of the sodium salt of compound (1).

(A) Compound (1) is mixed with a solvent (an alcohol-based solvent such as methanol or ethanol) to prepare a solution containing compound (1). The solution containing compound (1) may be the actual post-reaction treated extract obtained from production of compound (1) in the examples. That is, extraction is performed with a solvent (an alkylketone-based solvent such as methyl isobutyl ketone or an ester-based solvent such as ethyl acetate, or the like) during treatment after reaction for production of compound (1), and the obtained solution containing compound (1) may be used directly without isolation and purification of compound (1).
(B) A base (sodium hydroxide, a sodium alkoxide or the like, or an aqueous solution or alcohol solution thereof) for production of the sodium salt is added.
(C) The mixture is subjected to stirring and/or sonication.
(D) The solvent is distilled off from the mixture.
(E) Another solvent (an alkyl-based solvent such as hexane or heptane, a cycloalkyl-based solvent such as cyclohexane, an ether-based solvent such as diethyl ether, an ester-based solvent such as ethyl acetate, or the like) is added to the mixture.
(F) The mixture is cooled or allowed to stand at the same temperature.
(G) The precipitate in the mixture is collected by filtration.
(H) The obtained precipitate is washed.
(I) The precipitate is dried.

Compound (1) used for crystallization may be in any form, as a solvate or an anhydride, in amorphous or crystalline form, or in a mixture thereof. After production of compound (1), the extract obtained after reaction treatment may be used directly without isolation of compound (1).
The solvent used for crystallization may be one or a mixture of two or more solvents selected from the group consisting of ether-based solvents, ester-based solvents and alcohol-based solvents. As simple solvents there are preferred methyl t-butyl ether, methyl isobutyl ketone and ethyl acetate. As mixed solvents there are preferred 1-propanol and methyl t-butyl ether, 2-propanol and methyl t-butyl ether, 1-butanol and methyl t-butyl ether, methyl isobutyl ketone and methyl t-butyl ether or ethanol and methyl t-butyl ether.
The mixing ratio (volume ratio) for such mixed solvents is preferably 1:100-1:1 and more preferably 1:60-1:2.
The amount of solvent used may be appropriately selected, with a lower limit as the amount which allows the free acid to dissolve at room temperature and an upper limit as the amount which does not notably reduce the crystal yield, but preferably the volume ratio with respect to the weight of compound (1) is 5-50 fold (v/w) and more preferably 8-35 fold (v/w).
Seed crystals (a small amount of A-form crystals of compound (I)) may be added for the crystallization.
The formed crystals are separated by an ordinary filtration procedure, and if necessary washed with solvent and dried to produce the desired crystals. The solvent used for rinsing of the crystals is the same as the crystallization solvent, and is preferably a simple solution with methyl t-butyl ether, ethyl acetate or methyl isobutyl ketone, or a mixed solution with methyl t-butyl ether and methyl isobutyl ketone.
The crystals separated by the filtration procedure may be appropriately dried by standing under a nitrogen stream, or by heaving. The drying may be carried out in a nitrogen stream or under reduced pressure. The degree of pressure reduction may be appropriately selected depending on the production volume, the drying apparatus and the drying temperature.
The drying time may be appropriately selected as the time at which the residual solvent falls below a prescribed amount, and will depend on the production volume, the drying apparatus and the drying temperature.

[General production process for C-form crystals of sodium salt]

The following procedures (A)-(H) may be appropriately selected and modified to obtain C-form crystals of the sodium salt of compound (1). Specifically, for example, C-form crystals of the sodium salt of compound (1) may be produced with reference to the examples described below.

(A) Compound (1) and the solvent are mixed to prepare a solution containing compound (1). The solution containing compound (1) may be the actual post-reaction extract obtained from production of compound (1) in the examples. That is, extraction is performed with a solvent (toluene or a mixed solvent containing toluene) during treatment after reaction for production of compound (1), and the obtained solution containing compound (1) may be used directly without isolation and purification of compound (1).
(B) A base (sodium hydroxide, a sodium alkoxide or the like, or a mixture thereof) for production of the sodium salt is added.
(C) The mixture is subjected to heating and/or sonication.
(D) Another solvent (an alkyl-based solvent such as hexane or heptane, a cycloalkyl-based solvent such as cyclohexane, an ether-based solvent such as diethyl ether, or the like) is added to the mixture.
(E) The mixture is cooled or allowed to stand at the same temperature.
(F) The precipitate in the mixture is collected by filtration.
(G) The obtained precipitate is washed.
(H) The precipitate is dried.

The sodium salt of compound (1) used for crystallization may be in any form, as a solvate or an anhydride, in amorphous or crystalline form, or in a mixture thereof. After production of compound (1), the extract obtained after reaction treatment may be used directly without isolation of compound (1).
The solvent used for crystallization may be one or a mixture of two or more solvents selected from the group consisting of benzene-based solvents such as benzene and toluene, alkyl-based solvents such as hexane and heptane, cycloalkyl-based solvents such as cyclohexane, ether-based solvents and alcohol-based solvents, and is preferably a mixed solvent of toluene, methyl t-butyl ether and methanol. A small amount of an alcohol-based solvent (methanol or the like) may also be separately added.
The mixing ratio (volume ratio) for a mixed solvent of toluene and methyl t-butyl ether is preferably 2:1-1:2 and more preferably 9:6-7:9. The amount of methanol used (volume ratio) is preferably 0.2-0.6 and more preferably 0.3-0.5.
The amount of solvent used may be appropriately selected, with a lower limit as the amount which allows the free acid to dissolve at room temperature and an upper limit as the amount which does not notably reduce the crystal yield, but preferably the volume ratio with respect to the weight of compound (1) is 10-50 fold (v/w) and more preferably 20-35 fold (v/w).
Seed crystals (a small amount of C-form crystals of compound (1)) may be added for the crystallization.
The formed crystals are separated by an ordinary filtration procedure, and if necessary washed with solvent and dried to produce the desired crystals. The solvent used for crystal washing may be the same crystallization solvent, and preferably it is a mixture of toluene and methyl t-butyl ether (9:6-7:8).
The crystals separated by the filtration procedure may be appropriately dried by standing under a nitrogen stream, or by heating. The drying may be carried out in a nitrogen stream or under reduced pressure. The degree of pressure reduction may be appropriately selected depending on the production volume, the drying apparatus and the drying temperature.
The drying time may be appropriately selected as the time at which the residual solvent falls below a prescribed amount, and will depend on the production volume, the drying apparatus and the drying temperature.

<u>[General production process for amorphous sodium salt (freeze-drying method)]</u>

The following procedures (A)-(D) may be appropriately selected and modified to produce an amorphous sodium salt of compound (1). Specifically, for example, an amorphous sodium salt of compound (1) may be produced with reference to the examples described below (freeze-drying method).

(A) The sodium salt of compound (1) is mixed with a solvent.
(B) The mixture of the sodium salt of compound (1) and the solvent is subjected to heating and/or sonication.
(C) The mixture of the sodium salt of compound (1) and the solvent is cooled to solidification.
(D) The solvent is distilled off from the solidified mixture of the sodium salt of compound (1) and the solvent under reduced pressure (freeze-drying).

The sodium salt of compound (1) used for production may be in any form, as a solvate or an anhydride, in amorphous or crystalline form, or in a mixture thereof.
The solvent used may be one or a mixture of two solvents selected from the group consisting of alcohol-based solvents and water, and is preferably water.
The ratio (volume ratio) when using water is 3-20 fold (v/w) and preferably 5-10 fold (v/w) with respect to the weight of the sodium salt of compound (1).

The amorphous substance separated by the filtration procedure may be dried at room temperature or with heating.
The drying may also be carried out under reduced pressure. The degree of pressure reduction may be appropriately selected depending on the production volume, the drying apparatus and the drying temperature. The drying time may be appropriately selected as the time at which the residual solvent falls below a prescribed amount, and will depend on the production volume, the drying apparatus and the drying temperature.

[General production process for amorphous sodium salt of compound (1) (trituration method)]

Trituration of a sodium salt of compound (1) (for example, trituration using a powdering machine such as a mortar, ball mill or the like) can yield an amorphous sodium salt of compound (1). The sodium salt of compound (1) used for production may be in any form, as a solvate or an anhydride, in amorphous or crystalline form, or in a mixture thereof. Specifically, for example, an amorphous sodium salt of compound (1) may be produced with reference to the examples described below (trituration method).

[General production process for amorphous sodium salt of compound (1) (spray drying method)]

Spray drying of a sodium salt of compound (1) can yield an amorphous sodium salt of compound (1). The sodium salt of compound (1) used for production may be in any form, as a solvate or an anhydride, in amorphous or crystalline form, or in a mixture thereof. The solvent used may be water, an organic solvent or a mixture thereof that can dissolve the sodium salt of compound (1).
When the salt or crystals of the invention are used as a medicament, the salt or crystals of the invention will usually be combined with appropriate additives and formulated for use. However, this does not negate the use of the salts and crystals of the invention in simple forms as medicaments.
As additives there may be mentioned excipients, binders, lubricants, disintegrators, coloring agents, taste correctives, emulsifiers, surfactants, dissolving aids, suspending agents, isotonizing agents, buffering agents, antiseptic agents, antioxidants, stabilizers, absorption accelerators and the like which are commonly used in medicaments, and these may also be used in appropriate combinations as desired.
As examples of excipients there may be mentioned lactose, saccharose, glucose, corn starch, mannitol, sorbitol, starch, pregelatinized starch, dextrin, crystalline cellulose, light anhydrous silicic acid, aluminum silicate, calcium silicate, magnesium aluminometasilicate and calcium hydrogenphosphate.
As examples of binders there may be mentioned polyvinyl alcohol, methyl cellulose, ethyl cellulose, gum arabic, tragacanth, gelatin, shellac, hydroxypropylmethylcellulose, hydroxypropylcellulose, carboxymethylcellulose sodium, polyvinylpyrrolidone and macrogol.
As examples of lubricants there may be mentioned magnesium stearate, calcium stearate, sodium stearyl fumarate, talc, polyethylene glycol and colloidal silica.
As examples of disintegrators there may be mentioned crystalline cellulose, agar, gelatin, calcium carbonate, sodium hydrogencarbonate, calcium citrate, dextrin, pectin, low-substituted hydroxypropylcellulose, carboxymethylcellulose, carboxymethylcellulose calcium, croscarmellose sodium, carboxymethyl starch and carboxymethyl starch sodium.
As examples of coloring agents there may be mentioned those approved for addition to pharmaceuticals, such as iron sesquioxide, yellow iron sesquioxide, carmine, caramel, β-carotene, titanium oxide, talc, riboflavin sodium phosphate, yellow aluminum lake and the like.
As taste correctives there may be mentioned cocoa powder, menthol, aromatic powder, peppermint oil, camphor, cinnamon powder and the like.
As emulsifiers or surfactants there may be mentioned stearyltriethanolamine, sodium lauryl sulfate, laurylaminopropionic acid, lecithin, glycerin monostearate, sucrose fatty acid esters and glycerin fatty acid esters.
As dissolving aids there may be mentioned polyethylene glycol, propylene glycol, benzyl benzoate, ethanol, cholesterol, triethanolamine, sodium carbonate, sodium citrate, polysorbate 80 and nicotinic acid amide.
As suspending agents there may be mentioned the aforementioned surfactants, as well as hydrophilic polymers such as polyvinyl alcohol, polyvinylpyrrolidone, methylcellulose, hydroxymethylcellulose, hydroxyethylcellulose and hydroxypropylcellulose.
As isotonizing agents there may be mentioned glucose, sodium chloride, mannitol, sorbitol and the like.
As buffering agents there may be mentioned buffering solutions containing phosphoric acid salts, acetic acid salts, carbonic acid salts, citric acid salts and the like.
As antiseptic agents there may be mentioned methylparaben, propylparaben, chlorobutanol, benzyl alcohol, phenethyl alcohol, dehydroacetic acid and sorbic acid.
As antioxidants there may be mentioned sulfurous acid salts, ascorbic acid, α-tocopherol and the like.

As stabilizers there may be mentioned those commonly used in medicaments.

As absorption accelerators there may also be mentioned those commonly used in medicaments.

As formulations there may be mentioned oral forms such as tablets, powders, granules, capsules, syrups, lozenges and inhalants; external forms such as suppositories, ointments, eye salves, tapes, eye drops, nose drops, ear drops, poultices, lotions, and the like; and injections.

The aforementioned oral forms may be formulated with appropriate combinations of the aforementioned additives. Their surfaces may also be coated if necessary.

The aforementioned external forms may be formulated with appropriate combinations of the additives mentioned above, and especially excipients, binders, taste correctives, emulsifiers, surfactants, dissolving aids, suspending agents, isotonizing agents, antiseptic agents, antioxidants, stabilizers and absorption accelerators.

Injections may also be formulated with appropriate combinations of the additives mentioned above, and especially emulsifiers, surfactants, dissolving aids, suspending agents, isotonizing agents, buffering agents, antiseptic agents, antioxidants, stabilizers and absorption accelerators.

A formulation comprising a salt, crystalline form or amorphous form of compound (1) according to the invention to be used for treatment or prevention in humans may be obtained by a method commonly employed in pharmaceutics, or by the formulation examples described below.

When a compound of the invention is used as a medicament, the amount thereof used will differ depending on the symptoms and patient age, but normally it will be used at 0.15-5000 mg (preferably 0.5-1500 mg) for an oral form, 0.5-1500 mg (preferably 1.5-500 mg) for an external form and 0.3-5000 mg (preferably 1-500 mg) for an injection, either all at once or divided among 2-6 times per day. These values are the actual administered amounts in the case of oral formulations and injections, and are the amounts actually absorbed by the body in the case of external formulations.

The salt, crystalline form or amorphous form of compound (1) according to the invention may be produced by the methods described in the following examples, and the effects of the compounds may be confirmed by the methods described in the test examples described below. However, these specific examples are merely illustrative and are not intended to restrict the invention in any way, and various modifications may be implemented such as are within the scope of the invention.

The "room temperature" referred to in the Reference Examples and Examples is generally from about 10˚C to 35˚C. The percentage values are weight percentages, unless otherwise specified. The other symbols used in the examples stand for the following.

s: singlet
d: doublet
t: triplet
q: quartet
m: multiplet
br: broad
J: coupling constant
Hz: Hertz
$CDCl_3$: deutero chloroform
DMSO-$d_6$: deutero dimethylsulfoxide
$D_2O$: deuterated water
$CD_3OD$: deutero methanol
NMR: nuclear magnetic resonance

Examples

[Production Examples]

(Production Example 1) (2,2-dimethyl-1,3-dioxan-5-yl)methanol

[Chemical Formula 1]

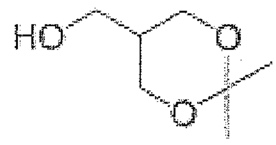

A mixture of 2-(hydroxymethyl)-1,3-propanediol (4.09 g, 38.5 mmol), acetone (130 ml, 1768 mmol) and 70% perchloric acid (1.37 g, 9.55 mmol) was stirred for 21 hours at room temperature. The pH of the reaction mixture was adjusted to 9 with concentrated aqueous ammonia and the reaction mixture was concentrated. The residue was purified by silica gel column chromatography (silica gel: 100 g, elution solvent: heptane, heptane/ethyl acetate= 1/3) to produce the title compound (4.83 g, yield: 85.8%) as a colorless oil.
$^{1}H$ NMR (400 MHz, DMSO-$d_6$) δ ppm; 1.29 (3H, s), 1.30 (3H, s), 1.64-1.74 (1H, m), 3.35-3.41 (2H, m), 3.61 (2H, dd, J=7, 12Hz), 3.82 (2H, dd, J=4, 12Hz), 4.54 (1H, t, J=5Hz).

(Production Example 2) 2,3,5-trimethylpyridine 1-oxide

[Chemical Formula 2]

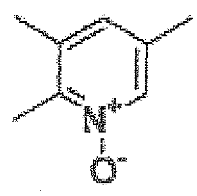

2,3,5-Trimethylpyridine (1.43 kg, 11.80 mol) was added to acetic acid (1.43 kg, 23.83 mol) over a period of 15 minutes. After 15 minutes, 35% aqueous hydrogen peroxide (1.38 kg, 14.2 mol) was added dropwise over a period of 30 minutes and then the mixture was stirred overnight at 90-95°C. Sodium sulfite (220 g) was poured into the reaction mixture. The reaction mixture was then poured into a mixture of sodium carbonate (2.5 kg) and water (12 L), and extraction was performed with chloroform (3.0 L x 4). The obtained organic layer was concentrated until precipitation of crystals, and then n-hexane (2.5 L) was added to the precipitate and the mixture was stirred overnight while cooling on ice. The obtained crystals were filtered to produce 1.53 kg of the target substance.

(Production Example 3) 2,3,5-trimethyl-4-nitropyridine 1-oxide

[Chemical Formula 3]

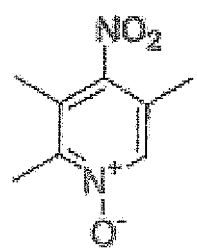

2,3,3-Trimethylpyridine 1-oxide (1.38 kg, 10.1 mol) was poured to 98% sulfuric acid (4.93 kg, 49.3 mol). After adding 97% nitric acid (1.44 kg) dropwise over a period of 50 minutes, the mixture was heated at 85˚C for 4 hours. The reaction mixture was poured into a mixture of ammonium hydrogencarbonate (10.6 kg) and water (9.0 L), and extraction was performed with ethyl acetate (3.0 L x 3). The obtained organic layer was concentrated and vacuum-dried overnight to produce 1.50 kg of the target substance.

(Production Example 4) 4-chloro-2,3,5-trimethylpyridine 1-oxide

[Chemical Formula 4]

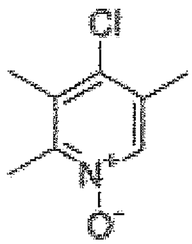

After adding water (400 g) and 36% concentrated hydrochloric acid (1.69 kg) to 2,3,5-trimethyl-4-nitropyridine 1-oxide (850 g, 4.67 mol), the mixture was heated at 70˚C. Next, N,N-dimethylformamide (115 mL) was added and the mixture was heated to 100˚C. After completion of the reaction, the mixture was cooled to 20˚C and poured into a mixture of potassium carbonate (1.40 kg) and water (7 L), extracted with chloroform (1.0 L x 3), dried over anhydrous sodium sulfate and then concentrated. The obtained crude product was stirred for 2 hours in a mixed solution of diisopropyl ether (500 mL) and n-hexane (1.0 L), and subjected to suction filtration. The obtained wet substance was vacuum-dried overnight to produce 666.4 g of the target substance.

(Production Example 5) 4-(2,2-dimethyl-1,3-dioxan-5-yl)methoxy-2,3,5-trimethylpyridine 1-oxide

[Chemical Formula 5]

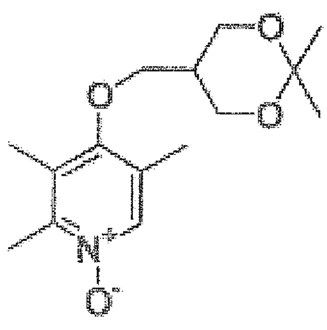

A mixture of 4-chloro-2,3,5-trimethylpyridine 1-oxide (840 g), (2,2-dimethyl-1,3-dioxan-5-yl)methanol (688 g) and toluene (2.52 L) was heated to reflux with removing the water. Potassium hydroxide (0.58 kg) was added over a period of 3 hours and 45 minutes while carrying out azeotropic dehydration, and azeotropic dehydration was continued for 2.5 hours. The reaction system was cooled to blow 30˚C, and then ethyl acetate (2.5 L) and a 17% aqueous solution of sodium chloride (3.5 L) were added and the mixture was allowed to stand overnight. The ethyl acetate layer was separated off and the aqueous layer was extracted with ethyl acetate (1.0 L x 3). The combined ethyl acetate layers were filtered through celite and then concentrated under reduced pressure to produce 1.20 kg of the target substance.

(Production Example 6) [4-(2,2-dimethyl-1,3-dioxan-5-yl)methoxy-3,5-dimethylpyridin-2-yl]methanol monohydrate

[Chemical Formula 6]

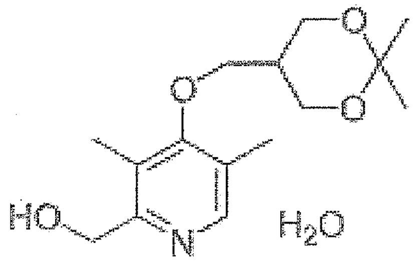

To a mixture of 4-(2,2-dimethyl-1,3-dioxan-5-yl)methoxy-2,3,5-trimethylpyridine 1-oxide (1.20 kg) and sodium acetate (0.18 kg) heated to 50˚C-60˚C was added dropwise acetic anhydride (1.10 kg) over a period of 1.5 hours. After 0.5 hour had passed, the mixture was heated at 80˚C for 4.5 hours, cooled to an internal temperature of below 30˚C and allowed to stand, and then concentrated under reduced pressure. The obtained residue was dissolved in methanol (1.0 L), and was added to a mixture of a 48% aqueous solution of sodium hydroxide (0.71 kg) and cold water (2.85 L) over a period of one hour. After stirring at room temperature for 5 hours and 45 minutes, the mixture was concentrated under reduced pressure. Water (3.0 L) was added to the concentration residue, extraction was performed with toluene (2.3 L x 4), and the combined toluene layers were washed with water (1.2 L). The obtained organic layer was filtered through celite and then concentrated. After adding diisopropyl ether (1.15 L) to the obtained residue at room temperature, warm water (45˚C, 74 mL) was further added. Upon confirming precipitation of crystals, stirring was carried out at 25˚C for 1 hour, heptane (3.6 L) was poured in and stirring was continued overnight. After further stirring for 5 hours while cooling on ice, the mixture was filtered to produce yellow crystals. Diisopropyl ether (3.5 L) was added to the obtained yellow crystals for dissolution at 50˚C. After removing the insoluble portion by filtration, it was slowly cooled and aged overnight at 5˚C. The obtained crystals were filtered and washed with heptane (0.5 L), and then dried under open air to produce 0.69 kg of the target substance.

(Production Example 7) 2-({[4-(2,2-dimethyl-1,3-dioxan-5-yl)methoxy-3,5-dimethylpyridin-2-yl]methyl}thio)-1H-benzimidazole

[Chemical Formula 7]

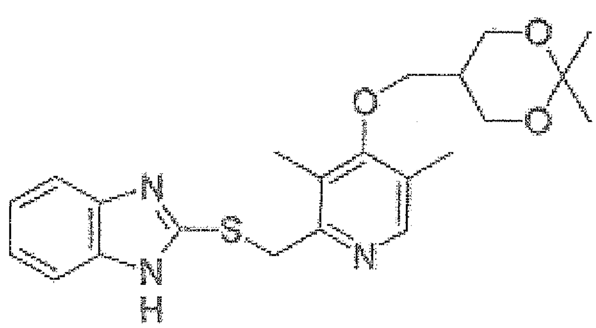

Toluene was added to [4-(2,2-dimethyl-1,3-dioxan-5-yl)methoxy-3,5-dimethylpyridin-2-yl]methanol monohydrate (690 g), and azeotropic dehydration was performed (2.1 L x 5, 1.75 L x 1). Toluene (393 mL) was added to the obtained concentrate to produce 921 g of a toluene solution of [4-(2,2-dimethyl-1,3-dioxan-5-yl)methoxy-3,5-dimethylpyridin-2-yl]methanol.
There were poured the toluene solution of [4-(2,2-dimethyl-1,3-dioxan-5-yl)methoxy-3,5-dimethylpyridin-2-yl]methanol (845.7 g, content: 61.7%, amount: 521.8 g, 1.855 mol), tetrahydrofuran (2609 mL), toluene (669 mL) and triethylamine (375.3 g, 3.709 mol) in that order under a nitrogen atmosphere, and the mixture was stirred while cooling with dry ice/ethanol. At 30 minutes after the start of cooling, methanesulfonyl chloride (254.9 g, 2.226 mol) was added dropwise over a period of 42 minutes. Upon completion of the dropwise addition, the mixture was stirred while cooling in an ice-water bath. After approximately 1.5 hours, a solution of 2-mercaptobenzimidazole (334.28 g, 2.226 mol) in tetrahydrofuran (3653 mL) was poured in for 2 minutes and stirring was continued at room temperature for approximately 18 hours.
After pouring toluene (3653 mL) into the reaction mixture, a 20% w/w aqueous solution of sodium hydroxide (1852.4 g) was added, and $H_2O$ (2322 mL) was further added prior to extraction and separation. The organic layer was washed twice with a 20% w/w aqueous solution of ammonium chloride (4174 g), and then further washed with $H_2O$ (4147 mL). The obtained organic layer was concentrated under reduced pressure (40˚C) to produce a light brown oil (2.40 kg, containing 1446 mL toluene, 168 mL tetrahydrofuran, calculated from $^1$H-NMR spectrum).
The obtained light brown oil was transferred to a crystallization vessel and washed with toluene (119 mL), and then stirred at room temperature. After 10 minutes, tert-butyl methyl ether (134 mL) was poured in and stirring at room temperature was continued. After 20 minutes, tert-butyl methyl ether (127 mL) was poured in and stirring at room temperature was continued. After 30 minutes, tert-butyl methyl ether (266 mL) was added dropwise over a period of 20 minutes and stirring at room temperature was continued. After one minute, tert-butyl methyl ether (522 mL) was again added dropwise, and 8 minutes thereafter precipitation of crystals was confirmed and the dropwise addition was completed at a total of 1 hour and 20 minutes. After stirring at room temperature for 40 minutes, heptane (2348 mL) was added dropwise over a period of 1 hour and 17 minutes, and the mixture was stirred overnight at room temperature.
About 15.5 hours after heptane was added, the precipitated crystals were suction filtered, washed with toluene/tert-butyl methyl ether/heptane (587 mL/391 mL/587 mL) and then suction dried. The obtained wet crystals were dried with forced air (50˚C) to produce the target substance.
Yield: 619.0 g, content: 96.5%, amount: 597.3 g, percentage yield: 77.8% (based on content), HPLC purity: 98.0%

<HPLC analysis conditions (reaction check, HPLC purity measurement and quantitation)>

Column: YMC-Pack Pro C18 AS-302 (5 μm, 4.6 mm I.D. x 150 mm I.D.)
Eluent: Solution A (acetonitrile/20 mM ammonium acetate aq. = 100/900 (v/v)),
Solution B (acetonitrile /20 mM ammonium acetate aq. = 800/200 (v/v))
Flow rate: 1.0 mL/min
Detection: UV 254 nm
Oven temp.: 25 ˚C
Sample temp.: 25˚C
Gradient condition (time/solution B conc.): 0.01 min/0%, 25 min/100%, 30 min/100%, 30.01 min/0%, 40 min/stop

[Examples]

Throughout the following examples, 2-({[4-(2,2-dimethyl-1,3-dioxan-5-yl)methoxy-3,5-dimethylpyridin-2-yl]methyl}thio)-1H-benzimidazole (alternate name: 2-[4-(2,2-dimethyl-1,3-dioxan-5-ylmethoxy)-3,5-dimethylpyridin-2-yl-methyl-sulfanyl]-1*H*-benzoimidazole) will also be referred to as "compound (2)"; (R)-2-({[4-(2,2-dimethyl-1,3-dioxan-5-yl)methoxy-3,5-dimethylpyridin-2-yl]methyl}sulfinyl)-1H-benzimidazole (alternate name: 2-[4-(2,2-dimethyl-1,3-dioxan-5-yl-methoxy)-3,5-dimethylpyridin-2-yl-methane-sulfinyl]-1*H*-benzoimidazole) will also be referred to as "compound (3)"; and (R)-2-({[4-(2,2-dimethyl-1,3-dioxan-5-yl)methoxy-3,5-dimethylpyridin-2-yl]methyl}sulfinyl)-1H-benzimidazole sodium salt (alternate name: (+)-sodium 2-[4-(2,2-dimethyl-1,3-dioxan-5-yl)methoxy-3,5-dimethylpyridin-2-yl]-methylsulfinyl-1*H*-benzimidazole) will also be referred to as "compound (4)". Throughout the present specification, compound (4) may be any crystalline form or amorphous form of compound (4), or a mixture thereof, unless otherwise specified.

<u>Example 1X Sodium salt of (R)-2-({[4-(2,2-dimethyl-1,3-dioxan-5-yl)methoxy-3,5-dimethylpyridin-2-yl]methyl}sulfinyl)-1H-benzimidazole (C-form crystals of sodium salt)</u>

There were poured 2-({[4-(2,2-dimethyl-1,3-dioxan-5-yl)methoxy-3,5-dimethylpyridin-2-yl]methyl}thio)-1H-benzimidazole (compound (2)) (580.3 g, content: 96.5%, amount: 560.0 g, 1.354 mol), toluene (3864 mL), $H_2O$ (2.81 g, 0.156 mol) under a nitrogen atmosphere, and the mixture was stirred while heating at 60˚C. After 6 minutes, L-(+)-diethyl tartrate (122.9 g, 0.596 mol) was poured into the suspension and washing was performed with toluene (560 mL). After

30 minutes, dissolution was confirmed. After 8 minutes, titanium(IV) tetraisopropoxide (77.0 g, 0.271 mol) was poured in, washing was performed with toluene (56 mL) and heated stirring was carried out for about one hour at the same temperature. The temperature was changed to 8˚C for cooling, N,N-diisopropylethylamine (56.01 g, 0.742 mol) was poured in, and washing was performed with toluene (280 mL). After 10 minutes, a solution of cumene hydroperoxide (259.2 g, 1.422 mol) in toluene (840 mL) was added dropwise over a period of 47 minutes, and the mixture was stirred at 8˚C for approximately 18.5 hours thereafter. A cooled aqueous solution of 30% w/w sodium thiosulfate (2240 g) was poured therein, stirring was continued for 12 minutes, and the aqueous layer was discarded. After then pouring a 4% w/w aqueous solution of sodium hydroxide (2240 g) into the organic layer, the mixture was stirred and allowed to stand, the aqueous layer was separated off, and a aqueous sodium hydroxide extract of (R)-2-({[4-(2,2-dimethyl-1,3-dioxan-5-yl)methoxy-3,5-dimethylpyridin-2-yl]methyl}sulfinyl)-1H-benzimidazole (compound (3)) was obtained as a brownish yellow suspension. The aqueous sodium hydroxide extract containing compound (3) (2.98 kg) was poured into toluene (7840 mL) and the mixture was stirred. Next, a 20% w/w aqueous solution of acetic acid (400 mL), an 8% aqueous solution of NaOH (50 mL) and a 20% w/w aqueous solution of acetic acid (8 mL) were poured into the mixture in that order while stirring for adjustment to pH 8.64, the mixture was allowed to stand for separation, and the aqueous layer was discarded. The organic layer was washed with 5% w/w aqueous solution of sodium chloride (2240 g) and separated to produce a toluene extract containing compound (3) (7.31 kg, compound (3) content: 567.7 g, 1.322 mol) as a brownish yellow solution.

A 28.3% sodium methoxide-methanol solution (245.6 g, 1.286 mol) was poured into the obtained toluene extract for one minute while stirring at room temperature. Next, tert-butyl methyl ether (1120 mL) was added dropwise to the solution over a period of 3 minutes, the mixture was stirred at room temperature, precipitation of crystals was confirmed after 6 minutes, and stirring was continued for approximately 30 minutes. Then tert-butyl methyl ether (7840 mL) was added dropwise over a period of 2 hours and 40 minutes, and stirring was continued overnight at room temperature.

At about 13 hours after tert-butyl methyl ether was added dropwise, the precipitated crystals were suction filtered and washed with toluene/tert-butyl methyl ether (1047 mL/1193 mL), and suction drying was carried out for 15 minutes. The obtained wet crystals were dried under reduced pressure (40˚C) to produce crystals of the title compound (hereinafter, "C-form crystals of sodium salt"). Yield: 546.8 g, content: 101.7%, amount: 546.8 g (at 100% content), percentage yield: 90.9% (based on yield), HPLC purity: 98.2%, enantiomeric excess: ≥99.5% ee

<HPLC analysis conditions (reaction check, HPLC purity measurement and quantitation)>

Column: YMC-Pack Pro C18 AS-302 (5 μm, 4.6 mm I.D. x 150 mm I.D.)
Eluent: Solution A (acetonitrile/20 mM ammonium acetate aq. = 100/900 (v/v)),
Solution B (acetonitrile /20 mM ammonium acetate aq. = 800/200 (vlv))
Flow rate: 1.0 mL/min
Detection: UV 254 nm
Oven temp.: 25˚C
Sample temp.: 25˚C
Gradient condition (time/solution B conc.): 0.01 min/0%, 25 min/100%, 30 min/100%, 30.01 min/0%, 40 min/stop
<HPLC analysis conditions (enantiomeric excess)>
Column: DAICEL CHIRALPAK IA (4.6 mm I.D. x 250 mm I.D.)
Eluent: etbanol/tert-butyl methyl ether = 1501/850 (v/v)
Flow rate: 1.0 mL/min
Detection: UV 284 nm
Oven temp.: 25˚C
Sample temp.: 25˚C

The aforementioned "(R)-2-({[4-(2,2-dimethyl-1,3-dioxan-5-yl)mehoxy-3,5-dimethylpyridin-2-yl]methyl}sufinyl)-1H-benzimidazole sodium salt" was the earlier eluting optically active compound according to analysis under the aforementioned HPLC analysis conditions (enantiomerie excess).

<u>Example 2X Sodium salt of (R)-2-({[4-(2,2-dimethyl-1,3-dioxan-5-yl)methoxy-3,5-dimethy]pyridin-2-yl]methyl}sulfinyl)-1H-benzimidazole (A-form crystals of sodium salt)</u>

Ethanol (18.30 kg) was added to the sodium salt of 2-({[4-(2,2-dimethyl-1,3-dioxan-5-yl)methoxy-3,5-dimethylpyridin-2-yl]methyl}sulfinyl)-1H-benzimidazole (C-form crystals of sodium salt as in Example 1X, 12.41 kg, net 11.6 kg), and then tert-butyl methyl ether (17.20 kg) was added.

The mixture was filtered through a Hyflo Super-cel (product of Wako Pure Chemical Industries, Ltd., Code: 534-02315) [2.30 kg, pre-washed with a mixture of ethanol (13.70 kg) and tert-butyl methyl ether (12.90 kg), and then washed with tert-butyl methyl ether (17.20 kg)], and the Hyflo Super-eel was washed with a mixture of ethanol (4.60 L) and tert-butyl methyl ether (4.60 L).
The combined filtrate was filtered and then washed with a mixture of ethanol (1.2 L) and tert-butyl methyl ether (1.2 L). Next, tert-butyl methyl ether (25.80 kg) was added dropwise to the combined filtrate while stirring at 20˚C for a period of 14 minutes. The mixture was stirred for 1 hour and 1 minute, and then tert-butyl methyl ether (145.93 kg) was added dropwise over a period of 2 hours and 46 minutes prior to stirring at 20˚C for 17 hours and 45 minutes.
The precipitate produced in the mixture was filtered out and washed with a mixture of ethanol (2.30 L) and tert-butyl methyl ether (15.50 kg) and then with tert-butyl methyl ether (17.23 kg), after which it was dried under reduced pressure at 50˚C for approximately 36 hours and passed through a sieve (1.00 mm mesh) to produce white crystals of the title compound (hereinafter, "A-form crystals of sodium salt") (10.02 kg).

HPLC PURITY: 99.2%, percentage content: 97.6%, enantiomeric excess: ≥99.5%

<HPLC conditions (reaction check, HPLC purity measurement and quantitation)>

Column: YMC-Pack Pro C18 AS-302, (5 μm, 4.6 mm I.D. x 150 mm I.D.)
Mobile phase:
A: Acetonitrile/20 mM ammonium acetate aq. = 100/900 (v/v)
B: Acetonitrile/20 mM ammonium acetate aq. = 800/200 (v/v)
Time (min.): B Conc., 0.01:0, 25.00:100, 30.00:100, 30.01:0 and 35.00: stop
Detection: 254 nm
Flow rate: 1.0 mL/min
Oven: 25˚C
Measuring time: 40 min
<HPLC conditions (enantiomeric excess)>
Column: CHIRALPAK IA, 4.6 mm I.D. x 250 mm I.D.
Mobile phase: tert-butyl methyl ether/ethanol = 85/15 (v/v)
Detection: 284 nm
Flow rate: 1.0 mL/min
Oven: 25˚C
Measuring time: 30 min

<u>Example 3X (trituration method) Sodium salt of (R)-2-({[4-(22-dimethyl-1,3-dioxan-5-yl)methoxy-3,5-dimethylpyridin-2-yl]methyl}sulfinyl)-1H-benzimidazole (amorphous form of sodium salt)</u>

The sodium salt of 2-({[4-(2,2-dimethyl-1,3-dioxan-5-yl)methoxy-3,5-dimethylpyridin-2-yl]methyl}sulfinyl)-1H-benzimidazole (compound (4): A-form crystals of sodium salt) obtained in Example 2X was triturated with a mortar for approximately 40 minutes under a nitrogen stream to produce an amorphous form of the title compound.

<u>Example 4X Sodium salt of (R)-2-({[4-(2,2-dimethyl-1,3-dioxan-5-yl)methoxy-3,5-dimethylpyridin-2-yl]methyl}sulfinyl)-1H-benzimidazole (B-form crystals of sodium salt)</u>

Compound (4) (A-form crystals of sodium salt, 1.5 g) was placed in a round-bottomed flask and heated for 2 hours at 60˚C (external temperature) in an evaporator with intermittent rotation (1 min/pause: 3sec/rotation) under reduced pressure (1 mmHg), and then sealed and kept overnight between 23˚C and 25˚C at ordinary pressure.
Compound (4) was then, in the same manner, heated for 2 hours at 80˚C (external temperature) in an evaporator with intermittent rotation (1 min/pause: 3sec/rotation) under reduced pressure (1 mmHg), subsequently heated for 6 hours at 100˚C (external temperature) under reduced pressure (1 mmHg), and then sealed and kept overnight between 23˚C and 25˚C at ordinary pressure.
Next, compound (4) was, in the same manner, heated for 4 hours at 100˚C (external temperature) in an evaporator with intermittent rotation (1 min/pause: 3sec/rotation) under reduced pressure (1 mmHg), to produce crystals of the title compound (hereinafter, "B-form crystals of sodium salt").

<HPLC conditions (reaction check, HPLC purity measurement and quantitation>

Solid phase: YMC-Pack Pro C18 AS-302(4.6 mm I.D. x 150 mm I.D., 5 μm)
Mobile phase:
Solution A: Acetonitrile:20 mM ammonium acetate aq.=200:1800 (v:v)
Solution B: Acetonitrile:20 mM ammonium acetate aq.=1600:400(v:v)
Flow rate: 1.0 mL/min
Temperature: 25°C
Detector: UV 254 nm
Gradient conditions: time (min)/solution B concentration (%), 0.01/0, 25/100, 30/100, 30.01/0 and 35/stop

Example 5X Sodium salt of (R)-2-({[4-(2,2-dimethyl-1,3-dioxan-5-yl)methoxy-3,5-dimethylpyridin-2-yl]methyl}sulfinyl)-1H-benzimidazole (D-form crystals of sodium salt)

To compound (4) (A-form crystals of sodium salt) (1.31 g) were added tert-butyl methyl ether (26 mL) and ethanol (1.3 mL). Water (209 mg) was then added to the mixture while stirring at room temperature, ethanol (0.65 mL) was added, water (52 mg) was added, ethanol (0.65 mL) was added, and the mixture was stirred for 2 hours and 29 minutes. The precipitate of the mixture was collected by filtration and then dried under reduced pressure to produce crystals of the title compound (hereinafter, "D-form crystals of sodium salt") (857 mg, white solid).

Example 6X Sodium salt of (R)-2({[4-(2,2-dimethyl-13-dioxan-5-yl)methoxy-3,5-dimethylpyridin-2-yl]methyl}sulfinyl)-1H-benzimidazole (G-form crystals of sodium salt)

To compound (2) (520 mg) were added L-(+)-diethyl tartrate (95 μL) and toluene (2.60 mL) under a nitrogen atmosphere, and the mixture was heated at 57°C. After adding titanium(IV) isopropoxide (73.7 μL) to the reaction mixture and heating and stirring at the same temperature for one hour, the reaction mixture was cooled to 33.5°C. Next, N,N-diisopropylethylamine (69.9 μL) was added to the reaction mixture and then cumene hydroperoxide (80%, 232 μL) was added over a period of at least 5 minutes, after which the mixture was stirred at room temperature for 17 hours.
A 30% aqueous solution of sodium thiosulfate (2.0 mL) was then added to the reaction mixture at room temperature, and after adequate stirring, the organic layer was separated off. Next, a 1N aqueous solution of sodium hydroxide (2.0 mL) was added to the organic layer, and after adequate stirring, the aqueous layer was separated off. Methyl isobutyl ketone (hereinafter, "MIBK") (2.0 mL) was added to the obtained aqueous layer, and 20% acetic acid water was slowly added while stirring until pH reached 9.71. The organic layer was separated off, and then a suspension of sodium tert-pentoxide (146 mg) in MIBK (1.40 mL) was added dropwise to the organic layer and the mixture was stirred for 15 hours and 50 minutes. MIBK (2.0 mL) was further added to the mixture and the precipitate was collected by filtration to produce crystals of the title compound (hereinafter, "G-form crystals of sodium salt") (328 mg, white solid).

Example 7X Sodium salt of (R)2-({[4-(2,2-dimethyl-,3-dioxan-5-yl)methoxy-3,5-dimethylpyridin-2-yl]methyl}sulfinyl)-1H-benzimidazole (H-form crystals of sodium salt)

Compound (4) (G-forin crystals of sodium salt) obtained in Example 6X was kept at approximately 60°C for one week to obtain crystals of the title compound (hereinafter, "H-form crystals of sodium salt").

Example 8X Sodium salt of (R)-2-({[4-(2,2-dimehyl1,3-dioxan-5-yl)methoxy-3,5-dimethylpyridin-2-yl]methyl}sulfinyl)-1H-benzimidazole (E-form crystals of sodium salt)

A solution of L-(+)-diethyl tartrate (550 mg) in toluene (2.5 mL) was added to a mixture of compound (2) (2.5 g) and toluene (15 mL). The mixture was heated at 50°C and stirred, titanium(IV) isopropoxide (344 mg) and toluene (2.5 mL) were added, and the mixture was heated and stirred for 1 hour and 10 minutes.
N,N-Diisopropylethylamine (337 μL) was then added to the reaction mixture at 19.8°C (internal temperature). Cumene hydroperoxide (80%, 1.15 g) and toluene (5.0 mL) at -5°C (external temperature) were added over a period of 5 minutes, at -0.2°C to -3.1°C.
The reaction mixture was then warmed to 20°C while stirring and kept at 20°C for 4 hours and 40 minutes, after which it was stirred at -1.1°C to 0.3°C for 15 hours.
The reaction mixture was cooled to -5°C (external temperature), 30% aqueous sodium thiosulfate (10 g) was added, the mixture was adequately stirred and the organic layer was separated off. A 1N aqueous solution of sodium hydroxide

(10 mL) was added to the organic layer, and after adequate stirring, the aqueous layer was separated off. MIBK (20 mL) was added to the obtained aqueous layer, and a 20% aqueous solution of acetic acid was slowly added while stirring until pH reached 7.3. After adequately stirring the mixture, the organic layer was separated off and a solution of sodium tert-pentoxide (701 mg) in toluene (7 mL) was added dropwise to the organic layer. Next, seed crystals (compound (4) (D-form crystals of sodium salt)) were added to the mixture, the mixture was stirred for 21 hours, and tert-butyl methyl ether (20 mL) was added. The precipitate produced in the mixture was collected by filtration. The obtained precipitate was washed with a mixed solution (5 mL) comprising tert-butyl methyl ether and MIBK (3:7), and then dried under a nitrogen stream to produce light yellow crystals (D-form crystals of sodium salt) (1.9 g).
The light yellow crystals were kept at 60˚C for 10 days to produce crystals of the title compound (hereinafter, "E-form crystals of sodium salt").

Example 9X (freeze-drying method) Sodium salt of (R)-2-({[4-(2,2-dimethyl-1,3-dioxan-5-yl)methoxy-3,5-dimethylpyridin-2-yl]methyl}sulfinyl)-1H-benzimidazole (amorphous form of sodium salt)

Sodium salt of 2-({[4-(2,2-dimethyl-1,3-dioxan-5-yl)methoxy-3,5-dimethylpyridin-2-yl]methyl}sulfinyl)-1H-benzimidazole (compound (4): A-form crystals of sodium salt) (1.52 g) was dissolved in distilled water (10 mL), and the solvent was distilled off under reduced pressure. Distilled water (4 mL) was added to the obtained residue (4.5 mL), and the solution was frozen in a dry ice-acetone bath. The solvent was distilled off from the frozen solution for 14 hours and 46 minutes at room temperature under reduced pressure to produce an amorphous form of the title compound (1,43 g, yellowish white solid).

Example 10X Sodium salt of (R)-2-({[4-(2.2-dimethyl-1,3-dioxan-5-yl)methoxy-3,5-dimethylpyridin-2-yl]methyl}sylfinyl)-1H-benzimidazole (F-form crystals of sodium salt)

Ethanol (8 mL) was added to compound (4) (4 g) while cooling in an ice bath, and then a 1N aqueous sodium hydroxide solution (9.31 mL) was added dropwise over a period of 10 minutes. Ethanol (4 mL) was added to the mixture and the solvent was distilled off under reduced pressure. Ethanol (8 mL) was then further added to the mixture and the solvent was distilled off under reduced pressure. Ethanol (8 mL) was still further added to the mixture and the solvent was distilled off under reduced pressure. Ethyl acetate (80 mL) was added to the obtained residue, and then seed crystals (compound (4) (F-form crystals of sodium salt)) were added into the mixture which was then allowed to stand at room temperature for 14 hours and at 4˚C for 25 hours. The precipitate produced in the mixture was filtered and washed with ethyl acetate (30 mL) to produce crystals of the title compound (hereinafter, "F-form crystals of sodium salt") (1.097 g. light yellow crystals).

Example 1Y (R)-2-({[4-22-dimethyl-1,3-dioxan-5-yl)-methoxy-3,5-dimethylpyridin-2-yl]methyl}sulfinyl)-1H-benzimidazole (A-form crystals of free acid)

To a mixture of compound (2) (10 g) and toluene (50 mL) were added L-(+)-dimethyl tartrate (1.83 mL) and water (52.5 $\mu$L) at room temperature under a nitrogen atmosphere, and the mixture was heated and stirred at 56˚C. Titanium(IV) isopropoxide (1.42 mL) was added to the reaction mixture at the same temperature, and the mixture was stirred at 50˚C to 60˚C for 1 hour. The reaction mixture was cooled, and then N,N-diisopropylethylamine (1.35 mL) was added at 28˚C. The reaction mixture was cooled to -3˚C, and then cumene hydroperoxide (80%, 4.47 mL) was added at -1.0˚C to 0.6˚C over a period of 20 minutes, after which the reaction mixture was stirred at -1˚C to 1˚C for 21 hours and 35 minutes.
A 30% aqueous sodium thiosulfate solution (38.4 mL) was added to the reaction mixture at 0.6˚C to 2.7˚C; and after adequate stirring, the organic layer was separated off. A 1N aqueous solution of sodium hydroxide (38.4 mL) was added to the organic layer, and after adequate stirring, the aqueous layer was separated off. To the obtained aqueous layer was added tert-butyl methyl ether (38.4 mL), and then a 20% aqueous solution of acetic acid was slowly added while stirring until the pH reached 9.87.
The top layer (organic layer 1) of the three separated layers was separated off. After adding tert-butyl methyl ether (40 mL) to the lower two layers, a 20% aqueous solution of acetic acid was slowly added while stirring until the pH reached 9.17. The upper layer (organic layer 2) was separated off, ethyl acetate (30 mL) was added to the remaining aqueous layer, the mixture was adequately stirred, and then the organic layer (organic layer 3) was separated off. Organic layers 1 to 3 were combined, dried over anhydrous magnesium sulfate (15 g) and filtered, and the solvent was distilled off from the filtrate under reduced pressure. The obtained residue (9.9 g) was purified by silica gel column chromatography (NH silica gel, developing solvent: ethyl acetate/methanol mixture (10:0, 9: 1)) to produce crystals of the title compound (hereinafter, "A-form crystals of free acid") (7.4 g, white crystals).

Example 2Y (R)-2-({[4-(2,2-dimethyl-1,3-dioxan-5-yl)methoxy-3,5-dimethylpyridin-2-yl]methyl}sulfinyl)-1H-benzimidazole (B-form crystals of free acid)

To a mixture of compound (2) (500 mg), toluene (2.5 mL) and water (2.62 μL) was added L-(+)-diethyl tartrate (91 μL), and the mixture was heated and stirred at 50°C. Titanium(IV) isopropoxide (71.5 μL) was added to the reaction mixture, and heating and stirring were continued for 1 hour and 20 minutes.
Next, N,N-diisopmpylethylamine (225 μL) was added to the reaction mixture while stirring at -2°C, and then cumene hydroperoxide (80%, 224 μL) was added at -3.3°C to -2.2°C.
The reaction mixture was stirred for 17 hours and 20 minutes, after which a 30% aqueous solution of sodium thiosulfate (2 mL) was added, the mixture was adequately stirred, and the organic layer was separated off. A 1N aqueous solution of sodium hydroxide (2 mL) was added to the organic layer, and after adequate stirring, the aqueous layer was separated off. To the obtained aqueous layer was added tert-butyl methyl ether (2 mL), and then a 20% aqueous solution of acetic acid was slowly added while stirring until the pH reached 8.5. After adequately stirring the mixture, the organic layer was separated off, and then seed crystals (compound (3)) were added to the organic layer and the mixture was stirred at room temperature for 3 hours. The precipitate produced in the mixture was collected by filtration, and washed with tert-butyl methyl ether (2 mL) to produce crystals of the title compound (hereinafter, "B-form crystals of free acid") (318 mg).

Example 3Y (R)-2-({[4-(2,2-dimethyl-1,3-dioxan-5-yl)methoxy-3,5-dimethylpyridin-2-yl]methyl}sulfinyl)-1H-benzimidazole (amorphous form of free acid)

After dissolving 2-({[4-(2,2-dimethyl-1,3-dioxan-5-yl)methoxy-3,5-dimethylpyridin-2-yl]methyl}sulfinyl)-1H-benzimidazole (320 mg) in a mixture of t-butyl alcohol (7 mL) and distilled water (5 mL), the solution was frozen in a dry ice-methanol bath. The solvent was distilled off from the frozen solution for 21 hours and 10 minutes at room temperature under reduced pressure to produce a solid. The obtained solid was dried for 3 days at room temperature under reduced pressure to produce an amorphous form of the title compound (324 mg, white powder).

Example 1Z Potassium salt of (R)-2-({[4-(2,2-dimethyl-1,3-dioxan-5-yl)methoxy-3,5-dimethylpyridin-2-yl]methyl}sulfinyl)-1H-benzimidazole

To a solution of compound (3) (100 mg) in toluene (0.36 mL) was added a solution of potassium methoxide (24.5 mg) in methanol (0.24 mL), and the mixture was stirred at room temperature for 73 hours and 28 minutes. The precipitate produced in the mixture was collected by filtration. The precipitate was washed with a mixed solution (0.4 mL) of toluene and methanol (3:2) and dried to produce the title compound (84 mg).

Example 2Z Magnesium salt of (R)-2-({[4-(2,2-dimethyl-1,3-dioxan-5-yl)methoxy-3,5-dimethylpyridin-2-yl]methyl}sulfinyl)-1H-benzimidazole

Magnesium methoxide (6%, 229 μL) was added to a solution of compound (3) (112 mg) in methanol (250 μl) at room temperature, and the mixture was stirred. After adding tert-butyl methyl ether (4.0 mL) and stirring for 1 hour and 25 minutes, the precipitate produced in the mixture was collected by filtration. The precipitate was washed with a mixed solution (approximately 3 mL) of tert-butyl methyl ether and methanol (9:1) and dried under reduced pressure to produce the title compound (78 mg, white solid).

Example 3Z Lithium salt of (R)-2-({[4-(2,2-dimethyl-1,3-dioxan-5-yl)methoxy-3,5-dimethylpyridin-2-yl]methyl}sulfinyl)-1H-benzimidazole

Compound (3) (156 mg) was added to a solution of lithium methoxide (18.3 mg) in ethanol (1 mL), and the mixture was shaken. The mixture was filtered and t-butyl methyl ether (10 mL) was added to the filtrate. The precipitate produced in the mixture was suction filtered, and the precipitate was then washed with t-butyl methyl ether (1 mL). The obtained precipitate was dried overnight under reduced pressure to produce the title compound (white solid, 53 mg).

Example 4Z Zinc salt of (R)-2-({[4-(2,2-dimethyl-1,3-dioxan-5-yl)methoxy-3,5-dimethylpyridin-2-yl]methyl}sulfinyl)-1H-benzimidazole

Compound (4) (468 mg) was added to a mixture of zinc chloride (608 mg) and purified water (1 mL), and the mixture was shaken. Purified water (2 mL) was then added to the mixture and it was further shaken with sonication (bath-type sonicator). The precipitate produced in the mixture was suction filtered and the precipitate was washed with purified water (2 mL). The obtained precipitate was dried overnight under reduced pressure to produce the title compound

(light yellow solid, 458 mg).

Example 5Z Calcium salt of (R)-2-({[4-(2,2-dimethyl-1,3-dioxan-5-yl)methoxy-3,5-dimethylpyridin-2-yl]methyl}sulfinyl)-1H-benzimidazole

Compound (4) (526 mg) was added to a mixture of calcium nitrate (892 mg) and purified water (1 mL), and the mixture was shaken. Purified water (3 mL) was then added to the mixture and it was further shaken with sonication (bath-type sonicator). The precipitate produced in the mixture was suction filtered and the precipitate was washed with purified water (1 mL). The obtained precipitate was dried overnight under reduced pressure to produce the title compound (white solid, 430 mg).

Example 1Q Sodium salt of (R)-2-({[4-(2,2-dimethyl-1,3-dioxan-5-yl)methoxy-3,5-dimethylpyridin-2-yl]methy6l}sulfinyl)-1H-benzimidazole (A-form crystals of sodium salt)

[Chemical Formula 8]

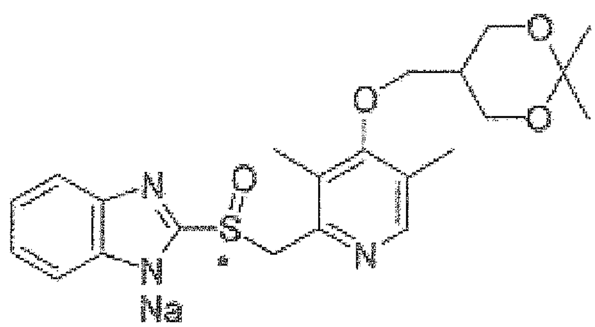

Ethanol (1074 mL) was added to the sodium salt of 2-({[4-(2,2-dimethyl-1,3-dioxan-5-yl)methoxy-3,5-dimethylpyridin-2-yl]methyl}sulfinyl)-1H-benzimidazole (compound (4): C-form crystals of sodium salt) (536.8 g, 1.189 mol) obtained in Example 1X for dissolution at room temperature, and then tert-butyl methyl ether (1074 mL) was further added. The obtained solution was suction filtered through a Hyflo Super-cel [107.4 g, pre-washed with a mixture of ethanol/tert-butyl methyl ether (1074 mL/1074 mL) and tert-butyl methyl ether (537 mL) in that order], and then the Hyflo Super-cel was washed with ethartol/tert-butyl methyl ether (215 mL/215 mL).
The obtained filtrate was transferred to a crystallization vessel and washed with ethanol/tert-butyl methyl ether (54 mL/54 mL), and then stirring was commenced at room temperature. After adding tert-butyl methyl ether (1610 mL) dropwise for a period of 6 minutes, stirring at room temperature was continued. After 11 minutes, tert-butyl methyl ether (268 mL) was added dropwise over a period of 2 minutes, stirring was continued, and precipitation of crystals was confirmed after one minute. After continuing to stir at room temperature for 31 minutes, tert-butyl methyl ether (268 mL) was added dropwise over a period of 9 minutes. After further stirring at room temperature for 8 minutes, tert-butyl methyl ether (8589 mL) was added dropwise for 1 hour and 10 minutes, and stirring at room temperature was continued.
About 22 hours after completing dropwise addition of tert-butyl methyl ether, the precipitated crystals were suction filtered while blowing in nitrogen, washed with ethanol/tert-butyl methyl ether (107 mL/966 mL) and tert-butyl methyl ether (1074 mL) in that order, and suction dried for 8 minutes. A 531.10 g portion of the obtained wet crystals (584.54 g) was dried under reduced pressure (50˚C) to produce the target substance.
Yield: 419.6 g, HPLC PURITY: 99.4%
The powder X-ray crystal diffraction data confirmed that the crystals were identical to those of Example 2X (A-form crystals of sodium salt).

<HPLC analysis conditions (HPLC purity measurement and quantitation)>

Column: YMC-Pack Pro C18 AS-302 (5 μm, 4.6 mm I.D. x 150 mm I.D.)
Eluent: Solution A (acetonitrile/20 mM ammonium acetate aq. = 100/900 (v/v)),
Solution B (acetonitrile /20 mM ammonium acetate aq. = 800/200 (v/v))
Flow rate: 1.0 mL/mm
Detection: UV 254 nm
Oven temp.: 25°C
Sample temp.: 25°C
Gradient condition (time/solution B conc.): 0.01 min/0%, 25 min/100%, 30 min/100%, 30.01 min/0%, and 40 min/stop

Example 20 2-({[4-(2,2-dimethyl-1,3-dioxan-5-yl)methoxy-3,5-dimethylpyridin-2-yl]methyl}sulfinyl)-1H-benzimidazole (racemic mixture)

[Chemical Formula 9]

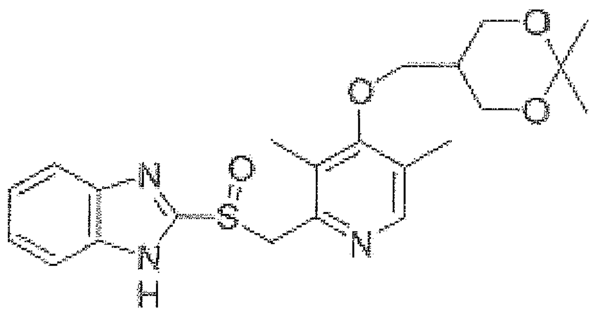

To a solution of compound (2) (424 mg, 1.03 mmol) in toluene (20 ml)-methanol (2 ml) was added dropwise a solution of 3-chloroperbenzoic acid (246 mg, 0.927 mmol as 65% content) in toluene (1 ml)-methanol (1 ml) at -65°C under a nitrogen atmosphere over a period of 5 minutes, and the mixture was stirred for 45 minutes under the same conditions. A saturated aqueous solution of sodium hydrogencarbonate was added to the reaction mixture. The mixture was extracted with ethyl acetate. The organic layer was dried over anhydrous sodium sulfate and then filtered and concentrated. The residue was purified by silica gel column chromatography (NH silica gel: 20 g, elution solvents: dichloromethane, dichloromethane/methanol = 10/1). The fractions containing the title compound were recovered using ethyl acetate and concentrated. Diethyl ether was added to the residue. The produced precipitate was filtered and washed with diethyl ether to produce the title compound (274 mg, yield: 61.9%) as a white solid.
$^1$H NMR (400 MHz, DMSO-$d_6$) δ ppm; 1.32 (3H, s), 1.36 (3H, s), 2.02-2.13 (1H, m), 2.16 (3H, s), 2.20 (3H, s), 3.74-3.84 (4H, m), 4.00 (2H. dd, J=4, 12Hz), 4.70 (1H, d, J=14Hz), 4.79 (1H, d, J=14Hz), 7.26-7.33 (2H, m), 7.60-7.70 (2H, m), 8.18 (1H, s).

Example 3Q Sodium salt of 2-({[4-(2,2-dimethyl-1,3-dioxan-5-yl)methoxy-3,5-dimethylpyridin-2-yl]methyl}sulfinyl)-1H-benzimidazole (racemic mixture)

[Chemical Formula 10]

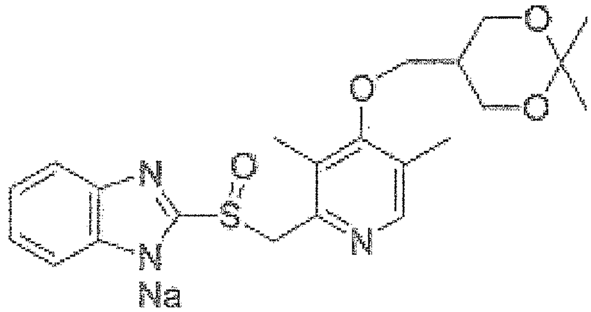

To a solution of 2-({[4-(2,2-dimethyl-1,3-dioxan-5-yl)methoxy-3,5-dimethylpyridink-2-yl]methyl}sulfinyl)-1H-benzimidazole (274 mg, 0.638 mmol) in ethanol (10 ml) was added a 1N aqueous solution of sodium hydroxide (635 μl, 0.638 mmol as 1.004 M concentration) at room temperature, and the mixture was concentrated. The residue was azeotropically distilled twice with ethanol. The residue was suspended in diethyl ether and subjected to sonication, and then concentrated to produce the title compound (260 mg, yield: 90.3%) as a white solid.
$^1$H NMR (400 MHz, DMSO-$d_6$) δ ppm; 1.33 (3H, s), 1.36 (3H, s), 2.03-2.14 (1H, m), 2.20 (3H, s), 2.21 (3H, s), 3.76-3.87 (4H, m). 4.00 (2H, dd, J=4, 11Hz), 4.39 (1H, d, J=13Hz), 4.75 (1H, d, J=13Hz), 6.81-6.91 (2H, m), 7.40-7.48 (2H, m), 8.23 (1H, s).

Example 4Q (S)-2-({[4-(2,2-dimethyl-1,3-dioxan-5-yl)methoxy-3,5-dimethylpyridin-2-yl]methyl}sulfinyl)-1H-benzimidazole

[Chemical Formula 11]

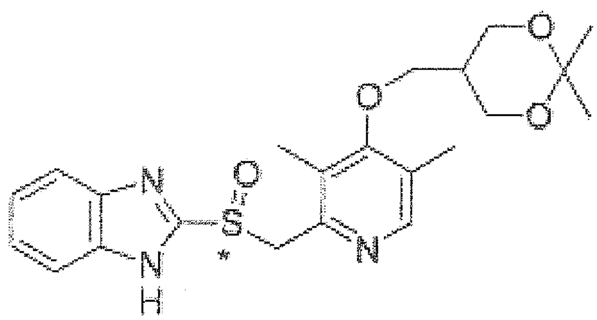

A solution of 2-({[4-(2,2-dimethyl-1,3-dioxan-5-yl)methoxy-3,5-dimethylpyridin-2-yl]methyl}thio)-1H-benzimidazole (compound (2)) (444 mg, 1.07 mmol) and D-(-)-diethyl tartrate (80.6 μl, 0.471 mmol) in toluene (anhydrous) (2.22 ml)-water (2.3 μl, 0.128 mmol) was stirred at 50°C for 10 minutes under a nitrogen atmosphere. Titanium(IV) isopropoxide (63.2 μl, 0.214 mmol) was added and the mixture was stirred for one hour under the same conditions. After cooling to room temperature, N,N-diisopropylethylamine (59.6 μl, 0.342 mmol) was added and the mixture was cooled to 0°C. Cumene hydroperoxide (611 μl, 3.31 mmol as 80% content) was added dropwise over a period of 5 minutes at 0°C to 2°C, and then the mixture was stirred at 0°C to 7°C for 3 hours and 35 minutes under a nitrogen atmosphere. A Saturated aqueous solution of sodium hydrogencarbonate was added to the reaction mixture, and then the mixture was extracted with ethyl acetate. The organic layer was dried over anhydrous sodium sulfate and then filtered and concentrated. The residue was purified by silica gel column chromatography (NH silica gel: 20 g, elution solvents: dichloromethane, dichloromethane/methanol = 20/1). The fractions containing the title compound were recovered using ethyl acetate and concentrated to produce the title compound (388 mg, yield: 84.4%) as a colorless foam.

$^1H$ NMR (400 MHz, DMSO-$d_6$) δ ppm; 1.32 (3H, s), 1.36 (3H, s), 2.02-2.13 (1H, m), 2.16 (3H, s), 2.20 (3H, s), 3.74-3.85 (4H, m), 4.00 (2H, dd, J=4, 12Hz), 4.70 (1H, d, J=14Hz), 4.79 (1H, d, J=14Hz), 7.26-7.34 (2H, m), 7.59-7.70 (2H, m), 8.18 (1H, s). HPLC
(Conditions)
Column: CHIRALPAK AD-H (product of Daicel Chemical Industries, Ltd.) (0.46 cm Ø x 25 cm), Eluent: hexane/ethanol = 1/1 (v/v), Flow rate; 0,6 ml/min,
Detection: UV (254 nm)
(Analysis results)
Retention time: 17.8 min, enantiomeric excess: 94.4% ee.

The aforementioned "(S)-2-({[4-(2,2-dimethyl-1,3-dioxan-5-yl)methoxy-3,5-dimethylpyridin-2-yl]methyl}sulfinyl)-1H-benzimidazole" was the later eluting optically active compound according to analysis under the aforementioned HPLC analysis conditions (enantiomeric excess).

Example 5Q Sodium salt of (S)-2-({[4-(2,2-dimethyl-1,3-dioxan-5-yl)methoxy-3,5-dimethylpyridin-2-yl]methyl}sulfinyl)-1H-benzimidazole

[Chemical Formula 12]

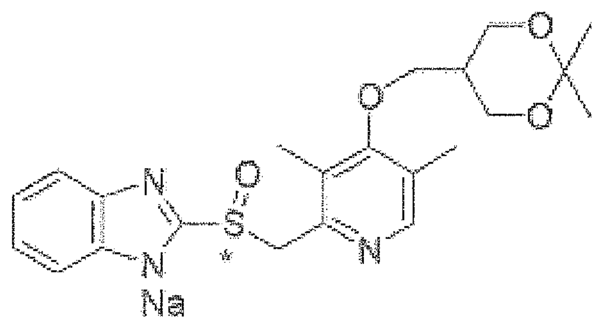

To a solution of (S)-2-({[4-(2,2-dimethyl-1,3-dioxan-5-yl)methoxy-3,5-dimethylpyridin-2-yl]methyl}sulfinyl)-1H-benzimidazole (379 mg, 0.882 mmol) in ethanol (10 ml) was added a 1N aqueous sodium hydroxide solution (878 μl, 0.882 mmol as 1.004 M concentration) at room temperature, and the mixture was concentrated. The residue was azeotropically distilled with ethanol. The residue was suspended in diethyl ether and subjected to sonication, and then concentrated to produce the title compound (365 mg, yield: 91.7%) as a white solid.
$^1H$ NMR (400 MHz, DMSO-$d_6$) δ ppm; 1.33 (3H, s), 1.36 (3H, s), 2.03-2.13 (1H, m), 2.20 (3H, s), 2.21 (3H, s), 3.76-3.88 (4H, m), 4.00 (2H, dd, J=4, 12Hz), 4.38 (1H, d, J=13Hz), 4.75 (1H, d, J=13Hz), 6.81-6.90 (2H, m), 7.40-7.47 (2H, m), 8.23 (1H, s).
HPLC
(Conditions)
Column: CHIRALPAK AD-H (product of Daicel. Chemical Industries, Ltd.) (0.46 cm Ø x 25 cm), Eluent: hexane/ethanol = 1/1 (v/v), Flow rate: 0.6 ml/min,
Detection: UV (254 nm)
(Analysis results)
Retention time: 17.0 min, enantiomeric excess: 94.9% ee.
Specific rotation: $[\alpha_D]$27.4 = -76.29 (c = 0.5, ethanol)

The aforementioned "sodium salt of (S)-2-({[4-(2,2-dimethyl-1,3-dioxan-5-yl)methoxy-3,5-dimethylpyridin-2-yl]methyl}sulfinyl)-1H-benzimidazole" was the later eluting optically active compound according to analysis under the aforementioned HPLC analysis conditions (enantiomeric excess).

Example 6Q (R)-2-({[4-(2,2-dimethyl-1,3-dioxan-5-yl)methoxy-3,5-dimethylpyridink-2-yl]methyl}sulfinyl)-1H-benzimidazole

[Chemical Formula 13]

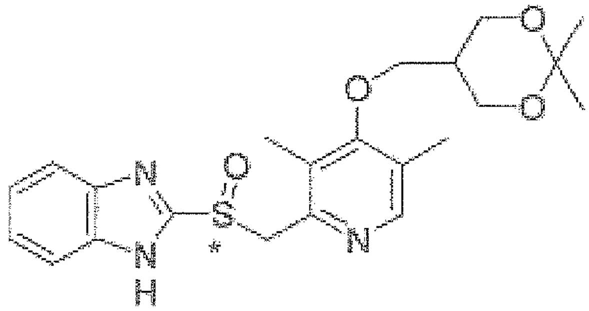

A solution of 2-({[4-(2,2-dimethyl-1,3-dioxank-5-yl)methoxy-3,5-dimethylpyridin-2-yl]methyl}thio)-1H-benzimidazole (compound (2)) (591 mg, 1.43 mmol) and L-(+)-diethyl tartrate (108 μl. 0.629 mmol) in toluene (anhydrous) (2.96 ml)-water (3.09 μl, 0.172 mmol) was stirred at 50˚C for 5 minutes under a nitrogen atmosphere. Titanium(IV) isopropoxide (84.4 μl, 0.286 mmol) was added and the mixture was stirred for one hour under the same conditions. After cooling to room temperature, N,N-diisopropylethylamine (79.7 μl, 0.458 mmol) was added and the mixture was cooled to 0˚C. Cumene hydroperoxide (816 μl, 4.42 mmol) as 80% content) was added dropwise at 0˚C to 1˚C over a period of 10 minutes, and then the mixture was stirred for 3 hours and 10 minutes under the same conditions. A saturated aqueous solution of sodium hydrogencarbonate was added to the reaction mixture, and then the mixture was extracted with ethyl acetate. The organic layer was dried over anhydrous sodium sulfate and then filtered and concentrated. The residue was purified by silica gel column chromatography (NH silica gel: 20 g, elution solvents: dichloromethane, dichloromethane/methanol = 20/1). The fractions containing the title compound were recovered using ethyl acetate and concentrated to produce the title compound (498 mg, yield: 81.1%) as a colorless foam.
$^1$H NMR (400 MHz, DMSO-$d_6$) δ ppm; 1.32 (3H, s), 1.36 (3H, s), 2.02-2.12 (1H, m), 2.16 (3H, s), 2.20 (3H, s), 3.74-3.84 (4H, m), 4.00 (2H, dd, J=4, 12MHz), 4.70 (1H, d, J=14Hz), 4.79 (1H, d, J=14Hz), 7.26-7.34 (2H, m), 7.58-7.70 (2H, m), 8.18 (1H, s).
HPLC
(Conditions)
Column: CHIRALPAK AD-H (product of Daicel Chemical Industries, Ltd.) (0.46 cm ∅ x 25 cm), Eluent: hexane/ethanol = 1/1 (v/v), Flow rate: 0.6 ml/min,
Detection: UV (254 nm)
(Analysis results)
Retention time: 14.6 min, enantiomeric excess: 95.4% ee.

The aforementioned "(R)-2-({[4-(2,2-dimethyl-1,3-dioxan-5-yl)methoxy-3,5-dimethylpyridin-2-yl]methyl}sulfinyl)-1H-benzimidazole" was the earlier eluting optically active compound according to analysis under the aforementioned HPLC analysis conditions (enantiomeric excess).

Example 7Q Sodium salt of (R)-2-({[4-(2,2-dimethyl-1,3-dioxan-5-yl)methoxy-3,5-dimethylpyridin-2-yl]methyl}sulfinyl)-1H-benzimidazole

[Chemical Formula 14]

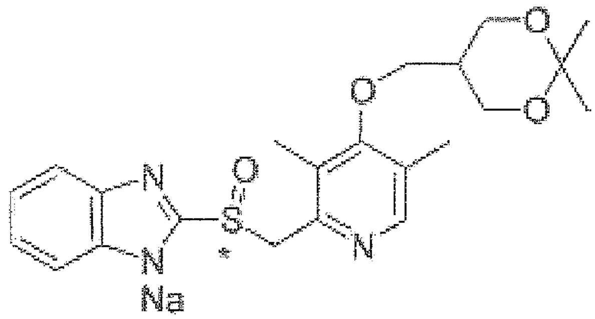

To a solution of (R)-2-({[4-(2,2-dimethyl-1,3-dioxan-5-yl)methoxy-3,5-dimethylpyridin-2-yl]methyl}sulfinyl)-1H-benzimidazole (480 mg, 1.12 mmol) in ethanol (10 ml) was added a 1N aqueous solution of sodium hydroxide (1.12 ml, 1.12 mmol) as 1.004 M concentration) at room temperature, and the mixture was concentrated. The residue was azeotropically distilled with ethanol. The residue was suspended in diethyl ether and subjected to sonication, and then concentrated to produce the title compound (447 mg, yield: 88.4%) as a white solid.
$^1$H NMR (400 MHz, DMSO-$d_6$) δ ppm; 1.33 (3H, s), 1.36 (3H, s), 2.03-2.14 (1H, m), 2.21 (6H, s), 3.76-3.87 (4H, m), 4.00 (2H, dd, J=4, 12Hz), 4.39 (1H, d, J=13Hz), 4.74 (1H, d, J=13Hz), 6.82-6.90 (2H, m), 7.40-7.48 (2H, m), 8.23 (1H, s).
HPLC
(Conditions)
Column: CHIRALPAK AD-H (product of Daicel Chemical Industries, Ltd.) (0.46 cm ∅ x 25 cm), Eluent: hexane/ethanol = 1/1 (v/v), Flow rate: 0.6 ml/min,
Detection: UV (254 nm)
(Analysis results)
Retention time: 14.4 min, enantiomeric excess: 95.4% ee.

(Test Example 1) Gastric secretion inhibitory action in chronic gastrostormy tube-implanted dogs

(1) Method

Large dogs (body weight: 14-19 kg) having chronic gastrostormy tubes implanted were used to examine the gastric secretion inhibitory action and gastric secretion inhibitory action durations of the example compounds. The experiment was conducted over a period of 2 days. On the first day, histamine (50 or 75 μg/kg/h) was continuously administered intravenously for 3 hours and gastric juice was taken every 20 minutes. One hour after starting histamine administration, the example compound suspended or dissolved in a 0.5% ethylcellulose solution was administered to the duodenum through an indwelling catheter at a dose of 0.1 ml/kg. The gastric secretion inhibitory action of the example compound was examined for 2 hours thereafter. On the second day (24 hours after administration of the example compounds), histamine was continuously administered intravenously for 2 hours and gastric juice was taken every 20 minutes to examine the gastric secretion inhibitory action duration. After measuring the gastric juice volume, 0.5 ml of gastric juice was sampled and subjected to neutralization titration to pH 7.0 with a 0.04 mol/l sodium hydroxide solution to measure the acid concentration. The acid concentration was multiplied by the gastric juice volume to determine the gastric acid output. The gastric secretion inhibitory action was evaluated by the gastric secretion inhibition (%) on the first day. The gastric secretion inhibitory action (%) was determined by the formula shown below. Where two or more cases were examined, the average of all the cases was determined.

$$\text{Gastric secretion inhibitory action (\%)} = (A-B)/A \times 100$$

[A]: Gastric acid output during 20 minutes, from 40 minutes to 1 hour after start of histamine administration
[B]: Gastric acid output during 20 minutes, from 1 hour and 40 minutes to 2 hours after administration of example compound
The gastric secretion inhibitory action duration was evaluated by the gastric secretion inhibition (%) on the second

day. The gastric secretion inhibitory action duration (%) was determined by the formula shown below.

$$\text{Gastric secretion inhibitory action duration (\%)} = (C\text{-}D)/C \times 100$$

[C]: Total gastric acid output from start to one hour after histamine administration on the first day
[D]: Total gastric acid output from start to one hour after histamine administration on the second day

(2) Results

[Table 1]

| Example compound | Administered dose (mg/kg, i.d.) | Number of cases | Gastric secretion inhibitory action (%) | Gastric secretion inhibition duration (%) |
|---|---|---|---|---|
| Compound A | 0.1 | 4 | 8 | 6 |
| Compound A | 0.2 | 4 | 65 | 46 |
| Compound A | 0.4 | 10 | 97 | 77 |
| Compound A | 0.8 | 8 | 100 | 89 |

Compound A: Sodium salt of (R)-2-({[4-(2,2-dimethyl-1,3-dioxan-5-yl)methoxy-3,5-dimethylpyridin-2-yl]methyl}sulfinyl)-1H-benzimidazole (A-form crystals of sodium salt)

(Preparation Example 1) Capsules

There were dissolved 30.0 g of compound A, 8.1 g of ethylcellulose (ETHOCEL™ by The Dow Chemical Company) and 16.2 g of hydroxypropylcellulose (HPC-L™ by Shin-Etsu Chemical Co., Ltd.) in 489 g of anhydrous ethanol. The solution was coated onto 500.1 g of the core material NON-PAREIL 108™ (Freund Corp.) using a Waster-type fluidized bed granulating coater (MULTIPLEX™, Powrex Corp.) and dried to produce granules.
A coating solution obtained by dissolving 48.6 g of ethylcellulose (ETHOCEL™ by The Dow Chemical Company) and 291.9 g of hydroxypropylcellulose (HPC-L™ by Shin-Etsu Chemical Co., Ltd.) in 6860 g of anhydrous ethanol and dispersing 136.8 g of magnesium stearate (Mallinckrodt, Inc.) was coated and dried onto 554.4 g of the aforementioned granules, to produce interlayer-coated granules.
Also, 460.2 g of hydroxypropylmethylcellulose phthalate (HP-55S™, Shin-Etsu Chemical Co., Ltd.) and 45.3 g of diacetylated monoglyceride (MYVACET™ by Quest Intl.) were dissolved in 11045 g of an 80% aqueous ethanol solution, and then after dispersing 42.3 g of talc (Talc™ by Matsumura Sangyo Co., Ltd.) and 24.3 g of titanium oxide (Titanium (IV) Oxide™ by Merck), the dispersion was coated onto 1031.7 g of the aforementioned interlayer-coated granules and dried to produce enteric-coated granules.
To 1603.8 g of the enteric-coated granules there were added 15.0 g of light anhydrous silicic acid (AEROSIL-200™ by Nippon Aerosil Co., Ltd. (Japanese Pharmacopeia)) and 15.0 g of talc (High Filler™ #17 by Matsumura Sangyo Co., Ltd.), and a vessel-type mixer (2/5 L vessel-type mixer, trade name of Toyo Packing Co., Ltd.) was used for mixing, after which the mixture was filled into capsules to 1 mg of compound A per capsule.

(Preparation Example 2) Capsules

Granules were produced with the following formulation by the same method as in Preparation Example 1, and were filled into capsules to 10 mg of compound A per capsule.
[Table 2]

| Component | |
|---|---|
| NON-PAREIL 108 | 465.0 |

(continued)

| | |
|---|---|
| Principal agent layer | |
| Compound A | 500.0 |
| Ethylcellulose | 135.0 |
| HPC-L | 270.0 |
| Interlayer | |
| Ethylcellulose | 40.0 |
| HPC-L | 240.0 |
| Mg stearate | 112.5 |
| Outer layer | |
| HP-55S | 380.0 |
| MYVACET | 37.5 |
| Talc | 35.0 |
| Titanium oxide | 20.0 |
| AEROSIL-200 | 30.0 |
| Talc | 30.0 |
| Units :g | |

NON-PAREIL 108™ (Freund Corp.)

Measurement of $^{13}C$ solid state NMR spectrum

The $^{13}C$ solid state NMR spectra for the crystals (A, B, C and D-type crystals of sodium salt) and amorphous forms obtained in each example were obtained under the following conditions.
Measuring apparatus: AVANCE 400 MHz (Bruker)
Probe: 7 mm-CP/MAS (Bruker)
NMR cell diameter: 7 mm
Cell spinning rate: 6000 rotations/sec
Measuring method: CPTOSS
Observed nucleus: $^{13}C$ (resonance frequency: 100.6248425 MHz)
Delay time: 5 sec
Contact time: 1000 microseconds
Number of scans: Each crystal: 1024 times, Amorphous forms: 18,296 times External standard: The chemical shift for the glycine carbonyl carbon was defined as 176.03 ppm.

The $^{13}C$ solid state NMR spectra for the A-form crystals of sodium salt obtained in Example 2X are shown in Figs. 1 and 2; the $^{13}C$ solid state NMR spectra for the B-form crystals of sodium salt obtained in Example 4X are shown in Figs. 3 and 4; the $^{13}C$ solid state NMR spectra for the C-form crystals of sodium salt obtained in Example 1X are shown in Figs. 5 and 6; the $^{13}C$ solid state NMR spectra for the D-form crystals of sodium salt obtained in Example 5X are shown in Figs. 7 and 8; the $^{13}C$ solid state NMR spectra for the amorphous form of sodium salt obtained in Example 9X are shown in Figs. 9 and 10; and the $^{13}C$ solid state NOR spectrum for the amorphous form of free acid obtained in Example 3Y is shown in Fig. 11.

The chemical shifts for the major peaks in a $^{13}C$ solid state NMR spectra of the crystals of sodium salt (A, B, C and D-form crystals) obtained in Examples 2X, 4X, 1X and 5X, the amorphous form of sodium salt obtained in Example 9X and the amorphous form of free acid obtained in Example 3Y are shown in Tables 3 to 8. In these tables, the numerical ranges in parentheses are the ranges of the broad peaks.

[Table 3]

| A-form crystals of sodium salt (ppm) | | | |
|---|---|---|---|
| 162.8 | 120.6 | 62.0 | 16.7 |
| 162.1 | 119.4 | 60.6 | 13.7 |

(continued)

| A-form crystals of sodium salt (ppm) | | | |
|---|---|---|---|
| 157.2 | 118.5 | 59.1 | 13.2 |
| 149.8 | 117.1 | 58.4 | 12.1 |
| 148.2 | 116.3 | 53.3 | 11.1 |
| 146.8 | 98.3 | 36.4 | 10.2 |
| 145.2 | 97.7 | 35.5 | |
| 126.1 | 70.4 | 32.5 | |
| 125.5 | 69.1 | 29.4 | |
| 124.9 | 68.4 | 19.6 | |
| 122.7 | 62.8 | 19.0 | |

[Table 4]

| B-form crystals of sodium salt (ppm) | | | |
|---|---|---|---|
| 163.0 | 122.6 | 53.3 | 12.2 |
| 162.4 | 120.0 | 35.7 | 11.1 |
| 157.4 | 119.0 | 35.3 | |
| 149.3 | 117.1 | 30.0 | |
| 148.6 | 116.2 | 29.4 | |
| 148.0 | 115.4 | 25.3 | |
| 146.8 | 98.3 | 21.7 | |
| 144.9 | 97.3 | 19.7 | |
| 126.1 | 70.2 | 18.9 | |
| 125.6 | 69.1 | 17.8 | |
| 124.8 | 61.2 | 14.2 | |
| 124.6 | 60.3 | 13.2 | |

[Table 5]

| C-form crystals of sodium salt (ppm) | | | |
|---|---|---|---|
| 162.9 | 126.5 | 71.1 | 30.2 |
| 161.9 | 125.9 | 68.6 | 29.1 |
| 157.7 | 125.2 | 62.2 | 22.7 |
| 156.9 | 123.8 | 61.6 | 19.4 |
| 150.2 | 122.0 | 61.0 | 18.1 |
| 148.4 | 119.6 | 60.3 | 13.5 |
| 147.0 | 118.7 | 59.3 | 13.0 |
| 145.3 | 117.6 | 52.7 | 12.5 |
| 144.6 | 116.8 | 36.5 | 11.8 |

(continued)

| C-form crystals of sodium salt (ppm) | | | |
|---|---|---|---|
| 144.0 | 98.6 | 34.7 | 11.5 |
| 137.7 | 98.3 | 32.7 | 9.5 |

[Table 6]

| D-form crystals of sodium salt (ppm) | | | |
|---|---|---|---|
| 165.2 | 119.2 | 59.2 | 10.6 |
| 162.7 | 117.8 | 49.6 | |
| 159.8 | 116.0 | 36.2 | |
| 151.1 | 98.3 | 33.5 | |
| 147.6 | 97.3 | 31.6 | |
| 145.9 | 72.2 | 28.5 | |
| 145.3 | 71.5 | 27.8 | |
| 127.5 | 68.2 | 20.3 | |
| 126.8 | 63.4 | 18.8 | |
| 125.3 | 61.8 | 13.3 | |
| 120.4 | 60.5 | 11.5 | |

[Table 7]

| Amorphous form of sodium salt (Example 9X) | |
|---|---|
| Peak center (ppm) | Peak half-width |
| 1.61.5 - 164.0 | ≥ 2.5 ppm |
| 145.5 - 151.0 | ≥ 5.5 ppm |
| 125.0 - 128.0 | ≥ 3.0 ppm |
| 116.5 - 120.5 | ≥ 4.0 ppm |
| 97.0 - 98.5 | ≥ 1.5 ppm |
| 69.0 - 72.0 | ≥ 3.0 ppm |
| 59.0 - 62.0 | ≥ 3.0 ppm |
| 34.0 - 36.0 | ≥ 2.0 ppm |
| 27.5 - 30.0 | ≥ 2.5 ppm |
| 18.0 - 20.0 | ≥ 2.0 ppm |
| 10.5 - 14.0 | ≥ 3.5 ppm |

[Table 8]

| Amorphous form of free compound (Example 3Y) | |
|---|---|
| Peak center (ppm) | Peak half-width |
| 161.5 - 164.0 | ≥ 2.5 ppm |
| 147.5 - 152.0 | ≥ 4.5 ppm |
| 142.5 - 145.0 | ≥ 2.5 ppm |
| 133.5 - 136.0 | ≥ 2.5 ppm |
| 119.0 -126.5 | ≥ 7.5 ppm |
| 110.5 - 114.0 | ≥ 3.5 ppm |
| 97.0 - 98.5 | ≥ 1.5 ppm |
| 68.5 - 72.0 | ≥ 3.5 ppm |
| 58.5 - 62.5 | ≥ 4.0 ppm |
| 33.5 - 36.0 | ≥ 2.5 ppm |
| 27.5 - 30.5 | ≥ 3.0 ppm |
| 17.5 - 20.5 | ≥ 3.0 ppm |
| 10.5 - 14.0 | ≥ 3.5 ppm |

[Measurement of infrared absorption spectrum]

The infrared absorption spectra for the crystals (A, B, C and D-form crystals of sodium salt) and amorphous forms obtained in each of the examples were measured under the following conditions, according to the ATR method for infrared spectrum measurement described in the general test method of the Japanese Pharmacopeia.
Measuring apparatus: FT/IR-620 by JASCO Corp.
Measuring method: ATR method
Measuring range: 4000 $cm^{-1}$-650 $cm^{-1}$
Revolution: 4 $cm^{-1}$

The infrared absorption spectrum (ATR method) for the A-form crystals of sodium salt obtained in Example 2X is shown in Fig. 12; the infrared absorption spectrum (ATR method) for the B-form crystals of sodium salt obtained in Example 4X is shown in Fig. 13; the infrared absorption spectrum (ATR method) for the C-form crystals of sodium salt obtained in Example 1X is shown in Fig. 14; the infrared absorption spectrum (ATR method) for the D-form crystals of sodium salt obtained in Example 5X is shown in Fig. 15; the infrared absorption spectrum (ATR method) for the amorphous form of sodium salt obtained in Example 9X is shown in Fig. 16; and the infrared absorption spectrum (ATR method) for the amorphous form of free acid obtained in Example 3Y is shown in Fig. 17.

Also, the wavenumbers ($cm^{-1}$) for the major absorption peaks in an infrared absorption spectra (ATR method) of the crystals (A, B, C and D-form crystals of sodium salt) obtained in Examples 2X, 4X, 1X and 5X, the amorphous form of sodium salt obtained in Example 9X and the amorphous form of free acid obtained in Example 3Y are shown in Tables 9 to 12.

[Table 9]

| A-form crystals of sodium salt ($cm^{-1}$) | | B-form crystals of sodium salt ($cm^{-1}$) | |
|---|---|---|---|
| 1473 | 973 | 1489 | 1155 |
| 1456 | 956 | 1473 | 1072 |
| 1417 | 916 | 1457 | 1022 |
| 1371 | 865 | 1436 | 917 |
| 1295 | 827 | 1418 | 829 |
| 1267 | 799 | 1396 | 798 |

(continued)

| A-form crystals of sodium salt ($cm^{-1}$) | | B-form crystals of sodium salt ($cm^{-1}$) | |
|---|---|---|---|
| 1197 | 739 | 1387 | 739 |
| 1154 | | 1374 | |
| 1099 | | 1339 | |
| 1072 | | 1317 | |
| 1021 | | 1289 | |
| 992 | | 1267 | |
| | | 1197 | |

[Table 10]

| C-form crystals of sodium salt ($cm^{-1}$) | | D-form crystals of sodium salt ($cm^{-1}$) | |
|---|---|---|---|
| 1489 | 1096 | 1489 | 1155 |
| 1473 | 1073 | 1473 | 1093 |
| 1457 | 1022 | 1457 | 1077 |
| 1436 | 1011 | 1436 | 1010 |
| 1418 | 916 | 1418 | 919 |
| 1396 | 822 | 1395 | 830 |
| 1372 | 799 | 1374 | 802 |
| 1339 | 738 | 1339 | |
| 1317 | | 1317 | |
| 1297 | | 1264 | |
| 1268 | | 1246 | |
| 1244 | | 1196 | |
| 1197 | | | |
| 1155 | | | |

[Table 11]

| Sodium salt amorphous form (Example 9X) ($cm^{-1}$) | |
|---|---|
| 1472 | 917 |
| 1456 | 822 |
| 1373 | 803 |
| 1339 | 745 |
| 1317 | |
| 1295 | |
| 1269 | |
| 1248 | |
| 1225 | |

(continued)

| Sodium salt amorphous form (Example 9X) ($cm^{-1}$) | |
|---|---|
| 1197 | |
| 1154 | |
| 1071 | |
| 1011 | |

[Table 12]

| Free compound amorphous form (Example 3Y) ($cm^{-1}$) | |
|---|---|
| 1472 | 1225 |
| 1457 | 1197 |
| 1436 | 1155 |
| 1417 | 1074 |
| 1396 | 1034 |
| 1372 | 1010 |
| 1339 | 916 |
| 1316 | 822 |
| 1295 | 800 |
| 1269 | 746 |

Powder X-ray diffraction pattern measurement

Powder X-ray diffraction measurement of the crystals obtained in each of the examples was carried out under the following measuring conditions, according to the powder X-ray diffraction measurement method described in the general test method of the Japanese Pharmacopeia.
(Apparatus)
Rigaku X-ray DTA system: RINT-2000 (product of Rigaku Corp.)
Powder X-ray diffraction apparatus: RAD-1R (product of Rigaku Corp.) (Example 1Z: potassium salt)
DISCOVER D8 with GADDS (Example 4Z (zinc salt), 5Z (calcium salt))
(Method of operation)
Each sample was placed on a 10 mm-diameter copper sheet, 16 mm x 20 mm glass panel (Example 1Z: potassium salt) or slide glass (Example 4Z: zinc salt, Example 5Z: calcium salt), and measured under the following conditions.
X-rays: CuKα rays
Tube voltage: 40 kV
Tube current: 200 mA
Divergence slit: 1/2 deg
Receiving slit: 0.3 mm
Scattering slit: 1/2 deg
Scanning speed: 2˚/min,
10˚/min (Example 10X: F-form crystals of sodium salt)
5˚/min (Example 6X: G-form crystals of sodium salt, Example 2Z: magnesium salt)
Scanning step: 0.02˚
Measuring range (2θ): 2-40˚
(Measuring conditions for potassium salt of Example 1Z)
X-rays: CuKα rays
Tube voltage: 40 kV
Tube current: 20 mA

Divergence slit: 1 deg
Receiving slit: 0.15 mm
Scattering slit: 1 deg
Scanning speed: 2˚/min
Scanning step: 0.02˚
Measuring range (2θ): 2-40˚

(Measuring conditions for zinc salt of Example 4Z and calcium salt of Example 5Z)

X-rays: CuKα rays
Tube voltage: 40 kV
Tube current: 20 mA
Collimator: 0.8 mm double slit
Sample-detector distance: 15 cm
Measuring time: 3 min
Measuring method: reflection method
Measuring range (2θ): 3-25˚
Resolution: 0.02˚

A powder X-ray diffraction pattern for the A-form crystals of sodium salt obtained in Example 2X is shown in Fig. 18; a powder X-ray diffraction pattern for the B-form crystals of sodium salt obtained in Example 4X is shown in Fig. 19; a powder X-ray diffraction pattern for the C-form crystals of sodium salt obtained in Example 1X is shown in Fig. 20; a powder X-ray diffraction pattern for the D-form crystals of sodium salt obtained in Example 5X is shown in Fig. 21; a powder X-ray diffraction pattern for the E-form crystals of sodium salt obtained in Example 8X is shown in Fig. 22; a powder X-ray diffraction pattern for the F-form crystals of sodium salt obtained in Example 10X is shown in Fig. 23; a powder X-ray diffraction pattern for the G-form crystals of sodium salt obtained in Example 6X is shown in Fig. 24; a powder X-ray diffraction pattern for the H-form crystals of sodium salt obtained in Example 7X is shown in Fig. 25; a powder X-ray diffraction pattern for the amorphous form of sodium salt obtained in Example 3X is shown in Fig. 26; a powder X-ray diffraction pattern for the amorphous form of sodium salt obtained in Example 9X is shown in Fig. 27; a powder X-ray diffraction pattern for the A-form crystals of free acid obtained in Example 1Y is shown in Fig. 28; a powder X-ray diffraction pattern for the B-form crystals of free acid obtained in Example 2Y is shown in Fig. 29; a powder X-ray diffraction pattern for the amorphous form of free acid obtained in Example 3Y is shown in Fig. 30; a powder X-ray diffraction pattern for the crystals of magnesium salt obtained in Example 2Z is shown in Fig. 31; a powder X-ray diffraction pattern for the crystals of potassium salt obtained in Example 1Z is shown in Fig. 32; a powder X-ray diffraction pattern for the crystals of lithium salt obtained in Example 3Z is shown in Fig. 33; a powder X-ray diffraction pattern for the crystals of zinc salt obtained in Example 4Z is shown in Fig. 34; and a powder X-ray diffraction pattern for the crystals of calcium salt obtained in Example 5Z is shown in Fig. 35.

Also, the diffraction angles (2θ) for the major peaks in the powder X-ray diffraction patterns for the sodium salts (A, B, C, D, E, F, G and H-form crystals of sodium salt), free acids (A and B-form crystals of free acid) and magnesium salt and potassium salt crystals of (R)-2-({[4-(2,2-dimethyl-1,3-dioxan-5-yl)methoxy-3,5-dimethylpyridin-2-yl]methyl} sulfinyl)-1H-benzimidazole obtained in each example is shown below.

A-form crystals of sodium salt (Example 2X)
4.9˚, 8.0˚, 9.9˚, 10.8˚, 11.7˚, 12.9˚, 15.9˚, 17.7˚

B-form crystals of sodium salt (Example 4X)
5.0˚, 9.5˚, 9.9˚, 11.5˚, 13.6˚, 15.6˚, 16.5˚, 17.6˚, 18.1˚

C-form crystals of sodium salt (Example 1X)
4.9˚, 9.8˚, 11.4˚, 11.7˚, 14.6˚, 15.7˚, 16.2˚, 17.3˚, 18.9˚, 22.6˚

D-form crystals of sodium salt (Example 5X)
4.1˚, 10.4˚, 12.2˚, 12.7˚, 17.1˚, 18.4˚, 19.5˚

E-form crystals of sodium salt (Example 8X)
4.4˚, 9.6˚, 10.3˚, 16.2˚, 17.5˚, 18.5˚, 19.7˚

F-form crystals of sodium salt (Example 10X)
4.2˚, 8.6˚, 9.7˚, 10.3˚, 12.7˚, 13.2˚, 16.5˚, 17.5˚, 18.5˚, 19.2˚, 19.7˚

G-form crystals of sodium salt (Example 6X)
4.1˚, 10.6˚, 17.0˚, 17.3˚, 17.9˚, 18.4˚, 19.4˚, 19.7˚, 24.8˚

H-form crystals of sodium salt (Example 7X)
4.4˚, 10.1˚, 10.3˚, 16.6˚, 17.8˚, 18.6˚, 19.8˚, 20.3, 21.3

A-form crystals of free acid (Example 1Y)
8.1˚, 12.5˚, 14.5˚, 14.8˚, 20.5˚. 24.8˚

B-form crystals of free acid (Example 2Y)
6.0˚, 9.0˚, 14.7˚, 17.2˚, 18.9˚

Crystals of potassium salt (Example 1Z)
5.2˚, 10.4˚, 14.5˚, 16.2˚, 19.5˚, 20.9˚, 25.0˚

Crystals of magnesium salt (Example 2Z)
5.4˚, 6.6˚, 9.4˚, 16.2˚, 18.0˚, 19.9˚

Crystals of lithium salt (Example 3Z)
4.9˚, 9.9˚, 15.9˚, 22.9˚

## Industrial Applicability

Salts, crystals and amorphous forms of compound (1) of the invention have excellent gastric secretion inhibitory action as well as excellent physical properties as medicaments for medical use, and can therefore be useful as therapeutic or prophylactic agents for gastroesophageal reflux, symptomatic gastroesophageal reflux, gastric ulcer, duodenal ulcer and the like. That is, according to the invention it is possible to provide salts, crystals and amorphous forms of compound (1) which can be utilized as drug substances or as production intermediates for production of drug substances.

## Claims

**1.** A salt of 2-[({4-[(2,2-dimethyl-1,3-dioxan-5-yl)methoxy]-3,5-dimethylpyridin-2-yl}methyl)sulfinyl]-1H-benzimidazole, wherein the salt is a salt selected from the group consisting of a potassium salt, a magnesium salt, a lithium salt, a calcium salt and a zinc salt.

**2.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having a peak at a chemical shift (±0.5 ppm) of 16.7 ppm in a $^{13}C$ solid state NMR spectrum.

**3.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having absorption peaks at wavenumbers (±2 $cm^{-1}$) of 827, 1021 and 1295 $cm^{-1}$ in an infrared absorption spectrum (ATR method).

**4.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having diffraction peaks at diffraction angles (2θ ±0.2˚) of 5.0˚, 8.0˚, 9.9˚, 10.8˚, 11.7˚ and 12.9˚ in a powder X-ray diffraction.

**5.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having a peak at a chemical shift (±0.5 ppm) of 25.3 ppm in a $^{13}C$ solid state NMR spectrum.

**6.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having absorption peaks at wavenumbers (±2 $cm^{-1}$) of 829, 1022 and 1289 $cm^{-1}$ in an infrared absorption spectrum (ATR method).

**7.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having diffraction peaks at diffraction angles (2θ ±0.2˚) of 5.0˚, 9.5˚, 9.9˚, 11.4˚, 13.6˚, 15.6˚ and 16.5˚ in a powder X-ray diffraction.

**8.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having a peak at a chemical shift (±0.5 ppm) of 137.7 ppm in a $^{13}C$ solid state NMR spectrum.

**9.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having absorption peaks at wavenumbers (±2 $cm^{-1}$) of 822 and 1022 $cm^{-1}$ in an infrared absorption spectrum (ATR method).

**10.** A crystalline form of 2-[({4-[(2,2-dimethyl-1,3-dioxan-5-yl)methoxy]-3.5-dimethylpyridin-2-yl}methyl)sulfinyl]-1H-benzimidazole sodium salt having diffraction peaks at diffraction angles (2θ ±0.2˚) of 4.9˚, 9.8˚, 11.4˚, 11.7˚, 14.6˚,

15.7°, 16.2°, 17.3° and 18.9° in a powder X-ray diffraction.

**11.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having a peak at a chemical shift (±0.5 ppm) of 159.8 ppm in a $^{13}C$ solid state NMR spectrum.

**12.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having an absorption peak at a wavenumber (±2 $cm^{-1}$) of 1077 $cm^{-1}$ in an infrared absorption spectrum (ATR method).

**13.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having diffraction peaks at diffraction angles (2θ ±0.2°) of 4.1°, 10.4°, 12.2°, 12.7°, 17.1°, 18.5° and 19.4° in a powder X-ray diffraction.

**14.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having diffraction peaks at diffraction angles (2θ ±0.2°) of 4.4°, 9.6°, 10.3°, 16.2°, 17.5°, 18.5° and 19.7° in a powder X-ray diffraction.

**15.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having diffraction peaks at diffraction angles (2θ ±0.2°) of 4.3°, 8.6°, 9.7°, 10.3°, 12.7°, 13.2°, 16.6°, 17.5°, 18.5°, 19.2° and 19.8° in a powder X-ray diffraction.

**16.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having diffraction peaks at diffraction angles (2θ ±0.2°) of 4.1°, 10.6°, 16.9°, 17.3°, 17.9°, 18.4°, 19.4° and 19.7° in a powder X-ray diffraction.

**17.** A crystalline form of 2-[({4-[(2,2-dimethyl-1,3-dioxan-5-yl)methoxy]-3,5-dimethylpyridin-2-yl}methyl)sulfinyl]-1H-benzimidazole sodium salt having diffraction peaks at diffraction angles (2θ ±0.2°) of 4.4°, 10.1°, 10.3°, 16.6°, 17.7°, 18.6°, 19.8°, 20.3° and 21.3° in a powder X-ray diffraction.

**18.** An amorphous form of 2-[({4-[(2,2-dimethyl-1,3-dioxan-5-yl)methoxy]-3,5-dimethylpyridin-2-yl}methyl)sulfinyl]-1H-benzimidazole sodium salt.

**19.** An amorphous form of 2-[({4-[(2,2-Dimethyl-1,3-dioxan-5-yl)methoxy]-3,5-dimethylpyridin-2-yl}methyl)sulfinyl]-1H-benzimidazole.

**20.** A salt according to claim 1, wherein the salt is a potassium salt.

**21.** A crystalline form of a salt according to claim 20, having diffraction peaks at diffraction angles (2θ ±0.2°) of 5.2°, 10.4°, 14.5°, 16.2° and 19.5° in a powder X-ray diffraction.

**22.** A salt according to claim 1, wherein the salt is a magnesium salt.

**23.** A crystalline form of a salt according to claim 22, having diffraction peaks at diffraction angles (2θ ±0.2°) of 5.4°, 16.2° and 19.9° in a powder X-ray diffraction.

**24.** A salt according to claim 1, wherein the salt is a lithium salt.

**25.** A crystalline form of a salt according to claim 24, having diffraction peaks at diffraction angles (2θ ±0.2°) of 4.9°, 9.9°, 15.9°, and 22.9° in a powder X-ray diffraction.

**26.** A salt according to claim 1, wherein the salt is a calcium salt.

**27.** A salt according to claim 1, wherein the salt is a zinc salt.

**28.** A crystalline form of 2-[({4-[(2,2-dimethyl-1,3-dioxan-5-yl)methoxy]-3,5-dimethylpyridin-2-yl}methyl)sulfinyl]-1H-henzimidazole, having diffraction peaks at diffraction angles (2θ ±0.2°) of 8.1°, 12.5° and 20.5° in a powder X-ray diffraction.

**29.** A crystalline form of 2-[({4-[(2,2-dimethyl-1,3-dioxan-5-yl)methoxy]-3,5-diimethylpyridin-2-yl}methyl)sulfinyl]-1H-benzimidazole, having diffraction peaks at diffraction angles (2θ ±0.2°) of 6.0°, 9.0° and 17.3° in a powder X-ray diffraction.

Fig.1

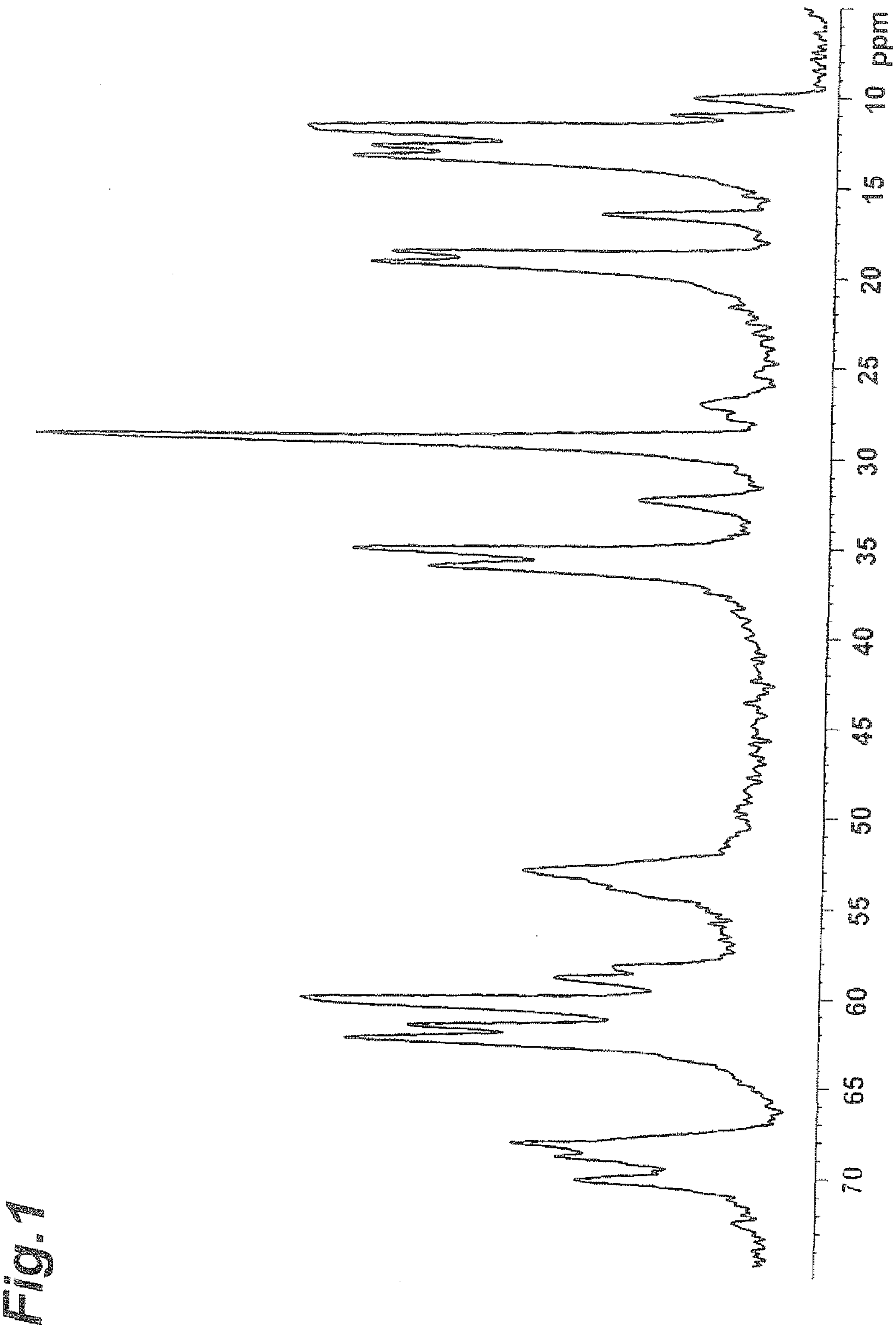

Fig.2

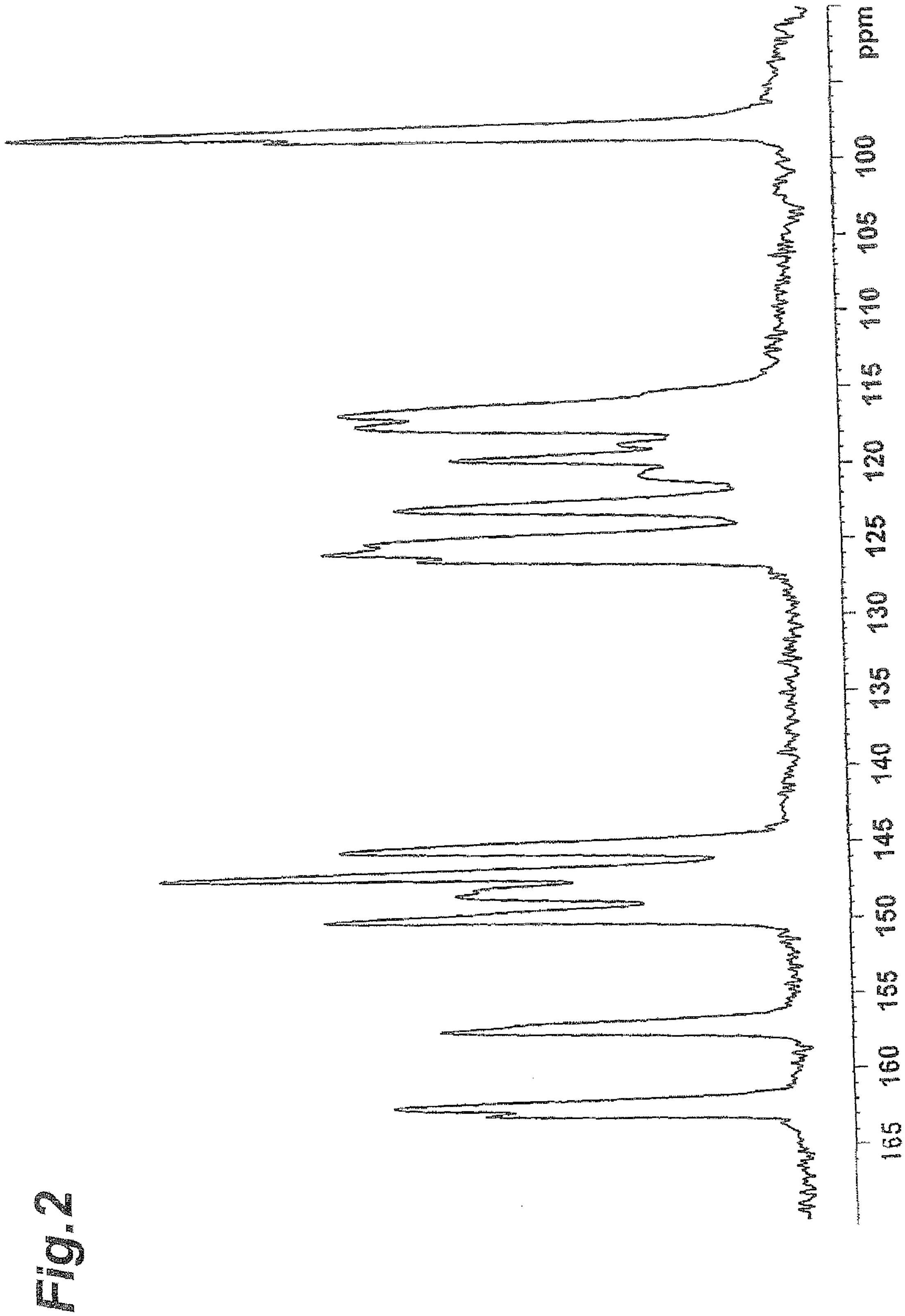

Fig.3

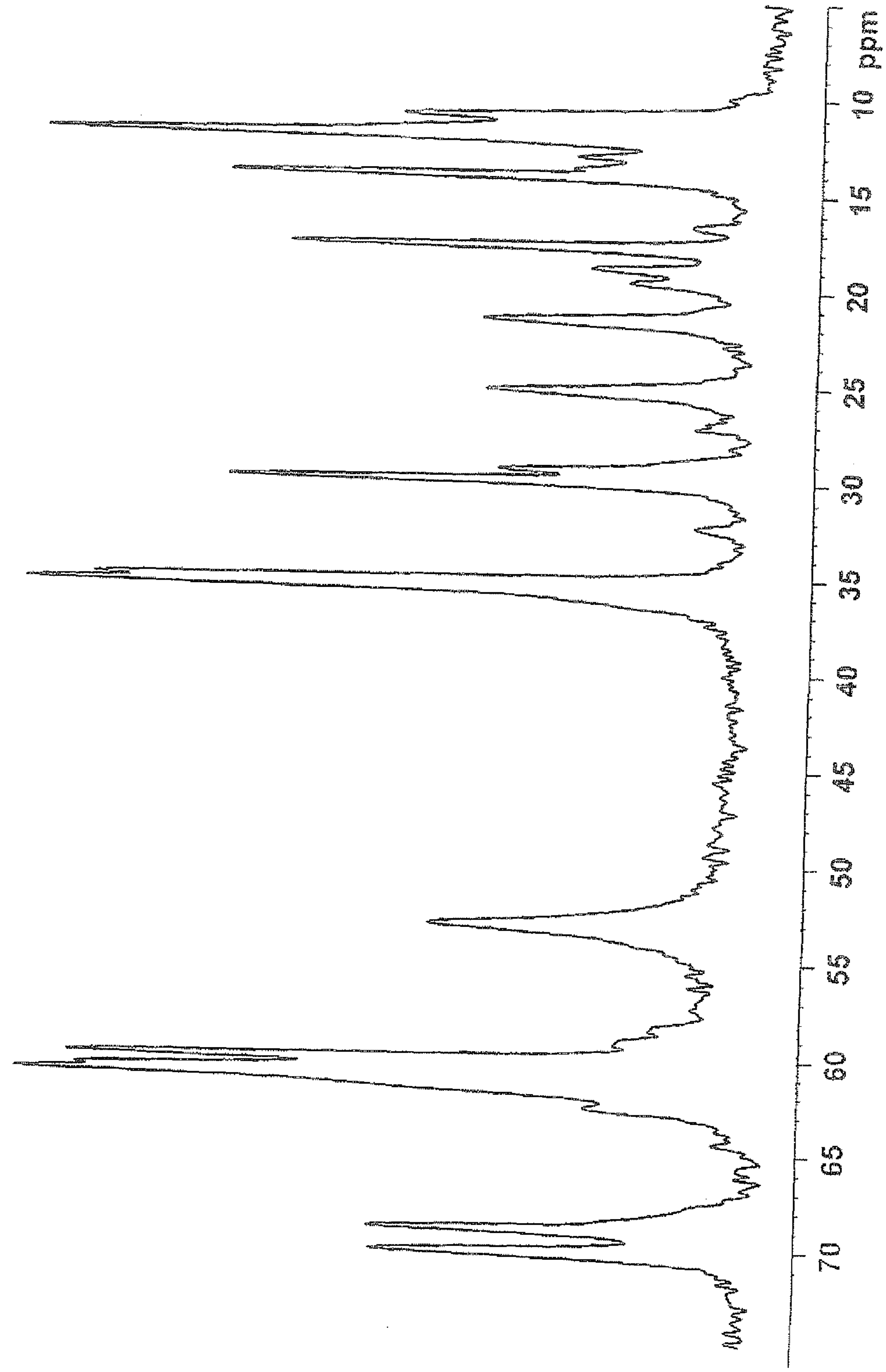

Fig.4

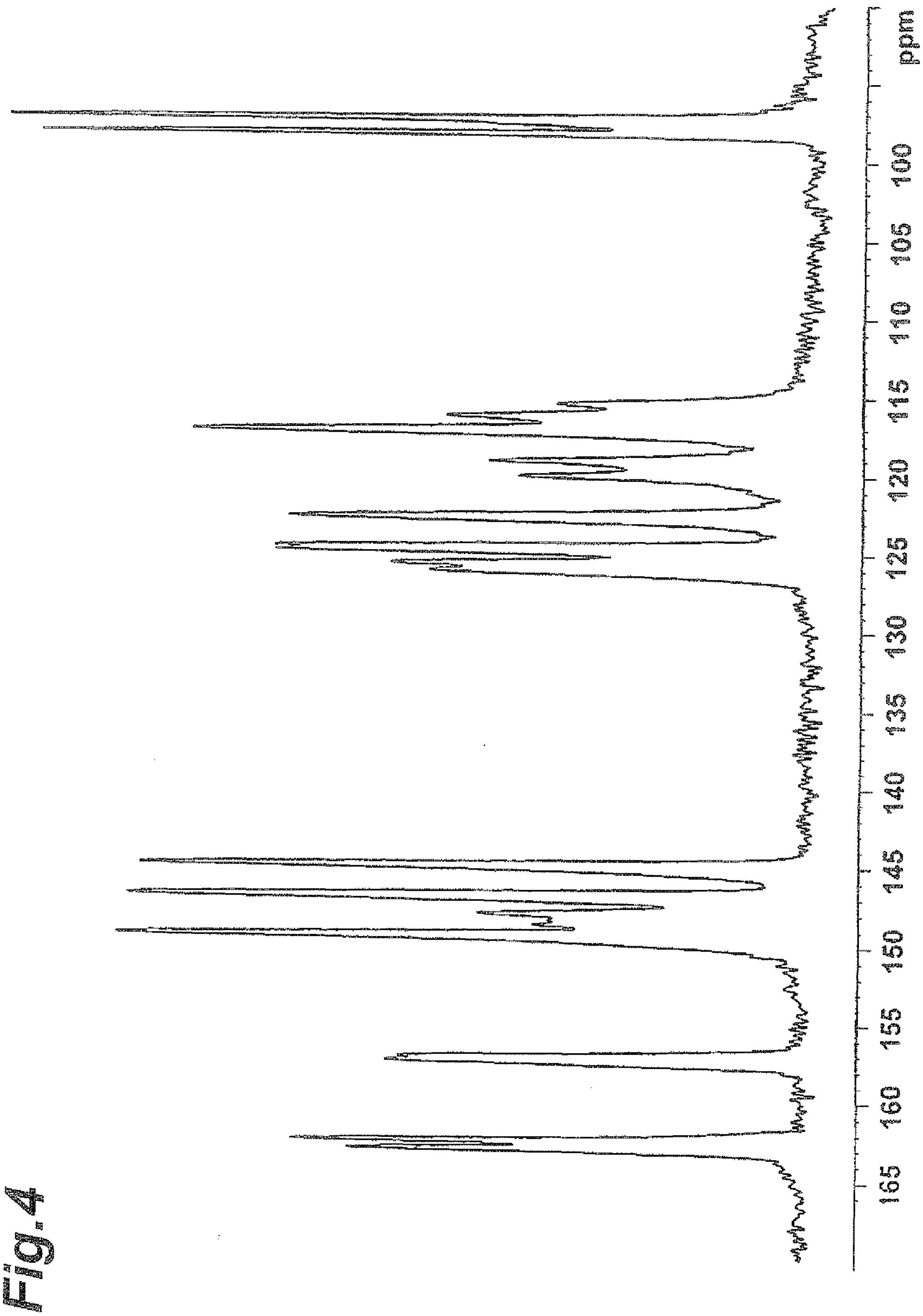

Fig.5

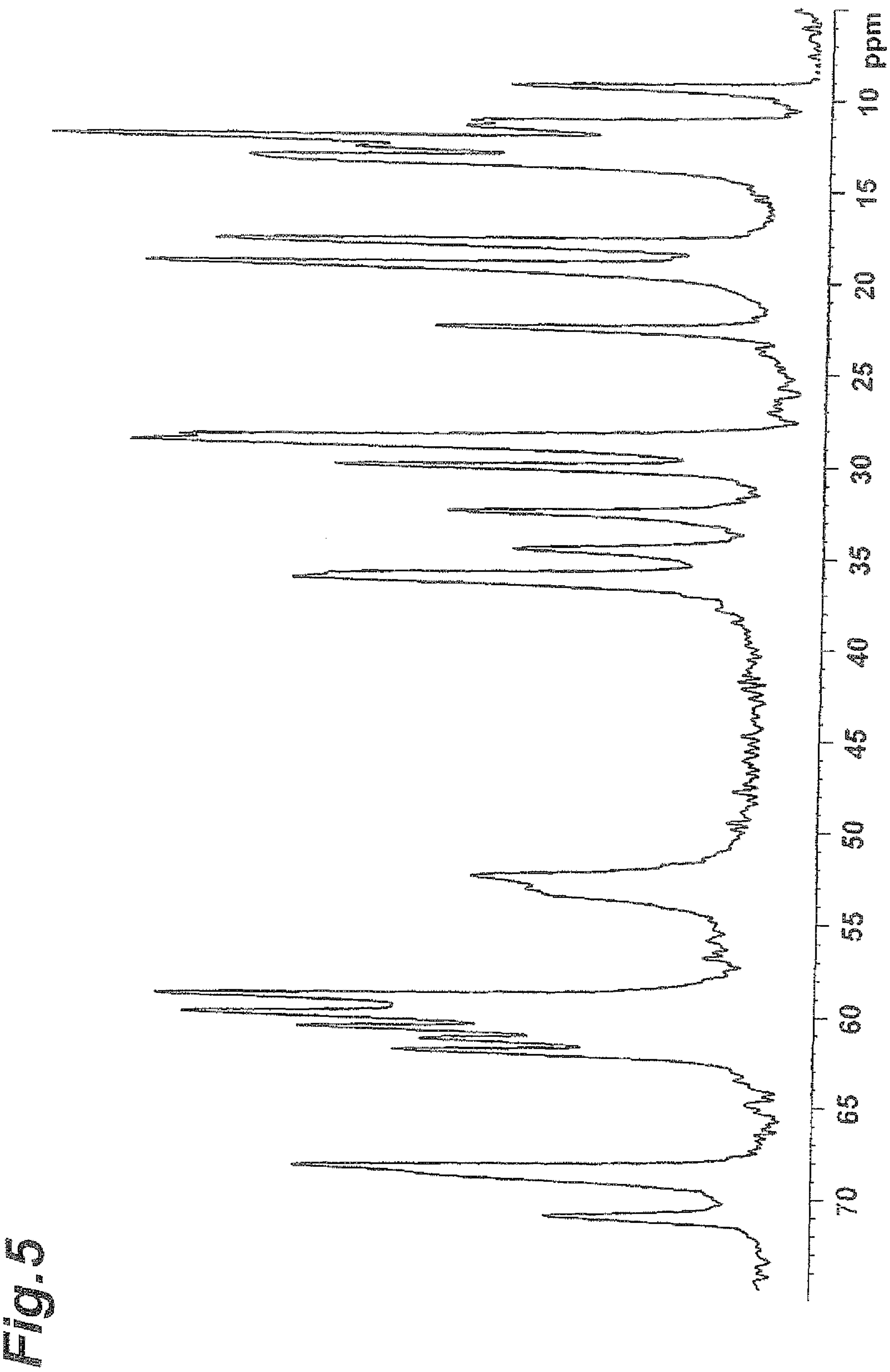

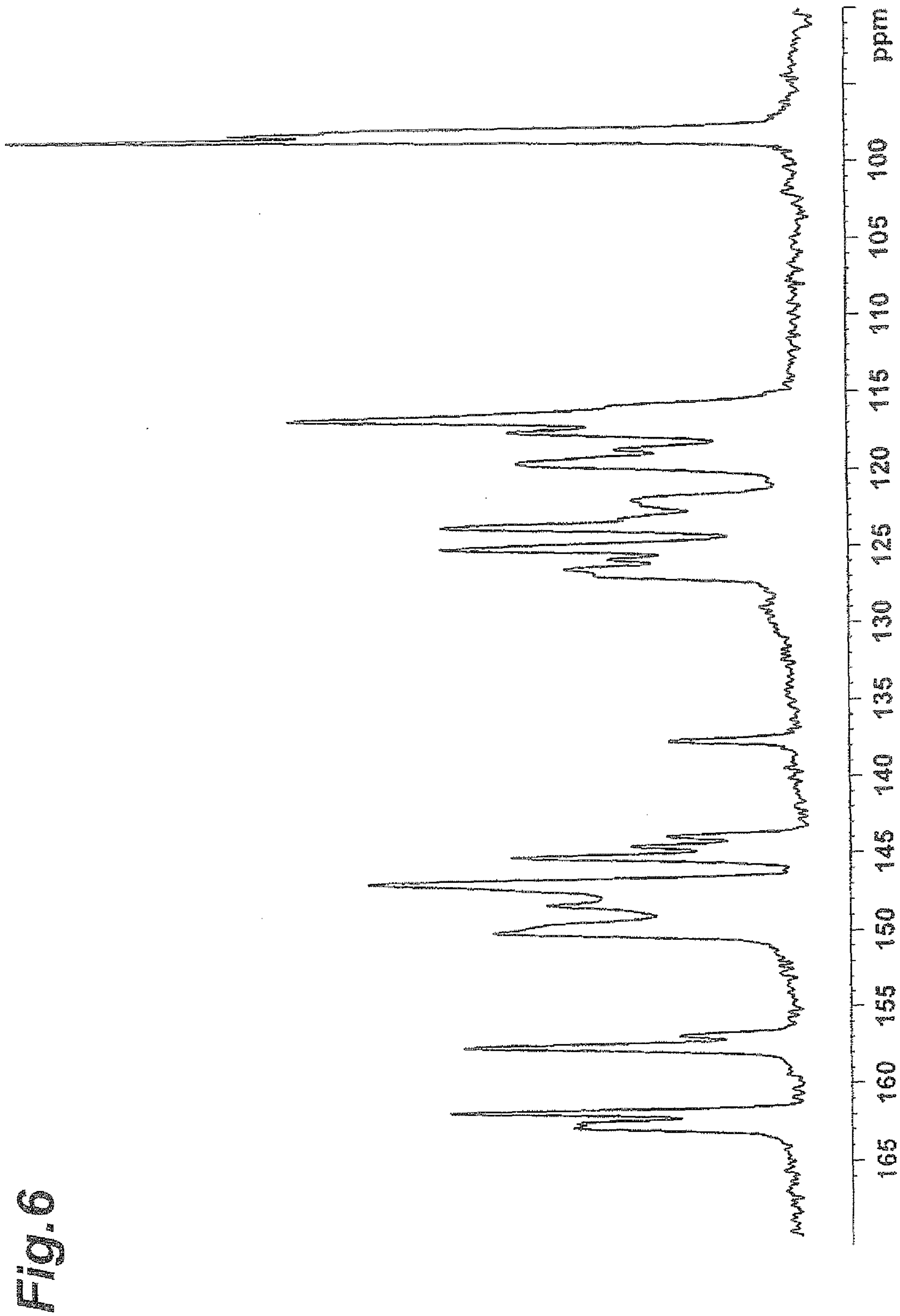


Fig.6

Fig. 7

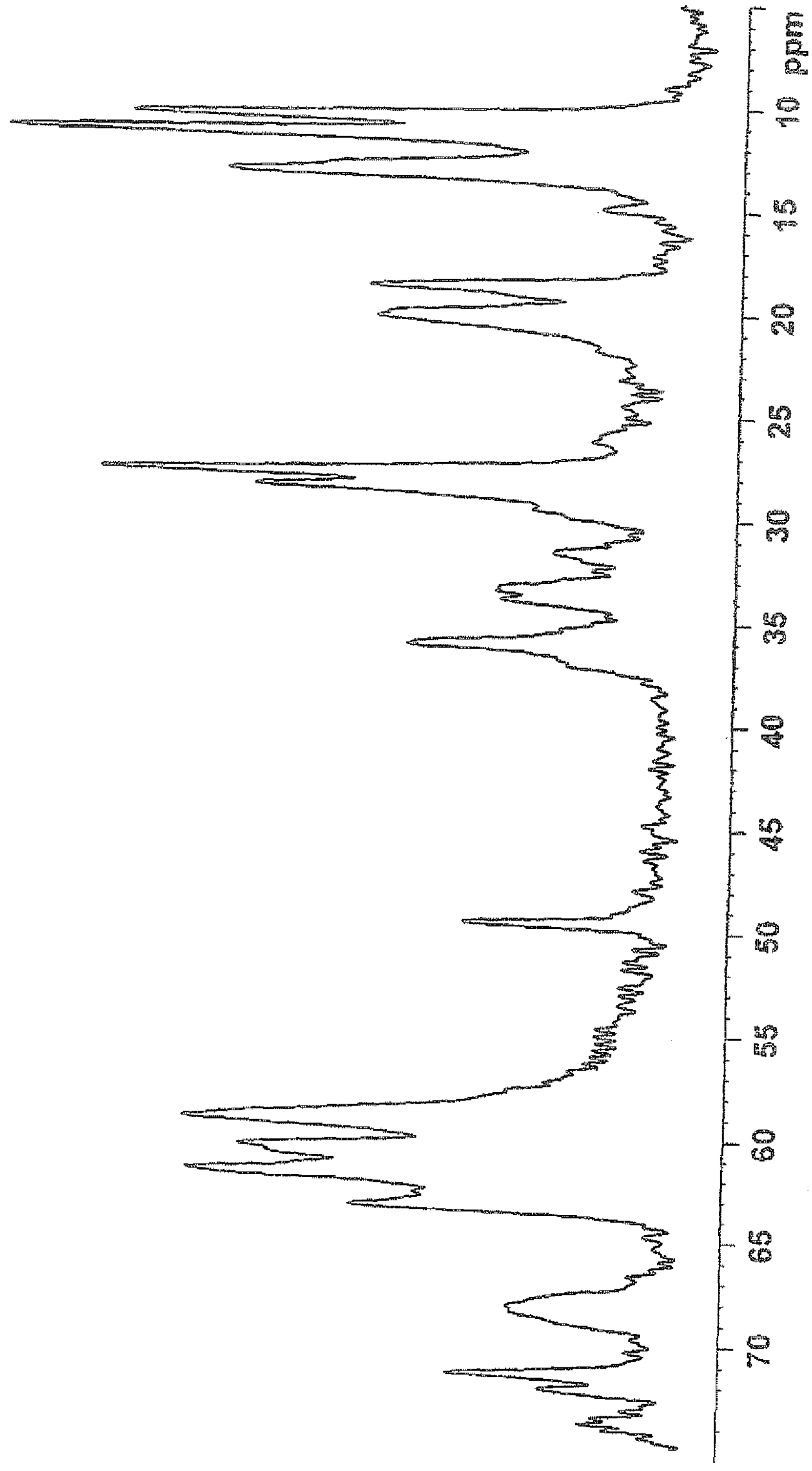

Fig. 8

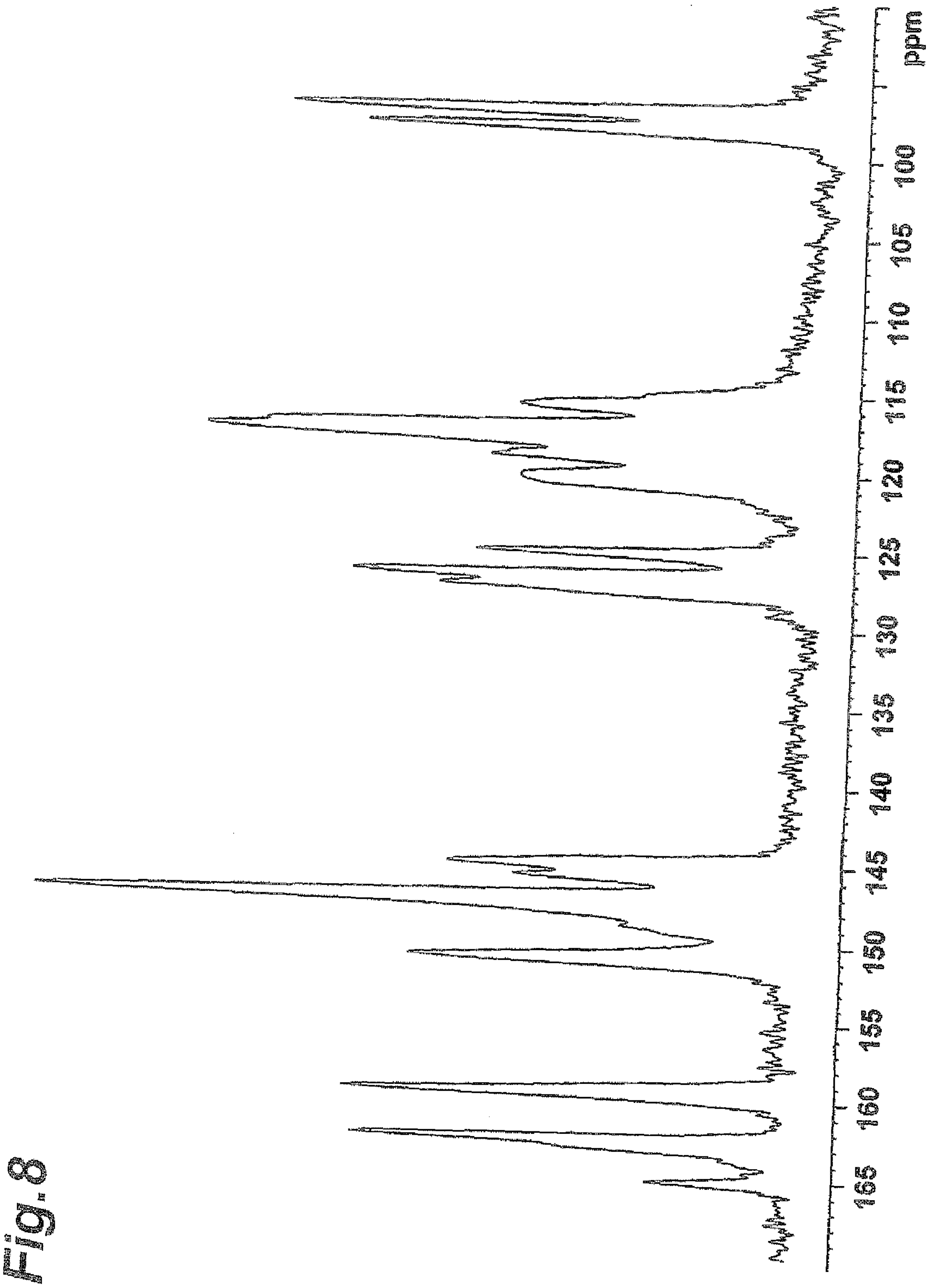

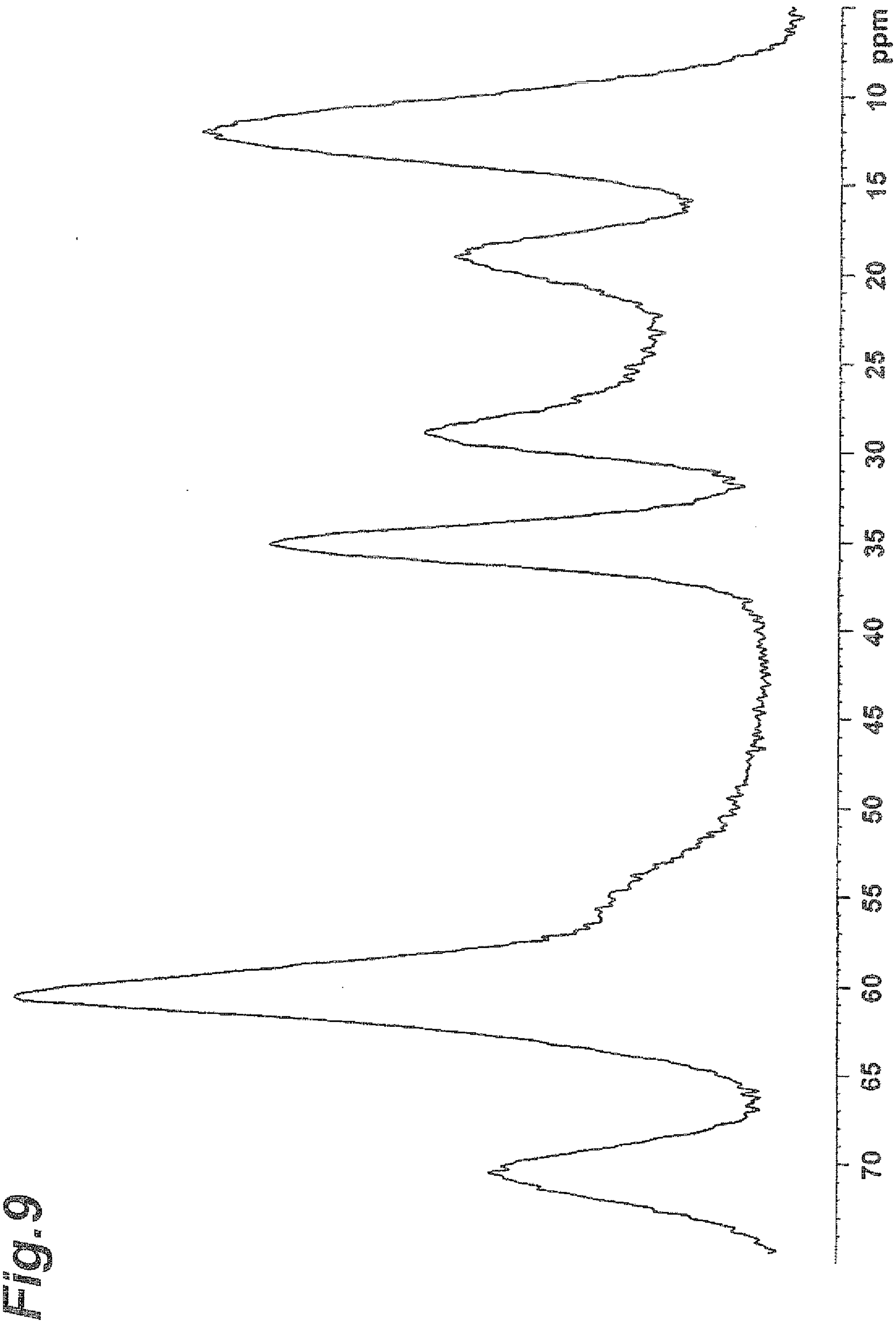


Fig.9

Fig. 10

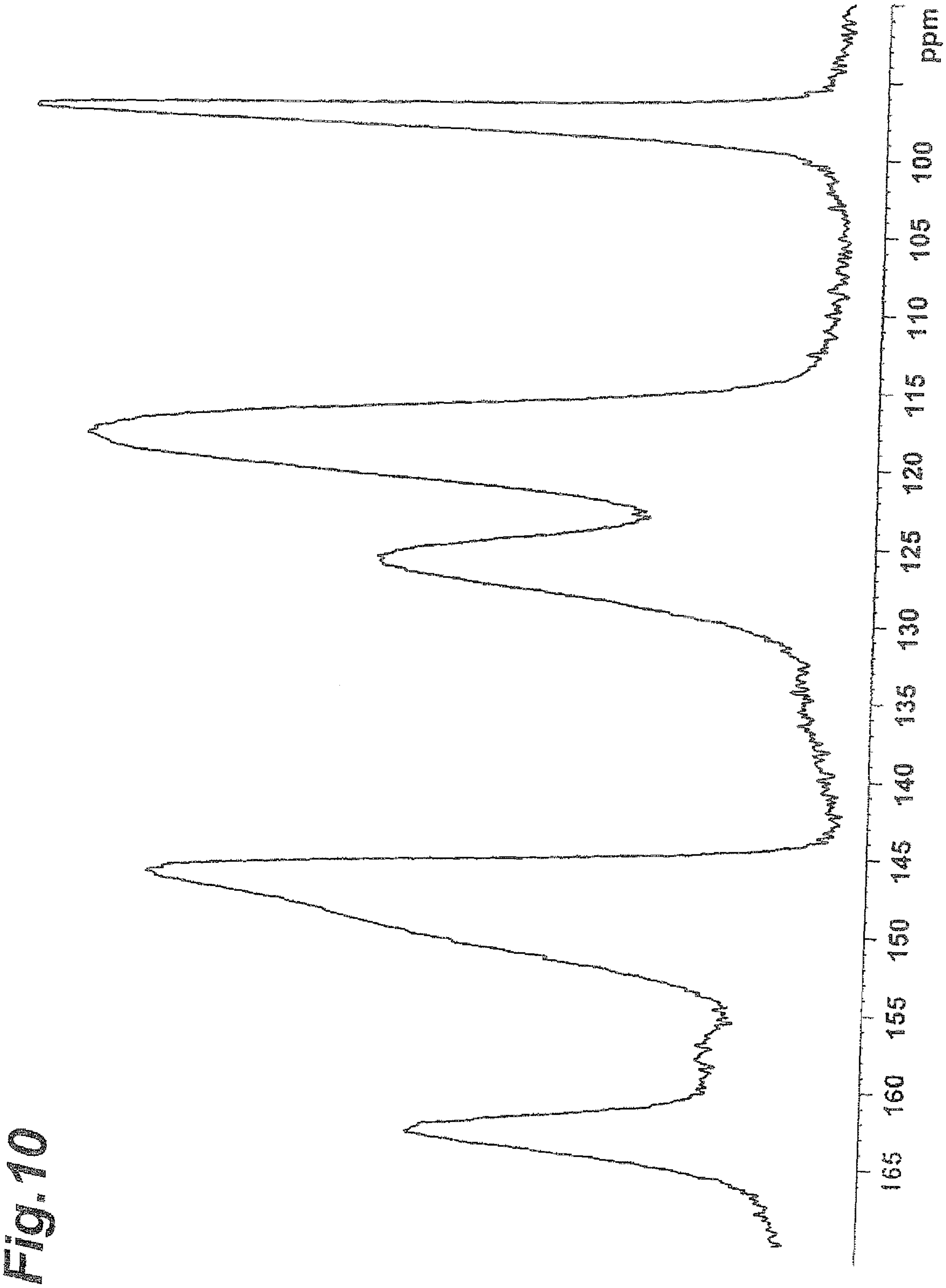

Fig.11

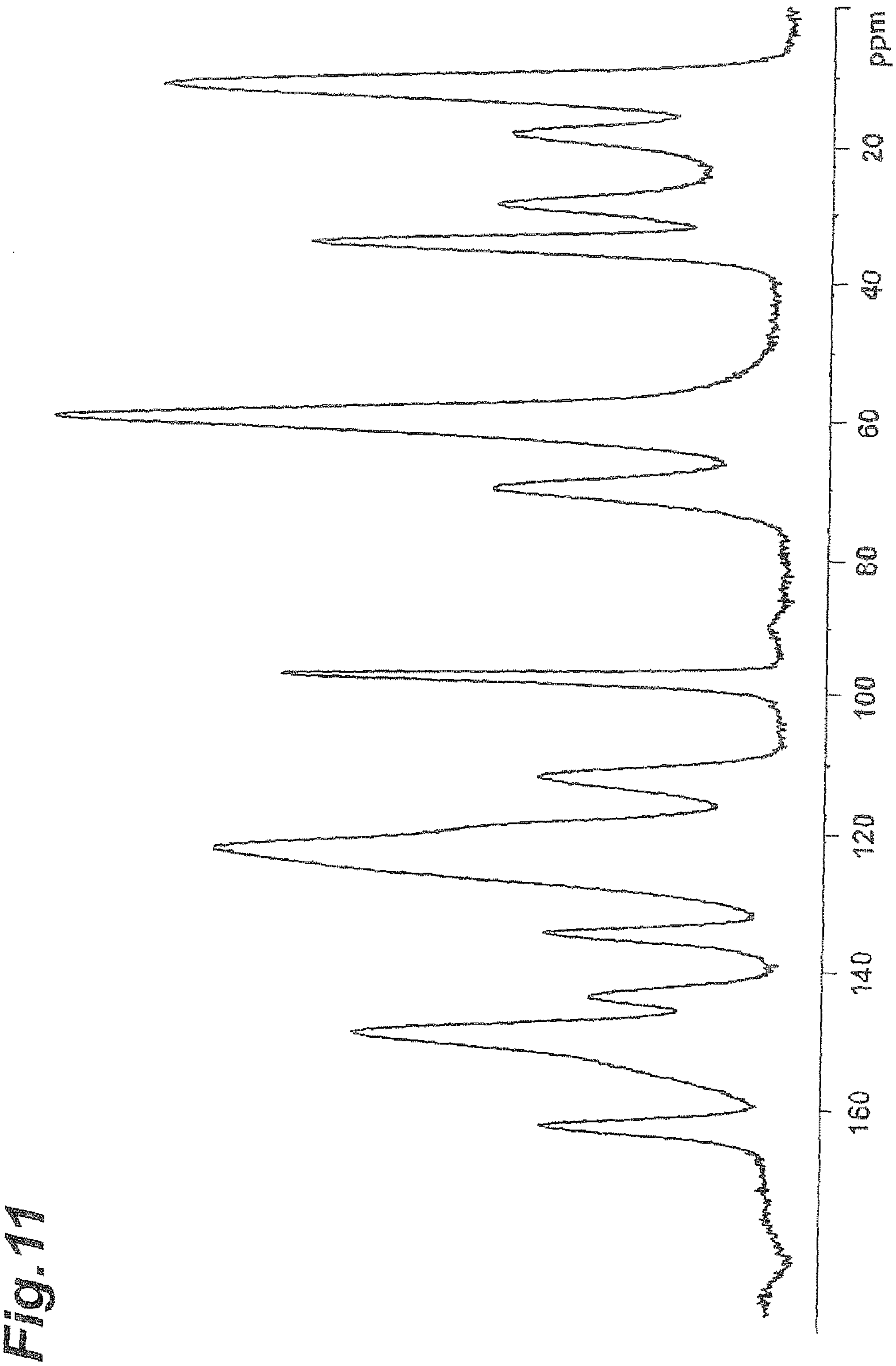

Fig.12

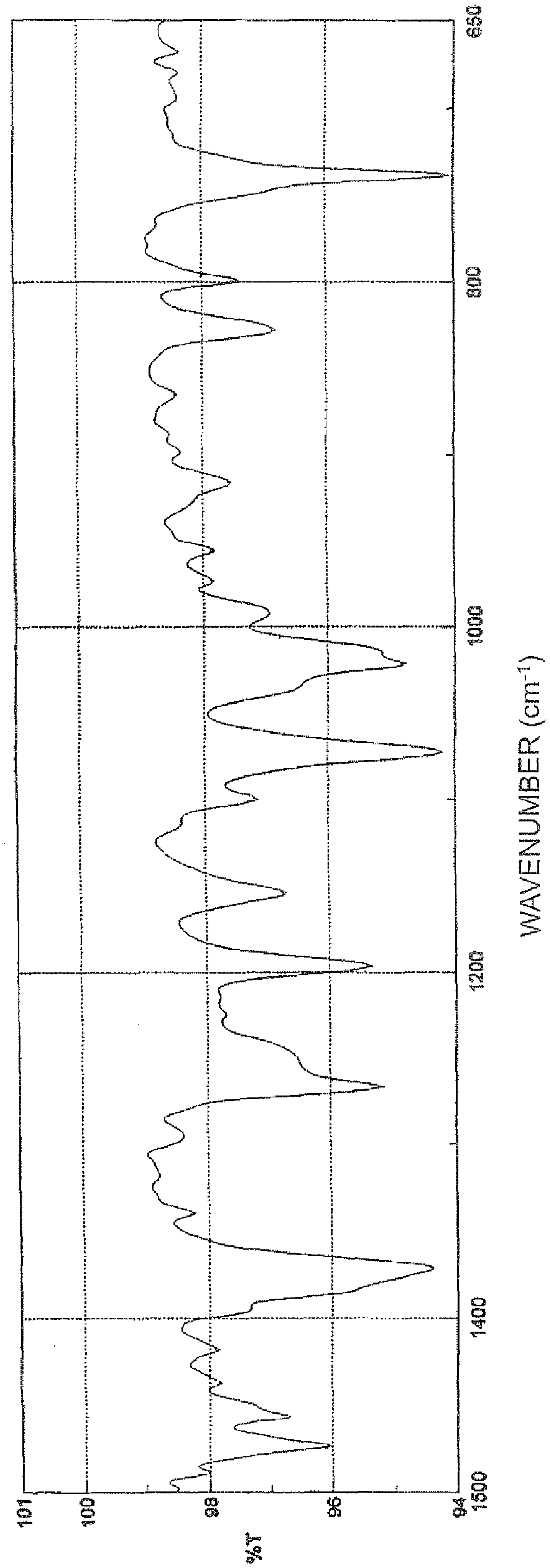

Fig.13

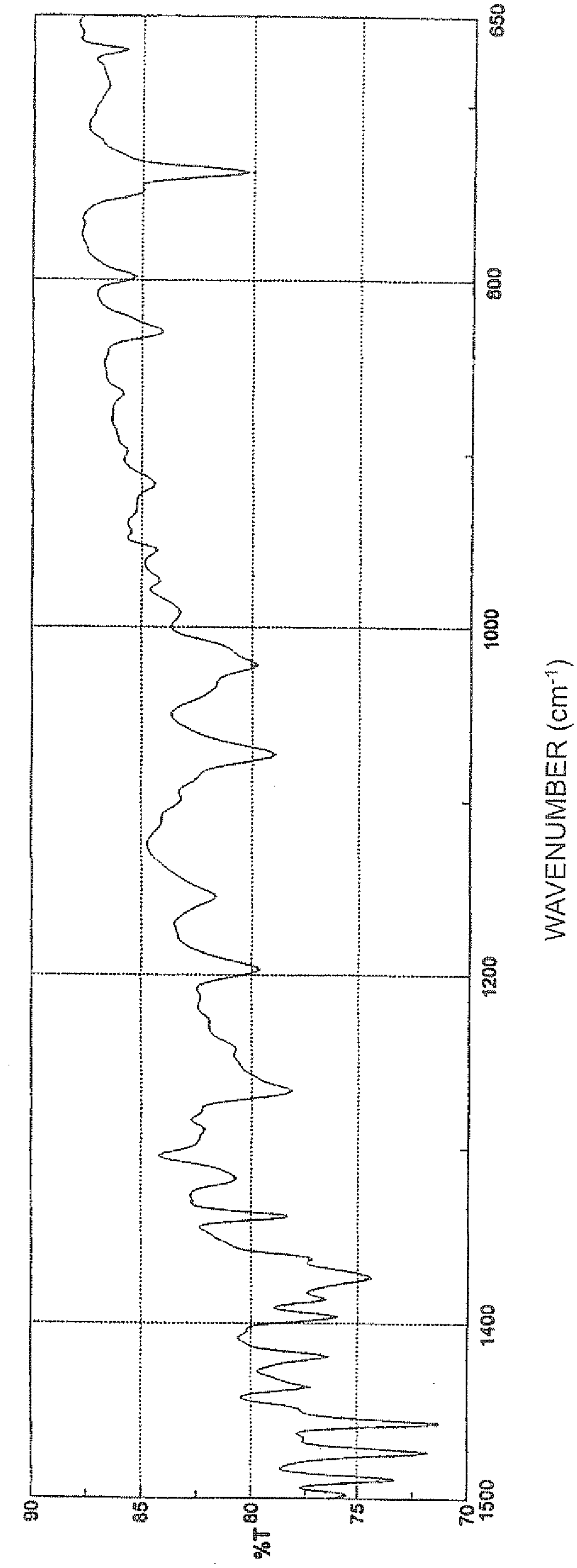

Fig.14

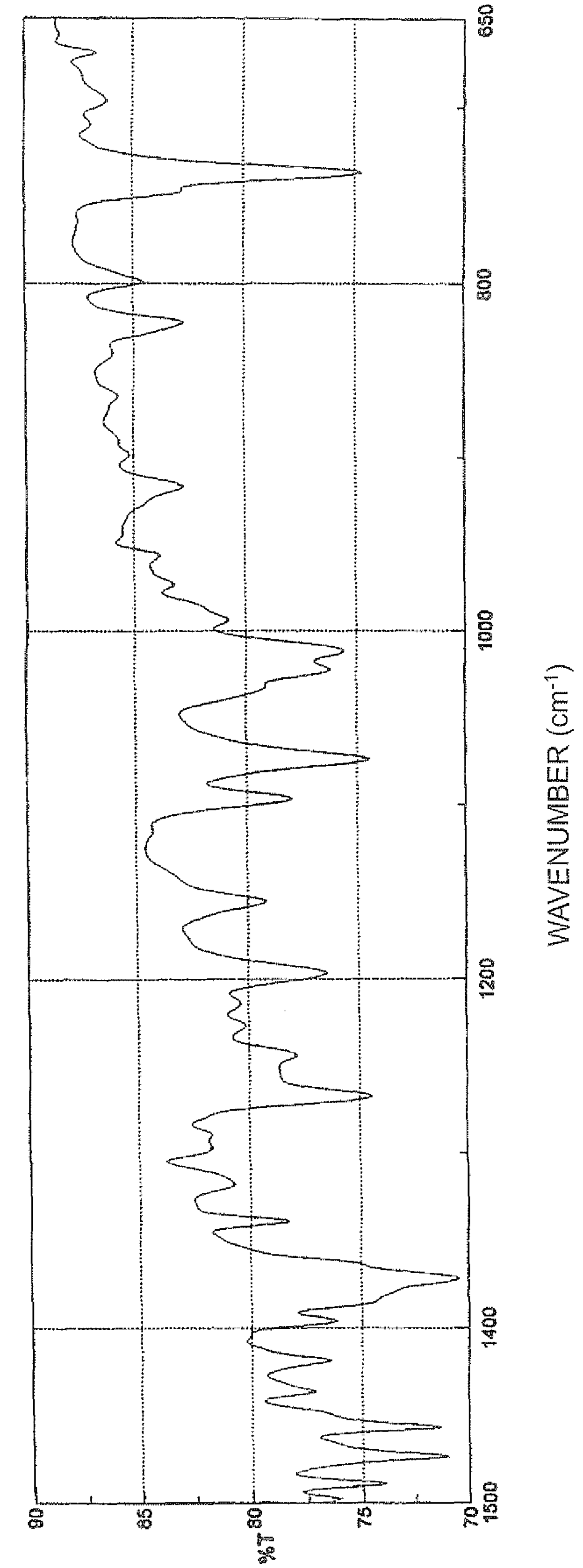

Fig.15

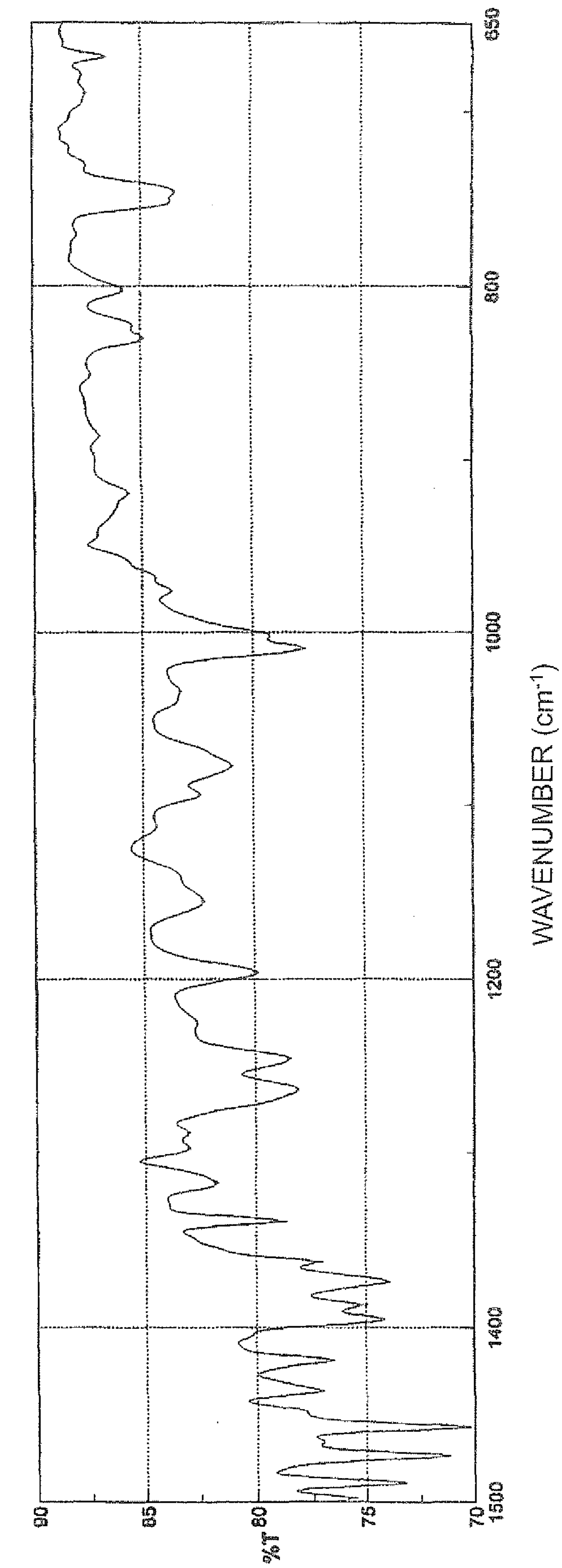

Fig.16

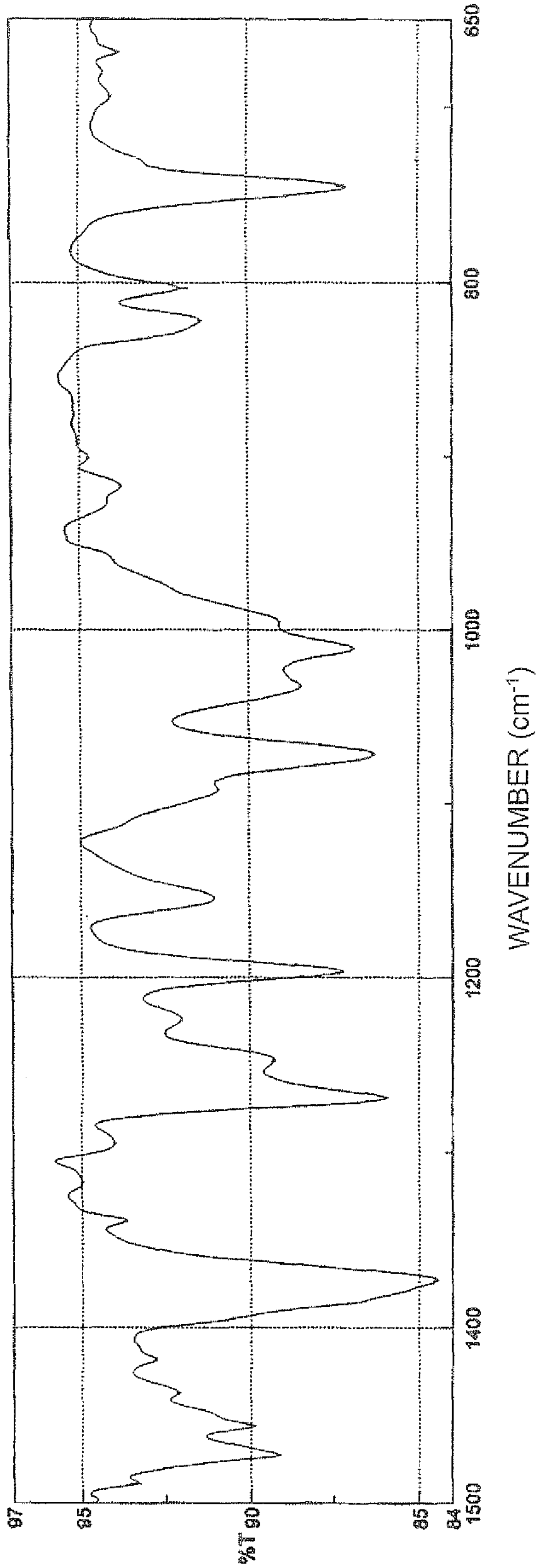

Fig. 17

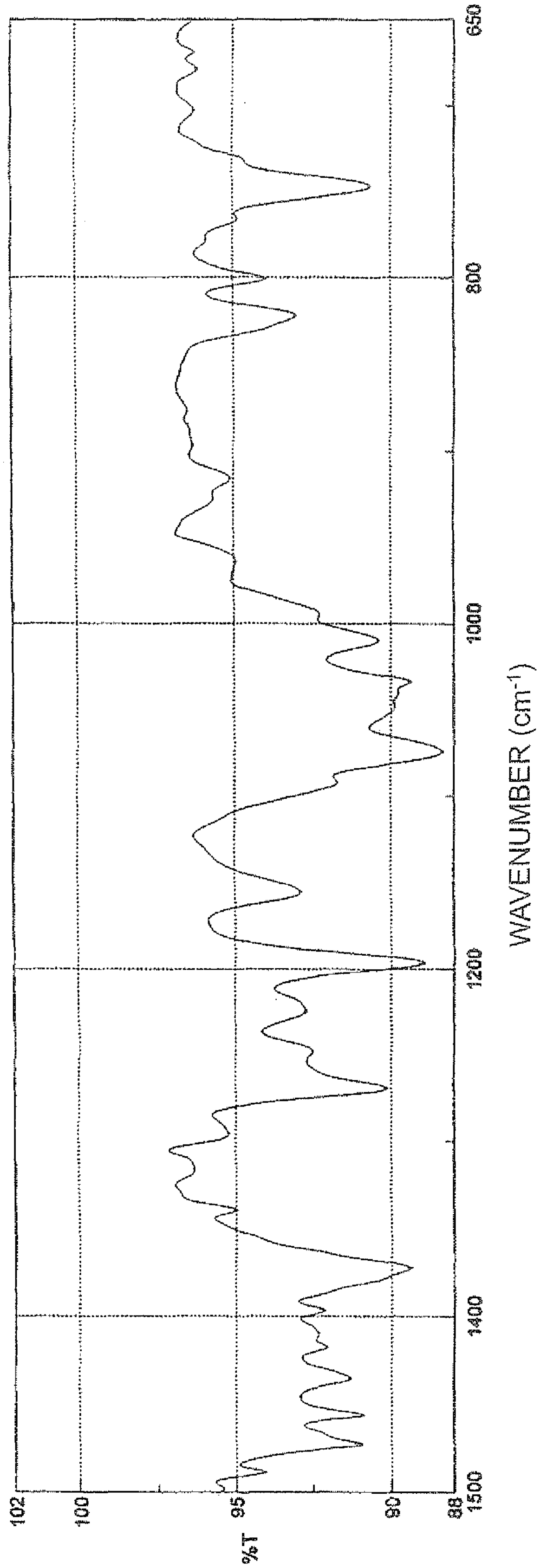

Fig. 18

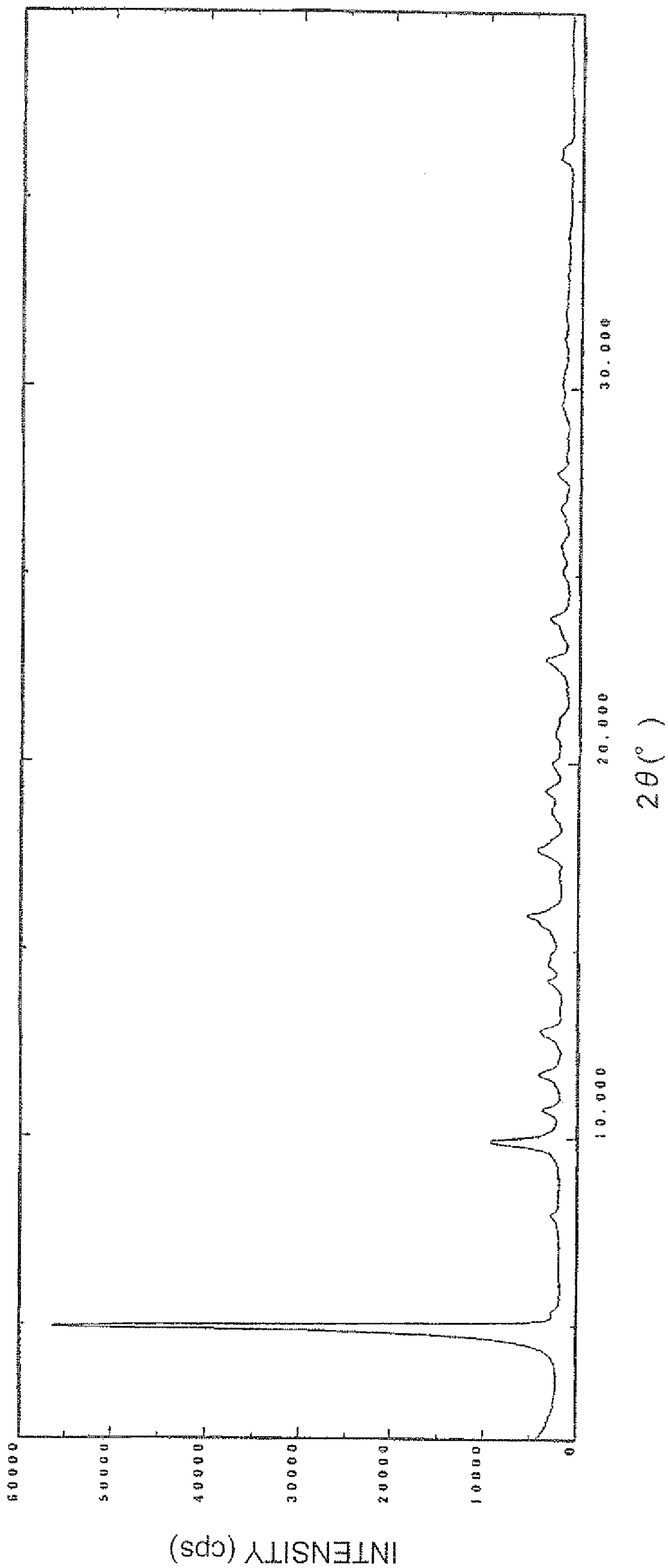
60000
50000
40000
30000
20000
10000
0
INTENSITY (cps)
10.000
20.000
30.000
2θ(°)

Fig. 19

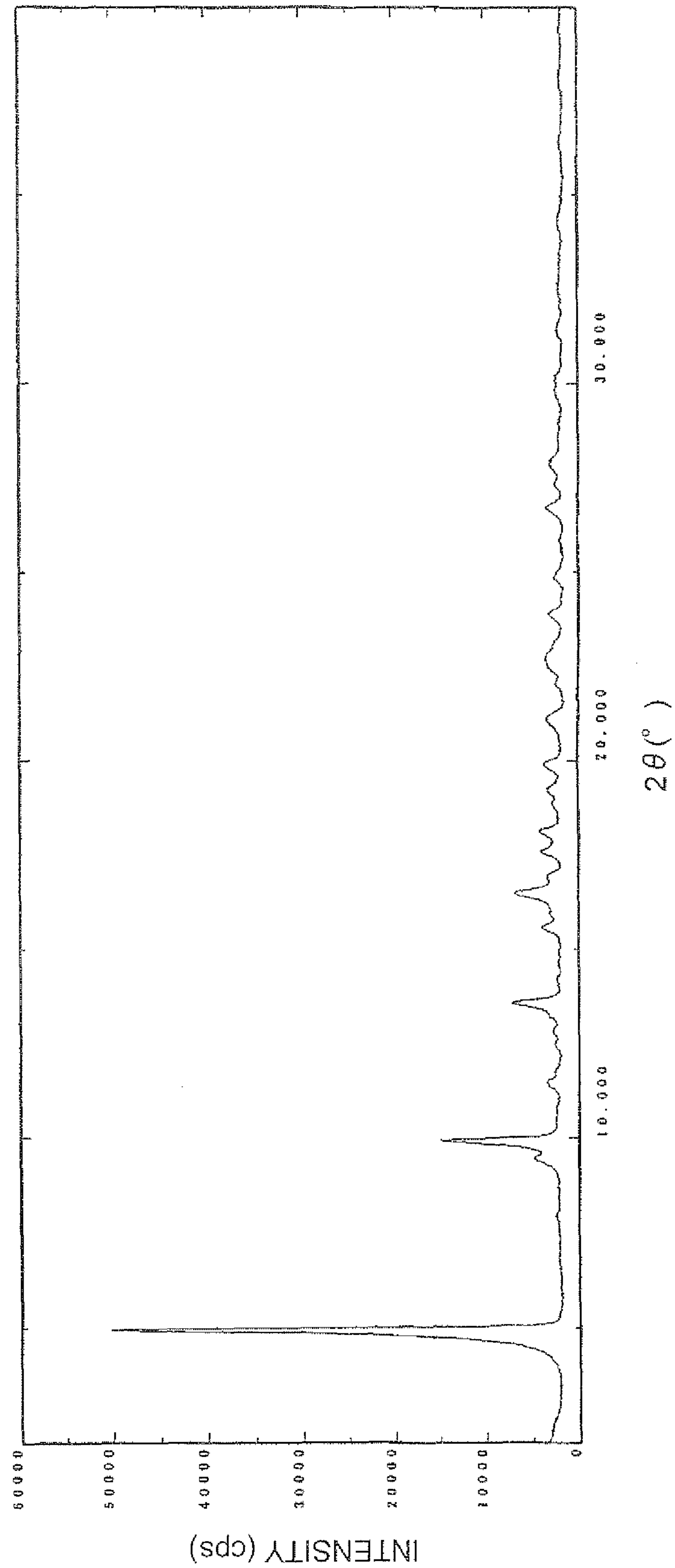
INTENSITY (cps)
60000
50000
40000
30000
20000
10000
0
10.000
20.000
30.000
2θ(°)

Fig. 20

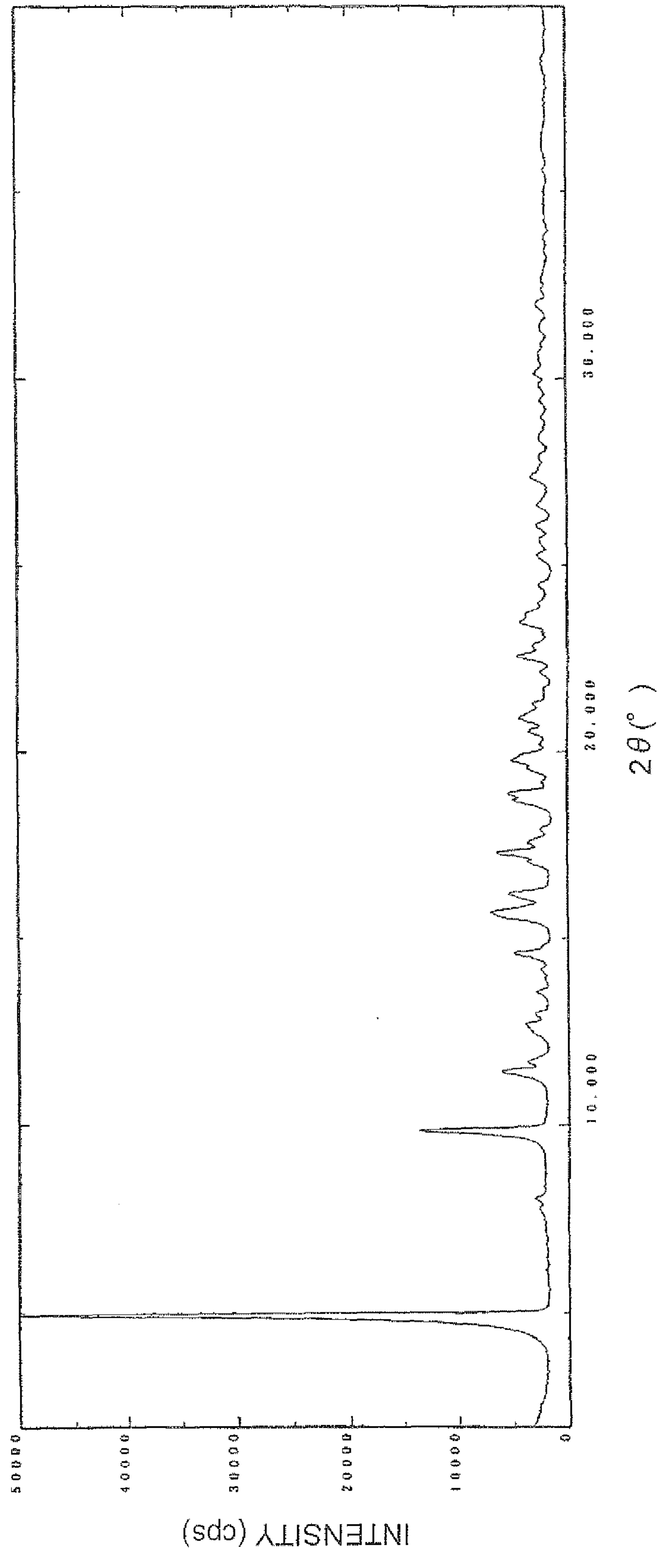
INTENSITY (cps)
50000
40000
30000
20000
10000
0
10.000
20.000
30.000
2θ(° )

Fig.21

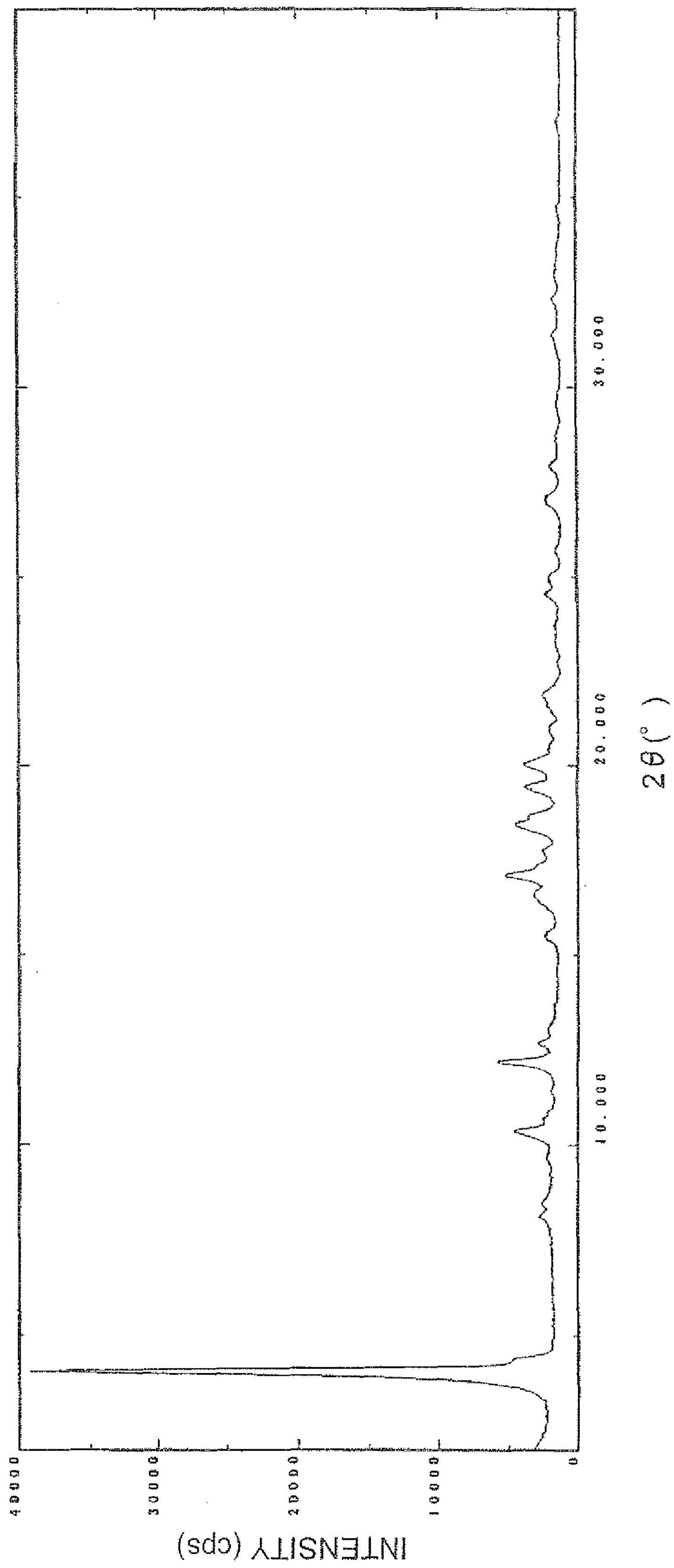

INTENSITY (cps)
40000
30000
20000
10000
0
10.000
20.000
30.000
2θ(°)

Fig. 22

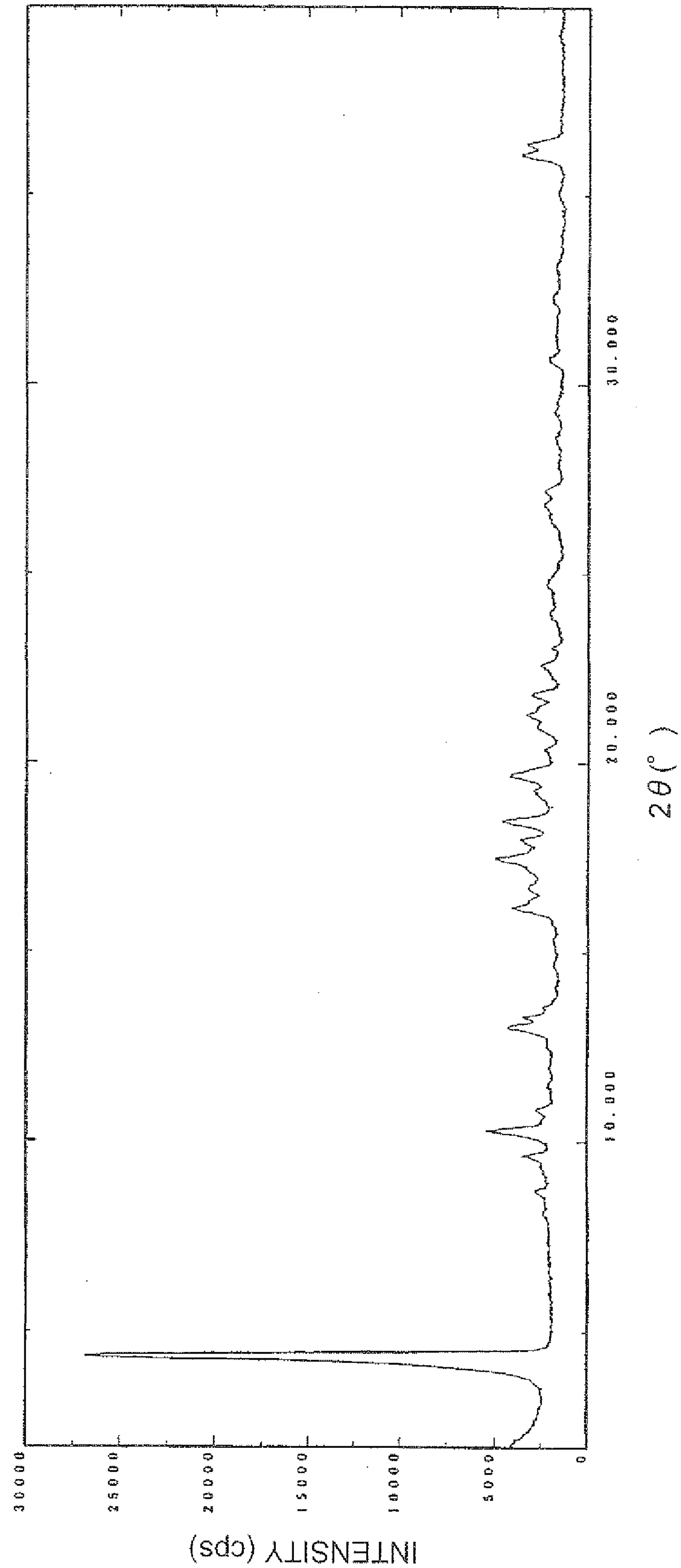
INTENSITY (cps)
30000
25000
20000
15000
10000
5000
0
10.000
20.000
30.000
2θ(° )

Fig.23

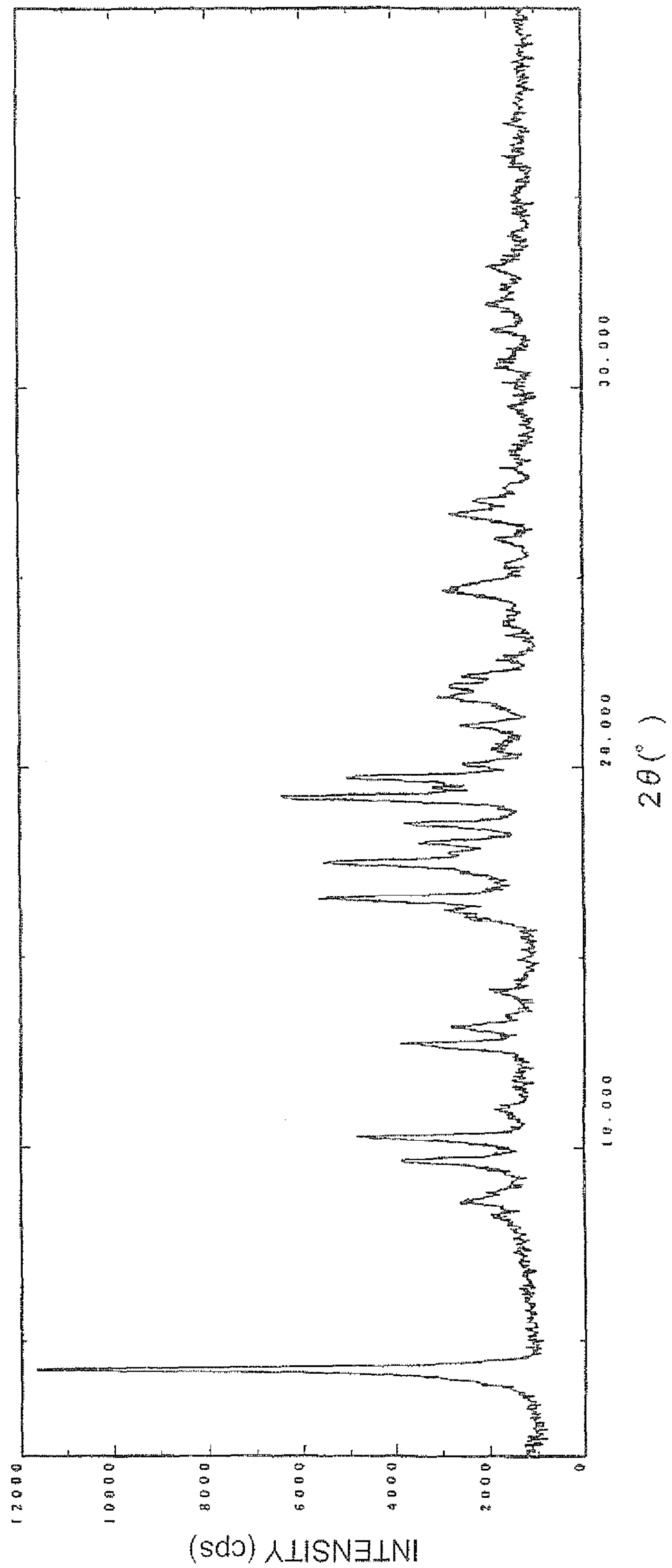
12000
10000
8000
6000
4000
2000
0
INTENSITY (cps)
10.000
20.000
30.000
2θ(°)

Fig.24

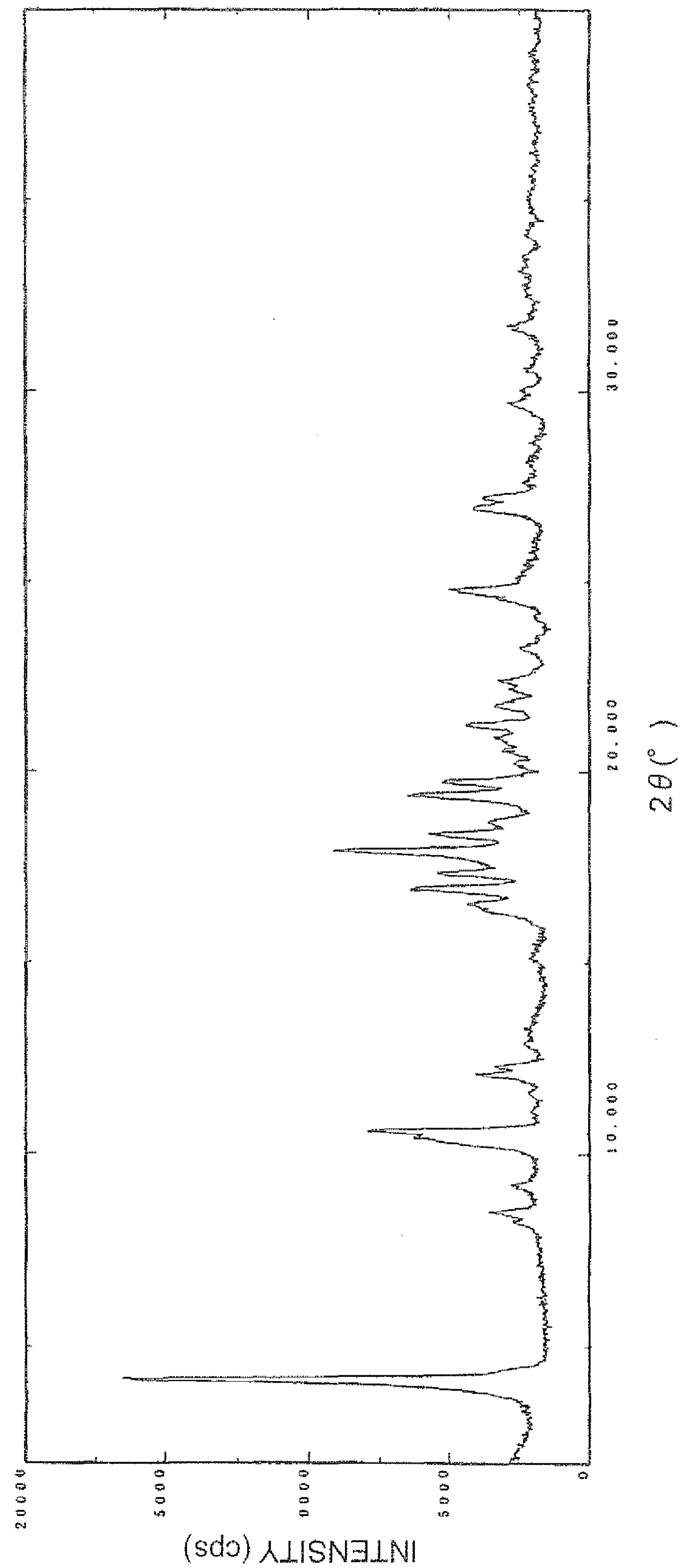

Fig.25

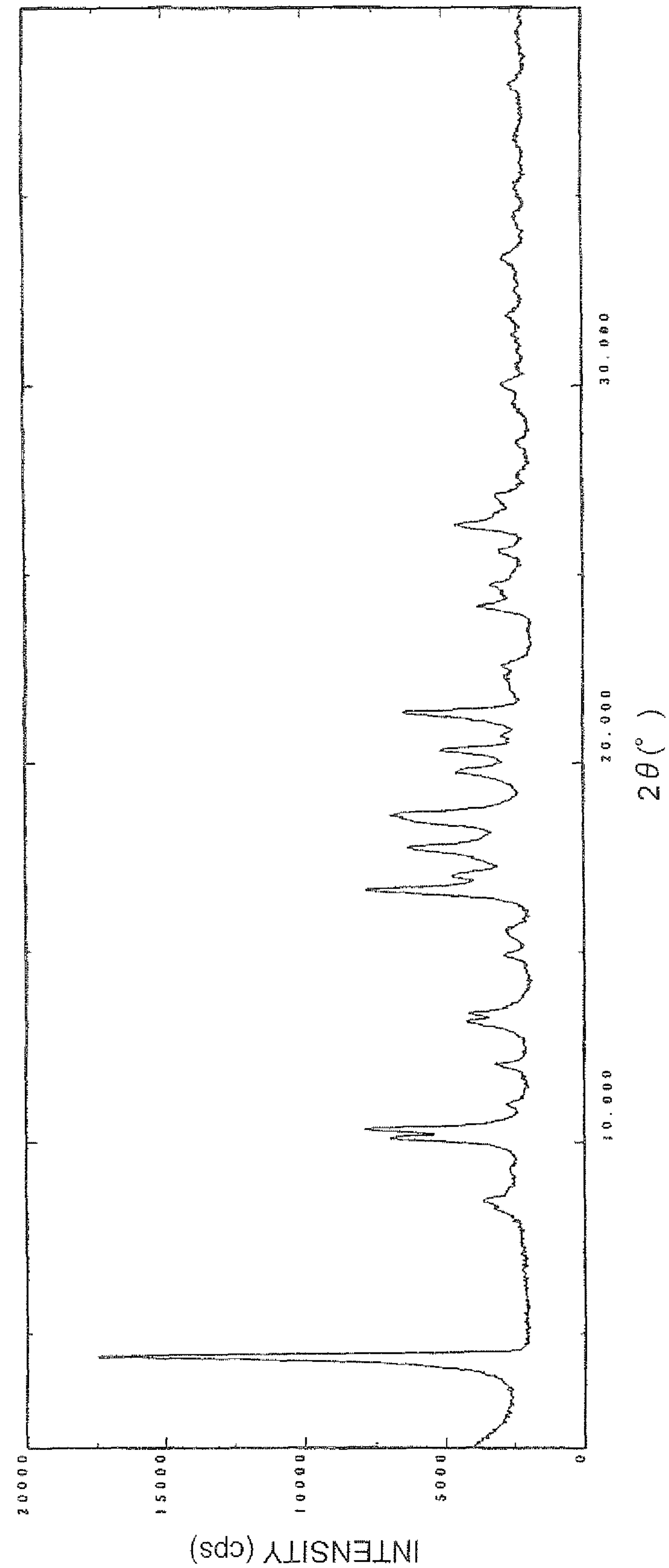

Fig.26

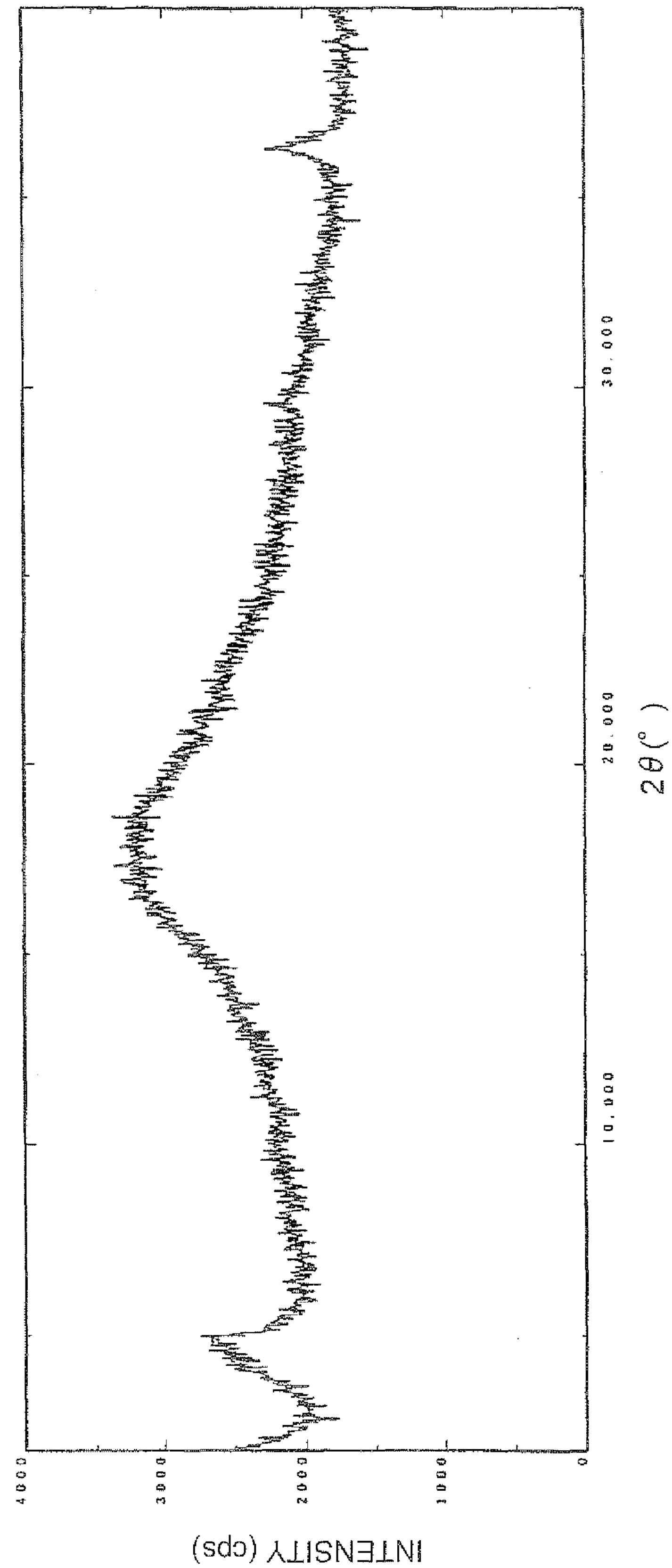

Fig.27

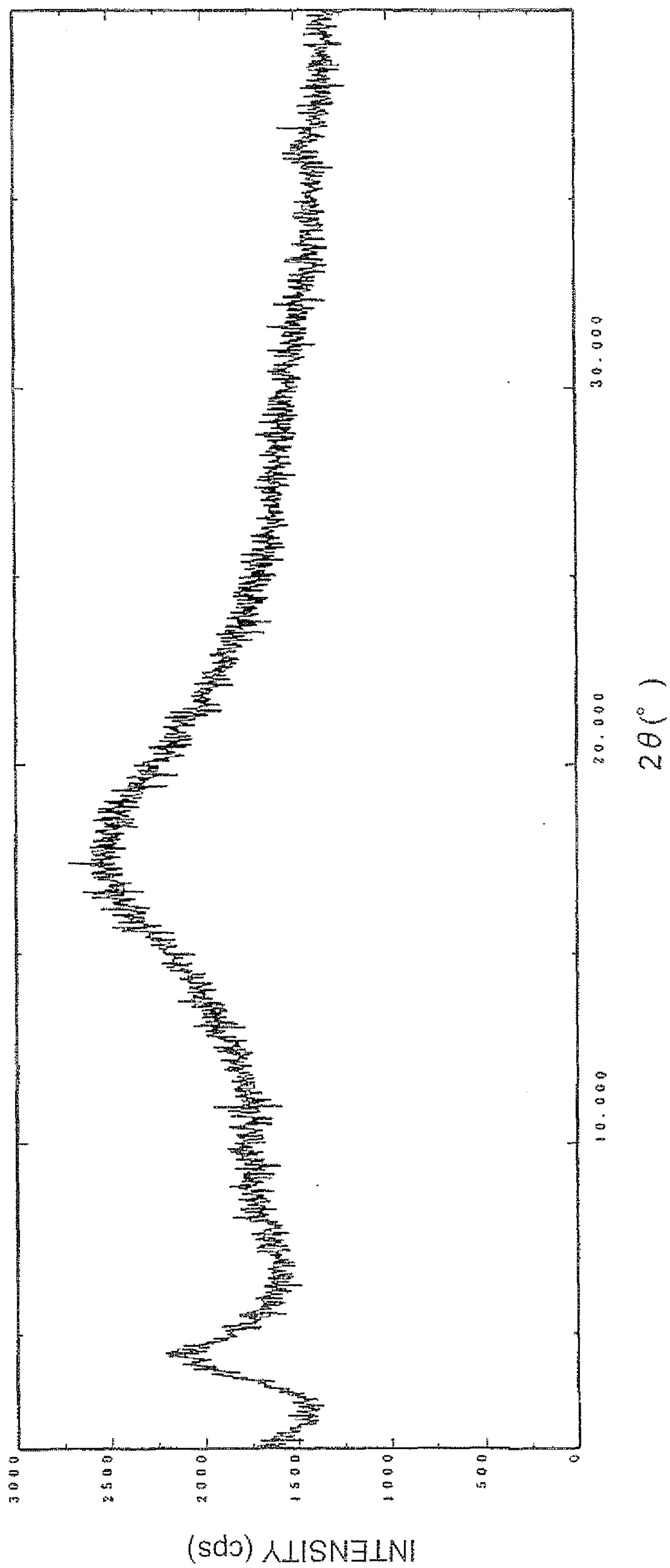
INTENSITY (cps)
2θ(°)
3000
2500
2000
1500
1000
500
0
10.000
20.000
30.000

Fig.28

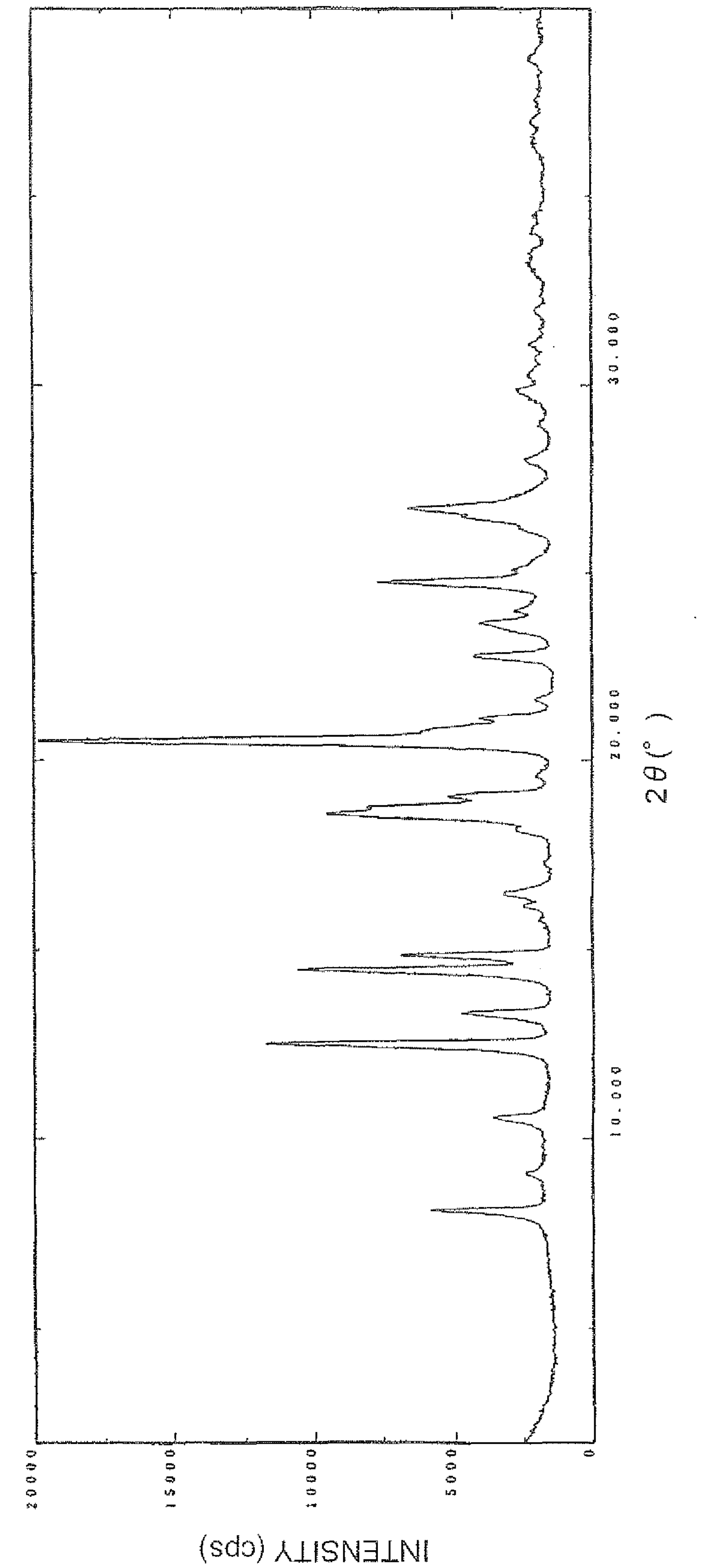

Fig.29

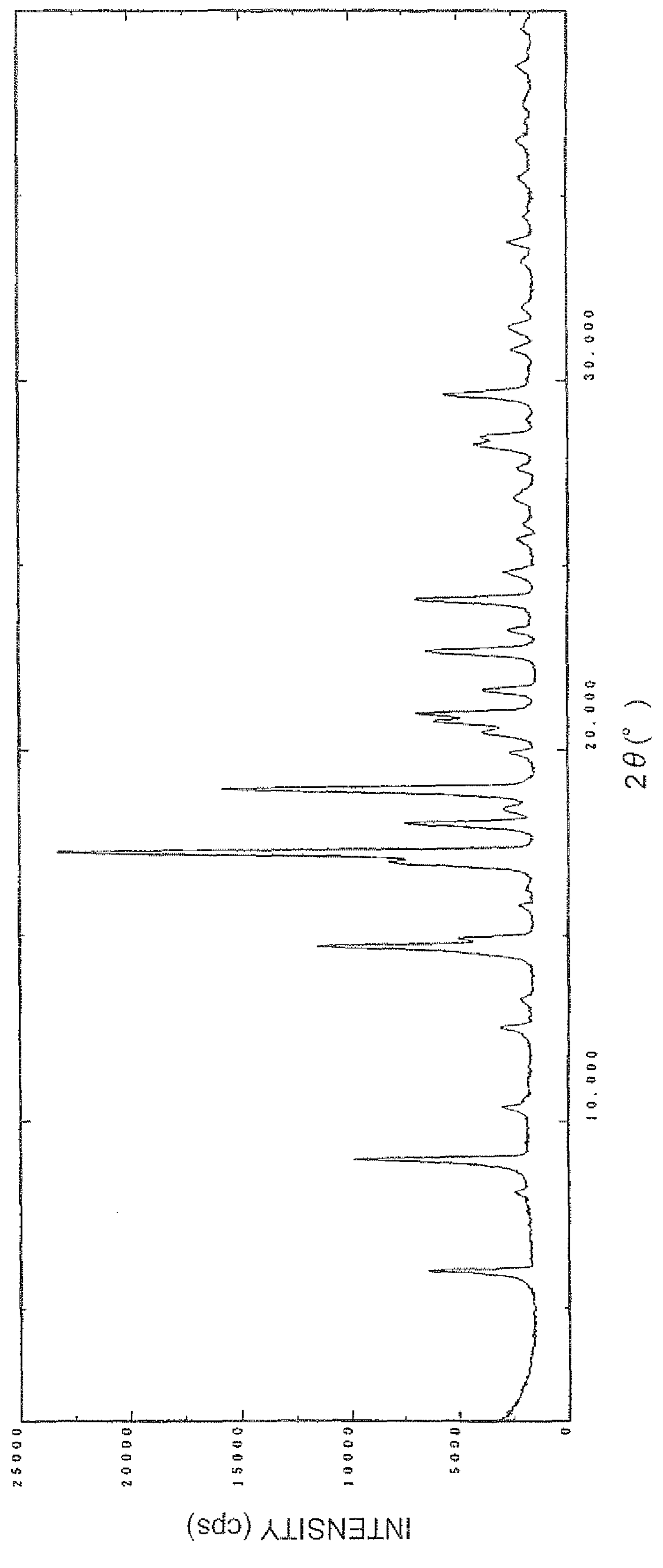
INTENSITY (cps)
25000
20000
15000
10000
5000
0
10.000
20.000
30.000
2θ(°)

Fig.30

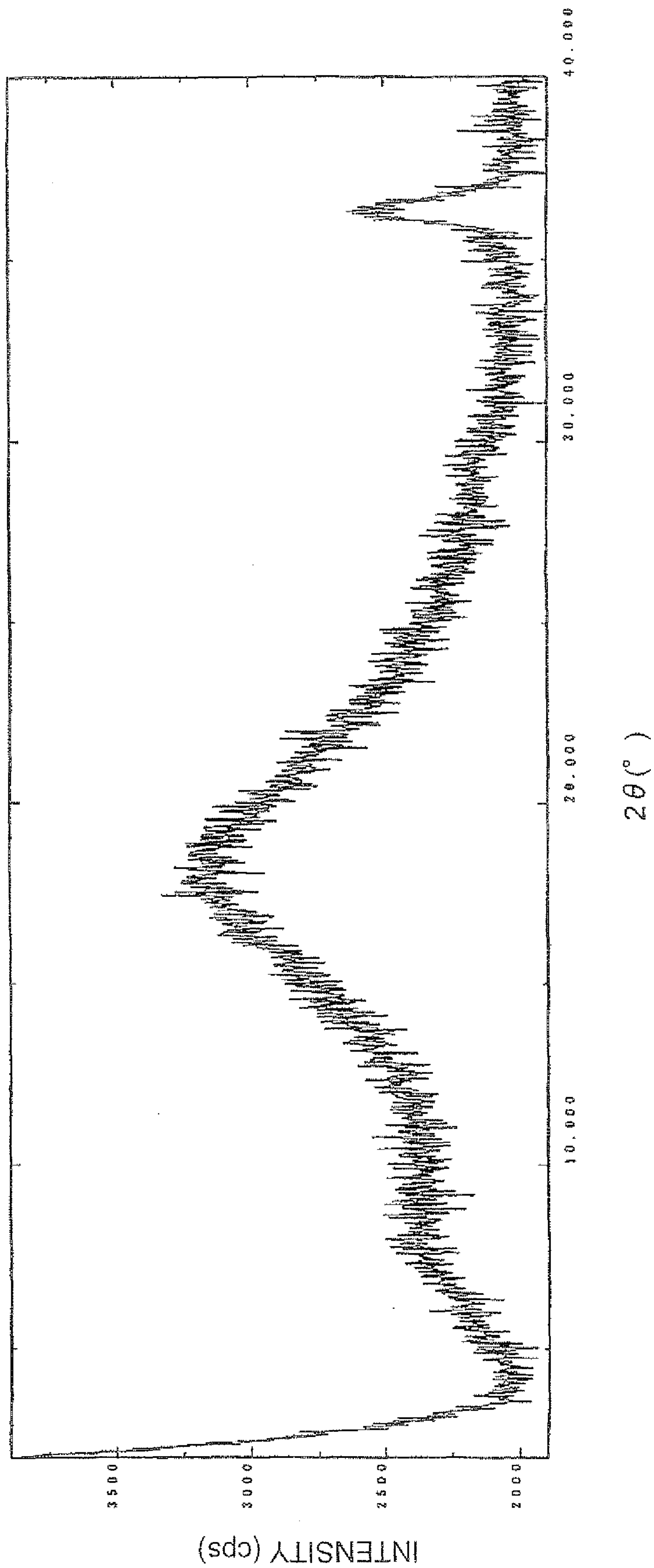
INTENSITY (cps)
3500
3000
2500
2000
2θ(°)
10.000
20.000
30.000
40.000

Fig.31

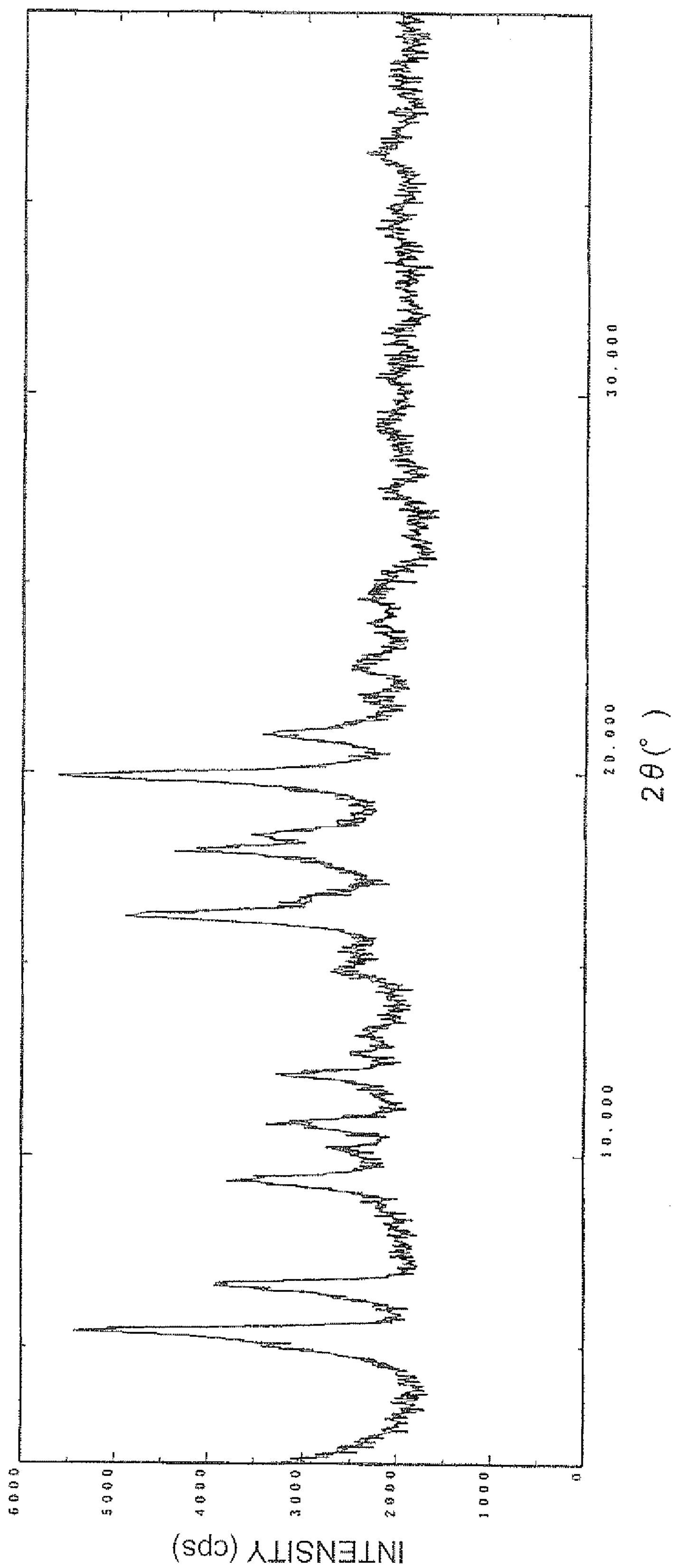
INTENSITY (cps)
6000
5000
4000
3000
2000
1000
0
10.000
20.000
30.000
2θ(°)

Fig.32

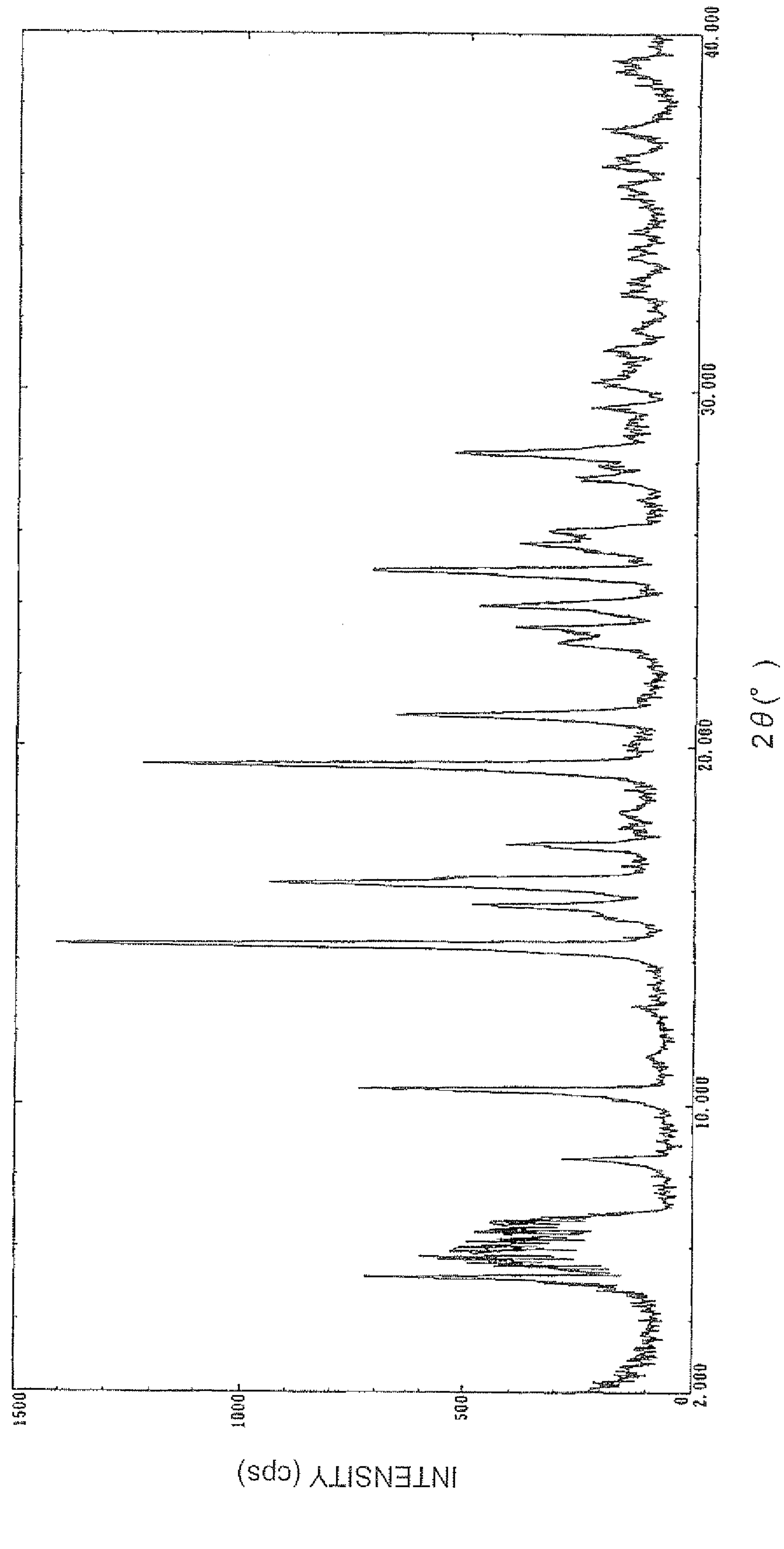
INTENSITY (cps)
1500
1000
500
0
2.000
10.000
20.000
30.000
40.000
2θ(°)

Fig. 33

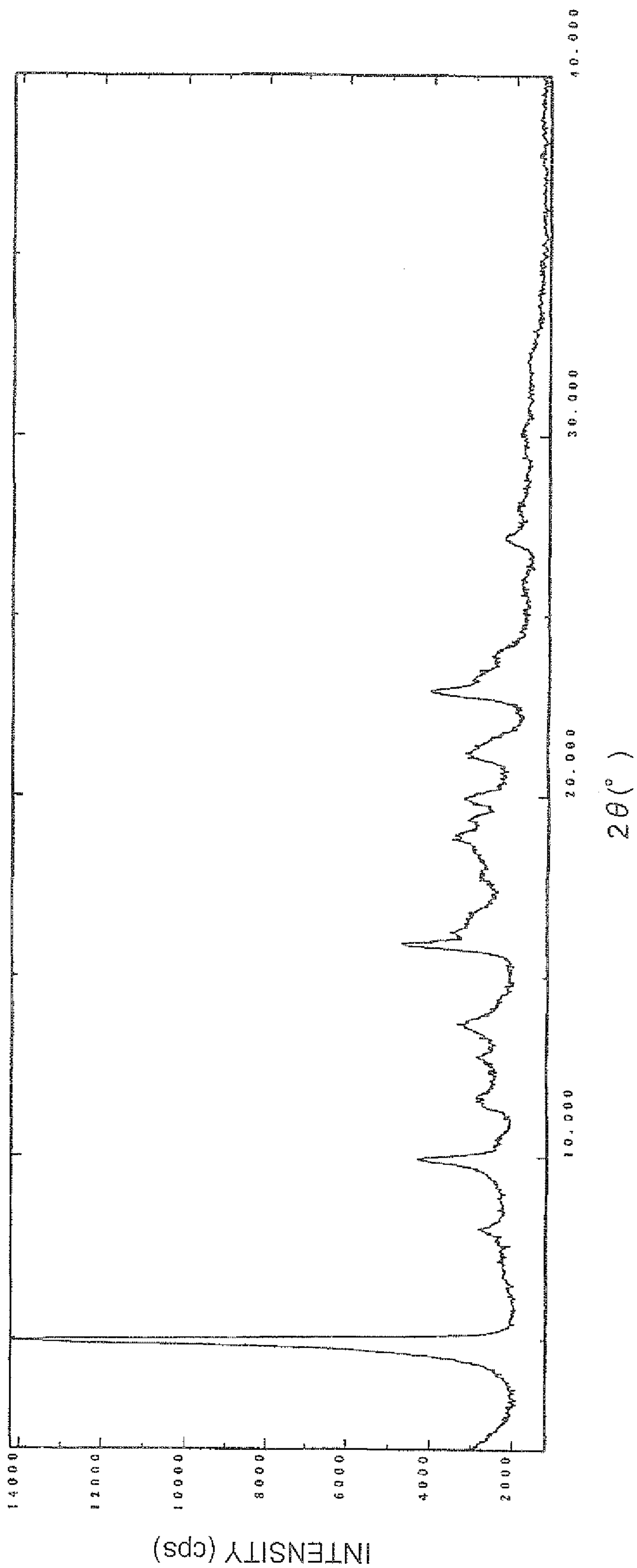
14000
12000
10000
8000
6000
4000
2000
INTENSITY (cps)
10.000
20.000
30.000
40.000
2θ(°)

Fig.34

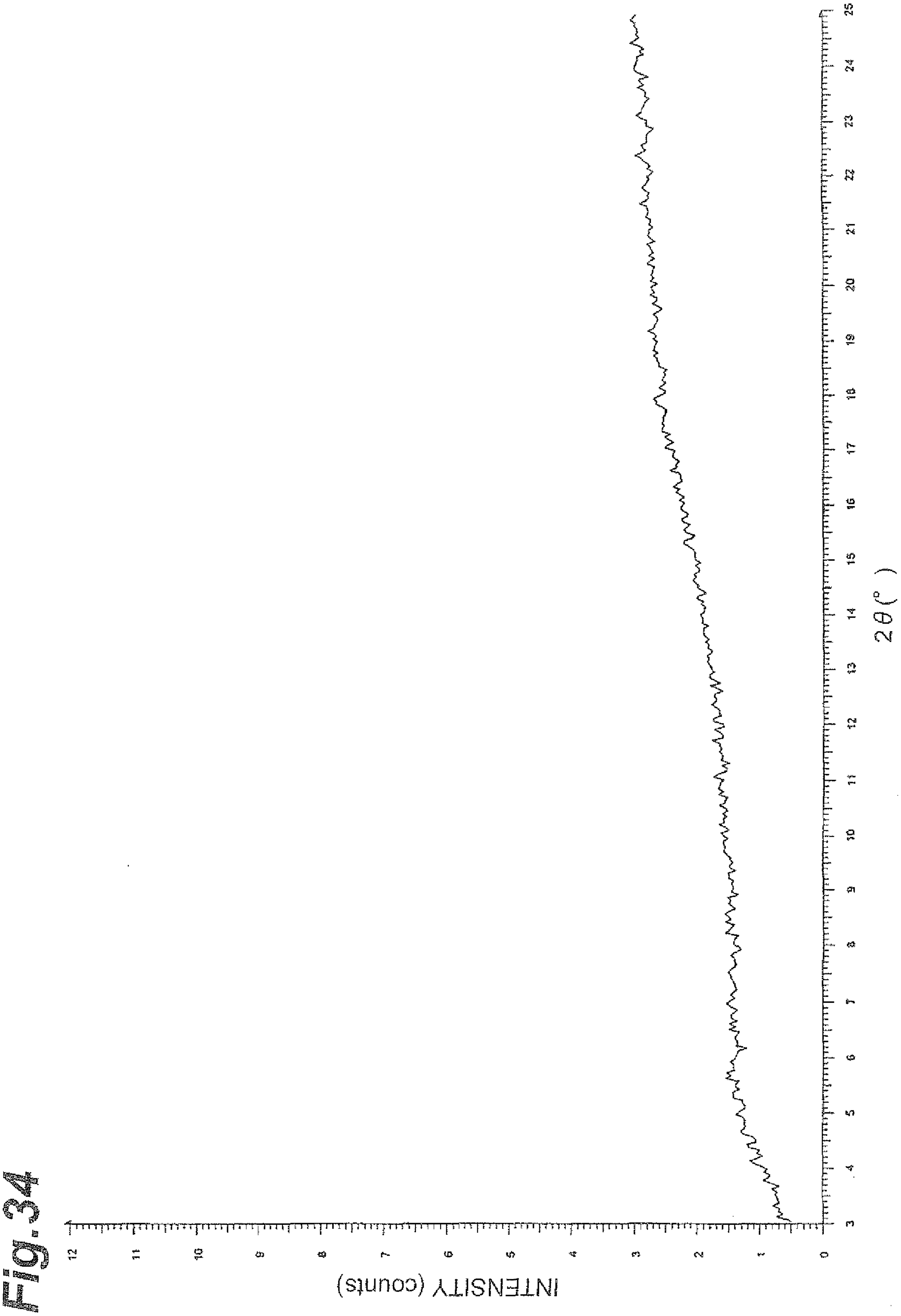
INTENSITY (counts)
0 1 2 3 4 5 6 7 8 9 10 11 12
2θ(° )
3 4 5 6 7 8 9 10 11 12 13 14 15 16 17 18 19 20 21 22 23 24 25

Fig.35

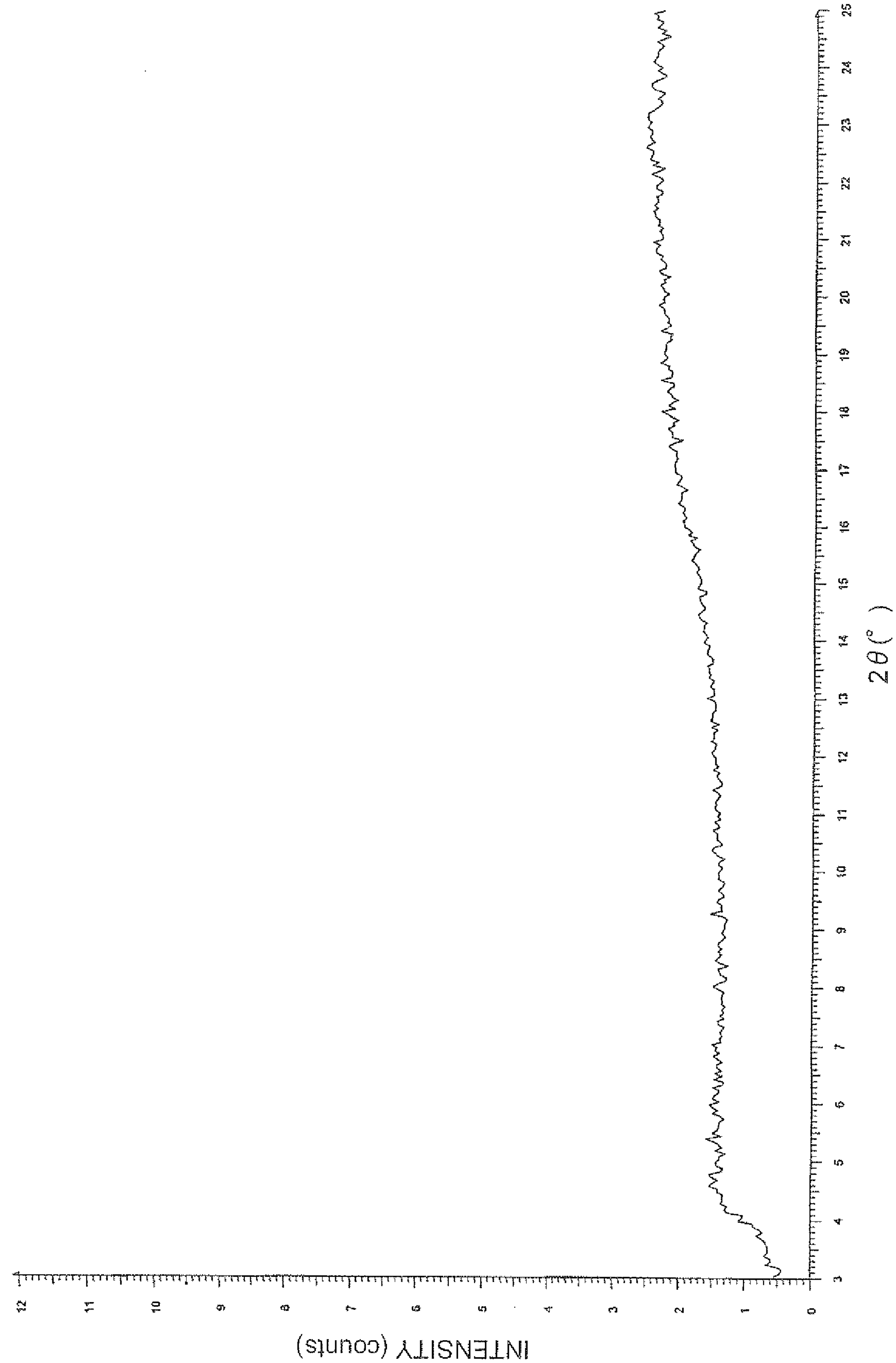
INTENSITY (counts)
2θ(°)
0
1
2
3
4
5
6
7
8
9
10
11
12
3
4
5
6
7
8
9
10
11
12
13
14
15
16
17
18
19
20
21
22
23
24
25

## INTERNATIONAL SEARCH REPORT

International application No.
PCT/JP2006/320496

A. CLASSIFICATION OF SUBJECT MATTER
*C07D405/14*(2006.01)i

According to International Patent Classification (IPC) or to both national classification and IPC

B. FIELDS SEARCHED

Minimum documentation searched (classification system followed by classification symbols)
C07D405/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 5-507713 A (Aktiebolaget Astra), 04 November, 1993 (04.11.93), Claims 1 to 18 & WO 91/19711 A1 & EP 535081 A1 | 1-29 |
| Y | JP 59-181277 A (Aktiebolaget Hassle), 15 October, 1984 (15.10.84), Claims 1 to 14 & US 5039806 A | 1-29 |
| Y | JP 5-117268 A (Yoshitomi Pharmaceutical Industries, Ltd.), 14 May, 1993 (14.05.93), Claim 1; Par. No. [0001] (Family: none) | 1-29 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 November, 2006 (21.11.06) | 28 November, 2006 (28.11.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/320496

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2-22273 A (Yoshitomi Pharmaceutical Industries, Ltd.), 25 January, 1990 (25.01.90), Claims 1 to 6; page 2, lower left column, lines 11 o 14 & WO 89/00566 A1 | 1-29 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9119712 A
- WO SHO59181277 A